(19) Europäisches Patentamt
European Patent Office
Office européen des brevets

(11) Veröffentlichungsnummer: **0 258 184 A2**

# (12) EUROPÄISCHE PATENTANMELDUNG

(21) Anmeldenummer: **87810451.2**

(22) Anmeldetag: **07.08.87**

(51) Int. Cl.⁴: **A 01 N 25/32**

(30) Priorität: **13.08.86 CH 3247/86**

(43) Veröffentlichungstag der Anmeldung: **02.03.88 Patentblatt 88/09**

(84) Benannte Vertragsstaaten: **AT BE CH DE ES FR GB GR IT LI NL SE**

(71) Anmelder: **CIBA-GEIGY AG**
**Klybeckstrasse 141**
**CH-4002 Basel (CH)**

(72) Erfinder: **Nyffeler, Andreas, Dr.**
**Gründlerstrasse 4**
**CH-4312 Magden (CH)**

**Schurter, Rolf, Dr.**
**Holzmattstrasse 45**
**CH-4102 Binningen (CH)**

**Hubele, Adolf, Dr.**
**Obere Egg 9**
**CH-4312 Magden (CH)**

## (54) Chinolinderivate als Herbizid- antagonisten für Kulturpflanzen.

(57) Die Verwendung von Chinolinderivaten der Formel

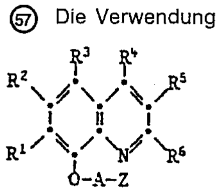

,

worin $R^1$, $R^2$ und $R^2$ unabhängig voneinander Wasserstoff, Halogen, Nitro, Cyano, $C_1$-$C_3$-Alkyl oder $C_1$-$C_3$-Alkoxy,
$R^4$, $R^5$ und $R^6$ unabhängig voneinander Wasserstoff, Halogen oder $C_1$-$C_3$-Alkyl,
A eine der Gruppen $-CH_2-$, $-CH_2CH_2-$ oder $-CH(CH_3)-$ und
Z a) Cyan oder Amidoxim, welches am Sauerstoffatom acyliert sein kann, oder
b) eine Carboxylgruppe oder ein Salz davon, eine Mercaptocarbonylgruppe oder ein Salz davon, eine Carbonsäureestergruppe, eine Carbonsäurethiolestergruppe, eine unsubstituierte oder substituierte Carbonsäureamidgruppe, ein cyclisiertes, unsubstituiertes oder substituiertes Derivat einer Carbonsäureamidgruppe oder eine Carbonsäurehydrazidgruppe, oder
A und Z zusammen einen unsubstituierten oder substituierten Tetrahydrofuran-2-on-Ring
bedeuten, unter Einschluss ihrer Säureadditionssalze und Metallkomplexe, zum Schützen von Kulturpflanzen gegen schädigende Wirkungen von herbizid wirksamen 2-[4-(5-Chlor-3-fluorpyridin-2-yloxy)-phenoxy]-propionsäure Derivaten.

Die vorgenannten herbiziden 2-[4-(5-Chlor-3-fluorpyridin-2-yloxy)-phenoxy]-propionsäure-Derivate entsprechen der Formel

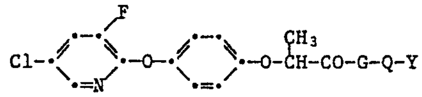

worin
G Sauerstoff oder Schwefel,
Q eine unsubstituierte oder einfach oder zweifach durch Methyl oder Phenyl substituierte $C_1$-$C_3$-Alkylenkette, und
Y eine Gruppe $-Si(OR^{17})_2R^{18}$, $-Si(R^{18})_3$, $-PO(OR^{19})-OR^{20}$, $-PO(OR^{19})-R^{20}$ oder $-O-PO(OR^{19})_2$
bedeuten, wobei
$R^{17}$ und $R^{18}$ unabhängig voneinander für $C_1$-$C_6$-Alkyl-und
$R^{19}$ und $R^{20}$ unabhängig voneinander für Wasserstoff, $C_1$-$C_4$-Alkyl, $C_3$-$C_4$-Alkenyl, $C_3$-$C_4$-Alkinyl, $C_2$-$C_4$-Halogenalkyl, $C_1$-$C_4$-Alkoxy-($C_2$-$C_4$)-alkyl, $C_1$-$C_4$-Cyanoalkyl oder einen unsubstituierten oder durch Halogen, $C_1$-$C_4$-Alkyl, $C_1$-$C_4$-Alkoxy, $C_1$-$C_4$-Halogenalkyl, $C_1$-$C_4$-Halogenalkoxy, Nitro oder Cyano ein- bis zweifach substituierten Phenyl-, Phenyl-($C_1$-$C_4$)-alkyl oder Naphthylrest stehen.

EP 0 258 184 A2

**Beschreibung**

Chinolinderivate als Herbizidantagonisten für Kulturpflanzen

Die vorliegende Erfindung betrifft die Verwendung von Chinolinderivaten zum Schützen von Kulturpflanzen gegen schädigende Wirkungen herbizid wirksamer Silizium- und Phosphor-haltiger 2-[4-(5-Chlor-3-fluorpyridin-2-yloxy)-phenoxy]-propionsäure-Derivate sowie herbizide Mittel, welche eine Kombination von Herbizid und schützendem Chinolinderivat enthält.

Beim Einsatz von Herbiziden wie beispielsweise den vorstehend genannten Propionsäure-Derivaten können in Abhängigkeit von Faktoren wie beispielsweise Dosis des Herbizids und Applikationsart, Art der Kulturpflanze, Bodenbeschaffenheit und klimatischen Bedingungen, wie beispielsweise Belichtungsdauer, Temperatur und Niederschlagsmengen, die Kulturpflanzen in erheblichem Masse geschädigt werden. Insbesondere kann es zu starken Schädigungen kommen, wenn im Rahmen der Fruchtfolge nach Kulturpflanzen, die gegen die Herbizide resistent sind, andere Kulturpflanzen angebaut werden, welche keine oder nur unzureichende Resistenz gegenüber den Herbiziden aufweisen.

Es ist aus den europäischen Patentpublikationen 86 750 und 94 349 bekannt, dass sich Chinolinderivate zum Schützen von Kulturpflanzen gegen schädigende Wirkungen aggresiver Agrarchemikalien einsetzen lassen.

Es wurde nun gefunden, dass überraschenderweise ein Schutz von Kulturpflanzen gegen Schäden, welche durch herbizid wirksame Silizium- und Phosphor-haltige 2-[4-(5-Chlor-3-fluorpyridin-2-yloxy)-phenoxy]-propionsäure-Derivate verursacht werden, durch Behandlung der Kulturpflanzen, von Teilen dieser Pflanzen oder von für den Anbau der Kulturpflanzen bestimmten Böden mit einem Safener aus einer Gruppe von Chinolinderivaten erzielt werden kann. Die herbizide Wirkung gegenüber Unkräutern und Ungräsern wird durch die Chinolinderivate nicht aufgehoben.

Chinolinderivate, welche zum Schützen von Kulturpflanzen vor schädigenden Wirkungen herbizid wirksamer 2-[4-(5-Chlor-3-fluorpyridin-2-yloxy)-phenoxy]-propionsäure-Derivate geeignet sind, entsprechen der Formel I

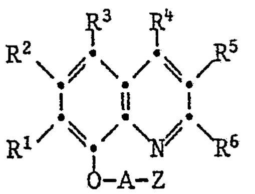

(I),

worin $R^1$, $R^2$ und $R^3$ unabhängig voneinander Wasserstoff, Halogen, Nitro, Cyano, $C_1$-$C_3$-Alkyl oder $C_1$-$C_3$-Alkoxy, $R^4$, $R^5$ und $R^6$ unabhängig voneinander Wasserstoff, Halogen oder $C_1$-$C_3$-Alkyl, A eine der Gruppen $-CH_2-$, $-CH_2-CH_2-$ oder $-CH(CH_3)-$ und Z a) Cyan oder Amidoxim, welches am Sauerstoffatom acyliert sein kann, oder b) eine Carboxylgruppe oder ein Salz davon, eine Mercaptocarbonylgruppe oder ein Salz davon, eine Carbonsäureestergruppe, eine Carbonsäurethiolestergruppe, eine unsubstituierte oder substituierte Carbonsäureamidgruppe, ein cyclisiertes, unsubstituiertes oder substituiertes Derivat einer Carbonsäureamidgruppe oder eine Carbonsäurehydrazidgruppe, oder A und Z zusammen einen unsubstituierten oder substituierten Tetrahydrofuran-2-on-Ring bedeuten, unter Einschluss ihrer Säureadditionssalze und Metallkomplexe.

Unter Amidoxim ist die Gruppe $-C(NH_2)=N-OH$ zu verstehen. Das Amidoxim kann am Sauerstoffatom acyliert sein. Als am Sauerstoffatom acylierte Amidoxime kommen solche der Formel $-C(NH_2)=N-O-CO-E$ in Betracht, in denen E für $-R^7$, $-OR^8$, $-SR^9$ oder $-NR^{10}R^{11}$ steht, wobei $R^7$ $C_1$-$C_7$-Alkyl, welches unsubstituiert oder durch Halogen oder $C_1$-$C_4$-Alkoxy substituiert ist, $C_3$-$C_6$-Cycloalkyl, $C_2$-$C_4$-Alkenyl, Phenyl, welches unsubstituiert oder durch Halogen, Nitro oder $C_1$-$C_3$-Alkyl substituiert ist, Benzyl, welches unsubstituiert oder durch Halogen, Nitro oder $C_1$-$C_3$-Alkyl substituiert ist, oder einen 5- bis 6-gliedrigen heterocyclischen Ring, welcher ein oder zwei Heteroatome aus der Gruppe N, O oder S enthält und unsubstituiert oder durch Halogen substituiert ist, $R^8$, $R^9$ und $R^{10}$ unabhängig voneinander $C_1$-$C_8$-Alkyl, welches unsubstituiert oder durch Halogen substituiert ist, $C_2$-$C_4$-Alkenyl, $C_3$-$C_6$-Alkinyl, Phenyl, welches unsubstituiert oder durch Halogen, $C_1$-$C_3$-Alkyl, $C_1$-$C_3$-Alkoxy, Trifluormethyl oder Nitro substituiert ist, oder Benzyl, welches unsubstituiert oder durch Halogen oder Nitro substituiert ist, $R^{11}$ Wasserstoff, $C_1$-$C_8$-Alkyl oder $C_1$-$C_3$-Alkoxy, oder $R^{10}$ und $R^{11}$ gemeinsam mit dem Stickstoffatom, an das sie gebunden sind, einen 5- bis 6-gliedrigen Heterocyclus, welcher noch ein weiteres Heteroatom aus der Gruppe N, O und S enthalten kann, bedeuten.

Bei $R^7$ als Heterocyclus kann es sich um gesättigte, teilgesättigte oder ungesättigte Heterocyclen handeln, wie beispielsweise Thiophen, Furan, Tetrahydrofuran und Pyrimidin.

Als Heterocyclen, welche von $R^{10}$ und $R^{11}$ gemeinsam mit dem Stickstoffatom, an das sie gebunden sind, gebildet werden, kommen gesättigte, teilgesättigte oder ungesättigte Heterocyclen in Betracht. Beispiele für solche Heterocyclen sind Pyrrolidin, Pyrrolin, Pyrrol, Imidazolidin, Imidazolin, Imidazol, Piperazin, Pyridin, Pyrimidin, Pyrazin, Thiazin, Oxazol, Thiazol und insbesondere Piperidin und Morpholin.

Unter Alkyl als Bestandteil des acylierten Amidoxims Z kommen im Rahmen der jeweils angegebenen Anzahl von Kohlenstoffatomen alle geradkettigen und alle verzweigten Alkylgruppen in Betracht.

In der Bedeutung von $R^7$ steht $C_3$-$C_6$-Cycloalkyl für Cyclopropyl, Cyclobutyl, Cyclopentyl und Cyclohexyl.

Von den $C_2$-$C_4$-Alkenyl- und $C_3$-$C_6$-Alkinylgruppen als Bestandteile des acylierten Amidoxims Z sind vor allem Vinyl, Allyl, 1-Propenyl, Methallyl und Propargyl zu erwähnen.

Für Z als Carbonsäureestergruppe oder Carbonsäurethiolestergruppe kommt ein entsprechender Säurerest in Betracht, der beispielsweise durch einen gegebenenfalls substituierten, aliphatischen Rest oder einen gegebenenfalls über einen aliphatischen Rest gebundenen und gegebenenfalls substituierten cycloaliphatischen, aromatischen oder heterocyclischen Rest verestert ist.

Als Carbonsäureesterrest bevorzugt ist der Rest -$COOR^{12}$ und als Carbonsäurethiolesterrest bevorzugt ist der Rest -$COSR^{13}$, wobei $R^{12}$ und $R^{13}$ die nachfolgend angegebenen Bedeutungen haben: gegebenenfalls substituierter Alkyl-, Alkenyl-, Alkinyl-, Cycloalkyl-, Phenyl- oder Naphthylrest oder gegebenenfalls substituierter heterocyclischer Rest. Die Reste -$COOR^{12}$ und -$COSR^{13}$ schliessen auch die freien Säuren ein, wobei $R^{12}$ und $R^{13}$ für Wasserstoff stehen, sowie die Salze davon, wobei $R^{12}$ und $R^{13}$ für ein Kation stehen. Als Salzbildner eignen sich hier besonders Metalle und organische Stockstoffbasen, vor allem quaternäre Ammoniumbasen. Hierbei kommen als zur Salzbildung geeignete Metalle Erdalkalimetalle, wie Magnesium oder Calcium, vor allem aber die Alkalimetalle in Betracht, wie Lithium und insbesondere Kalium und Natrium. Ferner sind als Salzbildner auch Uebergangsmetalle wie beispielsweise Eisen, Nickel Kobalt, Kupfer, Zink, Chrom oder Mangan geeignet. Beispiele für zur Salzbildung geeignete Stickstoffbasen sind primäre, sekundäre oder tertiäre, aliphatische und aromatische, gegebenenfalls am Kohlenwasserstoffrest hydroxylierte Amine, wie Methylamin, Aethylamin, Propylamin, Isopropylamin, die vier isomeren Butylamine, Dimethylamin, Diäthylamin, Dipropylamin, Diisopropylamin, Di-n-butylamin, Pyrrolidin, Piperidin, Morpholin, Trimethylamin, Triäthylamin, Tripropylamin, Chinuclidin, Pyridin, Chinolin, Isochinolin sowie Methanolamin, Aethanolamin, Propanolamin, Dimethanolamin, Diäthanolamin oder Triäthanolamin. Als organische Stickstoffbasen kommen auch quaternäre Ammoniumbasen in Betracht. Beispiele für quaternäre Ammoniumbasen sind Tetraalkylammoniumkationen, in den die Alkylreste unabhängig voneinander geradkettige oder verzweigte $C_1$-$C_6$-Alkylgruppen sind, wie das Tetramethylammoniumkation, das Tetraäthylammoniumkation oder das Trimethyläthylammoniumkation, sowie weiterhin das Trimethylbenzylammoniumkation, das Triäthylbenzylammoniumkation und das Trimethyl-2-hydroxyäthylammoniumkation. Besonders bevorzugt als Salzbildner sind das Ammoniumkation und Trialkylammoniumkationen, in denen die Alkylreste unabhängig voneinander geradkettige oder verzweigte, gegebenenfalls durch eine Hydroxylgruppe substituierte $C_1$-$C_6$-Alkylgruppen, insbesondere $C_1$-$C_2$-Alkylgruppen, sind, wie beispielsweise das Trimethylammoniumkation, das Triäthylammoniumkation und das Tri-(2-hydroxyäthylen)-ammoniumkation.

Für Z als Carbonsäureamidgruppe kommt ein entsprechender Amidrest in Betracht, welcher unsubstituiert oder am Stickstoffatom mono- oder disubstituiert sein kann oder in welchem das Stickstoffatom Bestandteil eines gegebenenfalls substituierten heterocyclischen Restes ist. Als Substituenten der Amidgruppe sind beispielsweise ein gegebenenfalls substituierter und gegebenenfalls über ein Sauerstoffatom gebundener aliphatischer Rest, ein gegebenenfalls über einen aliphatischen Rest gebundener und gegebenenfalls substituierter cycloaliphatischer, aromatischer oder heterocyclischer Rest oder eine gegebenenfalls mono- oder disubstituierte Aminogruppe zu nennen.

Als Carbonsäureamidrest bevorzugt ist der Rest -$CONR^{14}R^{15}$, worin $R^{14}$ für Wasserstoff, einen gegebenenfalls substituierten Alkyl-, Alkenyl-, Alkinyl- Cycloalkyl-, Phenyl- oder Naphthylrest, einen gegebenenfalls substituierten heterocyclischen Rest oder einen Alkoxyrest, $R^{15}$ für Wasserstoff, Amino, mono- oder disubstituiertes Amino oder einen gegebenenfalls substituierten Alkyl-, Alkenyl-, Cycloalkyl- oder Phenylrest oder $R^{14}$ und $R^{15}$ gemeinsam mit dem Stickstoffatom, an das sie gebunden sind, für einen gegebenenfalls substituierten heterocyclischen Rest stehen.

Als Substituenten der organischen Reste $R^{12}$, $R^{13}$, $R^{14}$ und $R^{15}$ kommen beispielsweise Halogen, Nitro, Cyan, Hydroxy, Alkyl, Halogenalkyl, Alkoxy, welches durch ein oder mehrere Sauerstoffatome unterbrochen sein kann, Alkylthio, Halogenalkoxy, Hydroxyalkoxy, welches durch ein oder mehrere Sauerstoffatome unterbrochen sein kann, Hydroxyalkylthio, Alkoxycarbonyl, Amino, Alkylamino, Dialkylamino, Hydroxyalkylamino, Di-(hydroxyalkyl)-amino, Aminoalkylamino, Cycloalkyl, gegebenenfalls substituiertes Phenyl, gegebenenfalls substituiertes Phenoxy oder ein gegebenenfalls substituierter heterocyclischer Rest in Betracht.

Unter heterocyclischen Resten als Bestandteile des Carbonsäureesterrestes, des Carbonsäurethiolesterrestes und des Carbonsäureamidrestes sind vorzugsweise 5- bis 6-gliedrige, gesättigte oder ungesättigte, gegebenenfalls substituierte monocyclische Heterocyclen mit 1 bis 3 Heteroatomen aus der Gruppe N, O und S zu verstehen, wie beispielsweise Furan, Tetrahydrofuran, Tetrahydropyran, Tetrahydropyrimidin, Pyridin, Piperidin, Morpholin und Imidazol.

Unter Cycloalkylresten als Bestandteile des Carbonsäureesterrestes, des Carbonsäurethiolesterrestes und des Carbonsäureamidrestes sind insbesondere solche mit 3 bis 8, vor allem 3 bis 6, Kohlenstoffatomen, zu verstehen,

Im Substituenten Z als Bestandteil des Carbonsäureesterrestes, des Carbonsäurethiolesterrestes und des Carbonsäureamidrestes vorliegende aliphatische, acyclische Reste können geradkettig oder verzweigt sein und enthalten zweckmässigerweise bis maximal 18 Kohlenstoffatome. Eine geringere Anzahl von Kohlenstoffatomen ist häufig, insbesondere bei zusammengesetzten Substituenten, von Vorteil.

Für Z als cyclisiertes Derivat einer Carbonsäureamidgruppe kommt insbesondere ein gegebenenfalls substituierter Oxazolin-2-yl-Rest, vorzugsweise ein unsubstituierter Oxazolin-2-yl-Rest, in Betracht.

A und Z können zusammen einen gegebenenfalls substituierten Tetrahydrofuran-2-on-Ring bilden, wobei der unsubstituierte Tetrahydrofuran-2-on-Ring bevorzugt ist, insbesondere der unsubstituierte Tetrahydrofuran-2-on-3-yl-Ring.

In den Verbindungen der Formel I bedeutet Halogen Fluor, Chlor, Brom und Jod, insbesondere Chlor, Brom und Jod.

Als Salzbildner für Säureadditionssalze kommen organische und anorganische Säuren in Betracht. Beispiele organischer Säuren sind Essigsäure, Trichloressigsäure, Oxalsäure, Benzolsulfonsäure und Methansulfonsäure. Beispiele anorganischer Säuren sind Chlorwasserstoffsäure, Bromwasserstoffsäure, Jodwasserstoffsäure, Schwefelsäure, Phosphorsäure, phosphorige Säure und Salpetersäure.

Als Metallkomplexbildner eignen sich beispielsweise Elemente der 3. und 4. Hauptgruppe, wie Aluminium und Zinn, sowie der 1. bis 8. Nebengruppe, wie beispielsweise Mangan, Eisen, Nickel, Zink, Kupfer und Silber. Bevorzugt sind die Nebengruppenelemente der 4. Periode.

Wenn in den Verbindungen der Formel I A für $-CH(CH_3)-$ steht, der Rest Z ein asymmetrisches Kohlenstoffatom enthält oder A und Z zusammen einen Tetrahydrofuran-2-on-Ring bilden, existieren optisch isomere Verbindungen. Im Rahmen der vorliegenden Erfindung sind unter den entsprechenden Verbindungen der Formel I sowohl die optisch reinen Isomere wie auch die Isomerengemische zu verstehen. Ist bei Vorhandensein eines oder mehrerer asymmetrischer Kohlenstoffatome die Struktur nicht näher angegeben, so ist stets das Isomerengemisch gemeint.

Besonders geeignet zur erfindungsgemässen Verwendung sind Verbindungen der Formel I, in denen $R^1$, $R^2$, $R^3$, $R^4$, $R^5$ und $R^6$ Wasserstoff bedeuten, $R^3$ für Wasserstoff oder Chlor und der Rest -A-Z für eine Gruppe $-CH_2-COOR^{16}$ oder $-CH(CH_3)-COOR^{16}$ steht, worin $R^{16}$ $C_1$-$C_{12}$-Alkyl, $C_3$-$C_6$-Alkenyl, Phenyl-$C_1$-$C_4$-alkyl oder Phenoxy-$C_1$-$C_4$-alkyl steht.

Als bevorzugte Einzelverbindungen der Formel I zur erfindungsgemässen Verwendung sind zu nennen:
2-Chinolin-8-yloxy-essigsäureisopropylester,
2-(5-Chlorchinolin-8-yloxy)-essigsäure-n-dodecylester,
2-(5-Chlorchinolin-8-yloxy)-essigsäure-n-butylester,
2-(5-Chlorchinolin-8-yloxy)-essigsäure-n-oktylester,
2-Chinolin-8-yloxy-essigsäure-s-butylester,
2-(5-Chlorchinolin-8-yloxy)-thioessigsäure-n-oktylester,
2-(5-Chlorchinolin-8-yloxy)-essigsäure-(2-butenyl)-ester,
2-(5-Chlorchinolin-8-yloxy)-essigsäuremethallylester,
2-(5-Chlorchinolin-8-yloxy)-essigsäure-(2-isopropyloxyäthyl)-ester,
2-(5-Chlorchinolin-8-yloxy)-essigsäure-(2-phenoxyäthyl)-ester,
2-(5-Chlorchinolin-8-yloxy)-essigsäure-(1-methylbutyl)-ester,
2-(5-Chlorchinolin-8-yloxy)-essigsäurecyclohexylester,
2-(5-Chlorchinolin-8-yloxy)-essigsäure-s-butylester,
2-(5-Chlorchinolin-8-yloxy)-essigsäure-(2-methylpentyl)-ester,
2-(5-Chlorchinolin-8-yloxy)-thioessigsäure-n-butylester,
2-(5-Chlorchinolin-8-yloxy)-essigsäure-(3,6-dioxadecyl)-ester,
2-(5-Chlorchinolin-8-yloxy)-essigsäure-(3-methoxybutyl)-ester,
2-(5-Chlorchinolin-8-yloxy)-essigsäure-(1-äthylbutyl)-ester,
2-(5-Chlorchinolin-8-yloxy)-essigsäure-(2-äthylbutyl)-ester,
2-(5-Chlorchinolin-8-yloxy)-essigsäure-(1-methylisopentyl)-ester,
2-(5-Chlorchinolin-8-yloxy)-essigsäure-n-undecylester,
2-(5-Chlorchinolin-8-yloxy)-essigsäure-(2-methylbutyl)-ester,
2-(5-Chlorchinolin-8-yloxy)-thioessigsäure-s-butylester,
2-(5-Chlorchinolin-8-yloxy)-essigsäure(3,6-dioxaheptyl)-ester,
2-(5-Chlorchinolin-8-yloxy)-essigsäure-n-heptylester,
2-(5-Chlorchinolin-8-yloxy)-thioessigsäure-n-docecylester,
2-(5-Chlorchinolin-8-yloxy)-thioessigsäure-n-decylester,
2-(5-Chlorchinolin-8-yloxy)-essigsäure-(1-propylpropargyl)-ester,
2-(5-Chlorchinolin-8-yloxy)-essigsäure-(1-methylisobutyl)-ester,
2-(5-Chlorchinolin-8-yloxy)-thioessigsäure-tert.butylester,
2-(5-Chlorchinolin-8-yloxy)-essigsäure-neopentylester,
2-(5-Chlorchinolin-8-yloxy)-thioessigsäure-n-propylester,
2-(5-Chlorchinolin-8-yloxy)-essigsäure-(1-methylhexyl)-ester,
2-(5-Chlorchinolin-8-yloxy)-thioessigsäureäthylester,
2-(5-Chlorchinolin-8-yloxy)-essigsäure-(2-äthylhexyl)-ester,
2-(5-Chlorchinolin-8-yloxy)-thioessigsäure-i-butylester,
2-Chinolin-8-yloxy-thioessigsäure-n-decylester,
2-(5-Chlorchinolin-8-yloxy)-essigsäure-i-pentylester,
2-(5-Chlorchinolin-8-yloxy)-essigsäure-(1-äthylpentyl)-ester,
2-(5-Chlorchinolin-8-yloxy)-essigsäure-(1-propylbutyl)-ester,
2-(5-Chlorchinolin-8-yloxy)-thioessigsäure-n-hexylester,
2-(5-Chlorchinolin-8-yloxy)-essigsäure-n-hexylester,
2-(5-Chlorchinolin-8-yloxy)-thioessigsäure-i-propylester,
2-(5-Chlorchinolin-8-yloxy)-essigsäure-(1-pentyallyl)-ester,

2-(5-Chlorchinolin-8-yloxy)-essigsäure-(1-methylpentyl)-ester,
2-(5-Chlorchinolin-8-yloxy)-essigsäure-(1,1-dimethylpropargyl)-ester,
2-(5-Chlorchinolin-8-yloxy)-essigsäure-(1-äthyl-1-methylpropargyl)-ester,
2-(5-Chlorchinolin-8-yloxy)-essigsäure-n-butyloxycarbonylmetnhyl-ester,
2-(5-Chlorchinolin-8-yloxy)-essigsäure-(1-n-butyloxycarbonyläthyl)-ester,
2-(5-Chlorchinolin-8-yloxy)-essigsäure-(1-methylisohexyl)-ester,
2-(5-Chlorchinolin-8-yloxy)-essigsäure-(1-phenylisobutyl)-ester,
2-(5-Chlorchinolin-8-yloxy)-essigsäure-[1-methyl-2-(2-methylphenoxy)-äthyl]-ester,
2-(5-Chlorchinolin-8-yloxy)-essigsäure-(1-phenyläthyl)-ester,
2-(5-Chlorchinolin-8-yloxy)-essigsäure-[1-methyl-2-(4-äthylphenoxy)-äthyl]-ester,
2-(5-Chlorchinolin-8-yloxy)-essigsäure-(1-methyl-2-phenyläthyl)-ester,
2-(5-Chlorchinolin-8-yloxy)-essigsäure-[1-methyl-2-(2-isopropylphenoxy)-äthyl]-ester,
2-(5-Chlorchinolin-8-yloxy)-essigsäure-(1-phenylpropyl)-ester,
2-(5-Chlorchinolin-8-yloxy)-essigsäure-[1-methyl-2-(2-äthylphenoxy)-äthyl]-ester,
2-(5-Chlorchinolin-8-yloxy)-essigsäure-[1-methyl-2-(3-äthylphenoxy)-äthyl]-ester,
2-(5-Chlorchinolin-8-yloxy)-essigsäure-(1-methyl-2-phenoxyäthyl)-ester,
2-(5-Chlorchinolin-8-yloxy)-essigsäure-(1-methyl-3-phenylpropyl)-ester,
2-(5-Chlorchinolin-8-yloxy)-essigsäure-[1-methyl-2-(3-methylphenoxy)-äthyl]-ester,
2-(5-Chlorchinolin-8-yloxy)-essigsäure[1-methyl-2-(4-isopropylphenoxy)-äthyl]-ester und
2-(5-Chlorchinolin-8-yloxy)-essigsäure-[1-methyl-2-(4-methylphenoxy)-äthyl]-ester.

Hervorzuheben ist im Rahmen der vorliegenden Erfindung besonders die Verwendung von:
2-(5-Chlorchinolin-8-yloxy)-essigsäuremethallylester,
2-(5-Chlorchinolin-8-yloxy)-essigsäure-(2-phenoxyäthyl)-ester,
2-(5-Chlorchinolin-8-yloxy)-essigsäure-(1-methylbutyl)-ester,
2-(5-Chlorchinolin-8-yloxy)-essigsäure-(1-äthylbutyl)-ester,
2-(5-Chlorchinolin-8-yloxy)-essigsäure-(1-methylisopentyl)-ester,
2-(5-Chlorchinolin-8-yloxy)-essigsäure-(1-methylhexyl)-ester,
2-(5-Chlorchinolin-8-yloxy)-essigsäure-(1-äthylpentyl)-ester,
2-(5-Chlorchinolin-8-yloxy)-essigsäure-(1-propylbutyl)-ester,
2-(5-Chlorchinolin-8-yloxy)-essigsäure-(1-pentylallyl)-ester,
2-(5-Chlorchinolin-8-yloxy)-essigsäure-(1-methylpentyl)-ester,
2-(5-Chlorchinolin-8-yloxy)-essigsäure-(1-methylisohexyl)-ester,
2-(5-Chlorchinolin-8-yloxy)-essigsäure-(1-phenylisobutyl)-ester,
2-(5-Chlorchinolin-8-yloxy)-essigsäure-(1-phenyläthyl)-ester,
2-(5-Chlorchinolin-8-yloxy)-essigsäure-[1-methyl-2-(4-äthylphenoxy)-äthyl]-ester,
2-(5-Chlorchinolin-8-yloxy)-essigsäure-(1-methyl-2-phenyläthyl)-ester,
2-(5-Chlorchinolin-8-yloxy)-essigsäure-(1-phenylpropyl)-ester,
2-(5-Chlorchinolin-8-yloxy)-essigsäure-(1-methyl-2-phenoxyäthyl)-ester,
2-(5-Chlorchinolin-8-yloxy)-essigsäure-(1-methyl-3-phenylpropyl)-ester und
2-(5-Chlorchinolin-8-yloxy)-essigsäure-[1-methyl-2-(4-methylphenoxy)-äthyl]-ester.

Als ganz besonders wirksam haben sich für diesen Zweck die folgenden Verbindungen erwiesen:
2-(5-Chlorchinolin-8-yloxy)-essigsäuremethallylester,
2-(5-Chlorchinolin-8-yloxy)-essigsäure-(2-phenoxyäthyl)-ester,
2-(5-Chlorchinolin-8-yloxy)-essigsäure-(1-methylisopentyl)-ester
und
2-(5-Chlorchinolin-8-yloxy)-essigsäure-(1-methylhexyl)-ester.

Beispiele für erfindungsgemäss zu verwendende Verbindungen mit Schutzwirkung gegen herbizid wirksame 2-[4-(5-Chlor-3-fluorpyridin-2-yloxy)-phenoxy]-propionsäure-Derivate zeigt die nachfolgende Tabelle 1.

Tabelle 1:

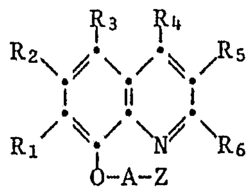

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.1 | H | H | H | H | H | H | $-CH_2-$ | $-CN$ | Smp. 118-119°C |
| 1.2 | H | H | H | H | H | H | $-CH_2-$ | 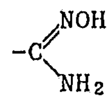 | Smp. 201-204°C (Zers.) |
| 1.3 | H | H | H | H | H | $CH_3$ | $-CH_2-$ | $-CN$ | Smp. 114-116°C |
| 1.4 | H | H | H | H | H | $CH_3$ | $-CH_2-$ | 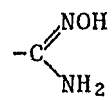 | Smp. 209-210°C (Zers.) |
| 1.5 | H | H | Cl | H | H | H | $-CH_2-$ | 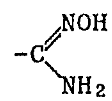 | Smp. 203-205°C (Zers.) |
| 1.6 | H | H | H | H | H | H | $-CH_2-$ | 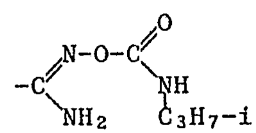 | Smp. 136-138°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.7 | H | H | Cl | H | H | H | $-CH_2-$ | $-CN$ | Smp. 159-160°C |
| 1.8 | H | H | H | H | H | H | $-CH_2-$ | 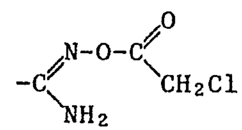 | Smp. 129-130°C |
| 1.9 | Br | H | Cl | H | H | H | $-CH_2-$ | 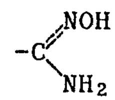 | Smp. 197-198°C (Zers.) |
| 1.10 | Br | H | Cl | H | H | H | $-CH_2-$ | $-CN$ | Smp. 150-151°C |
| 1.11 | H | H | H | H | H | H | $-CH_2-$ | 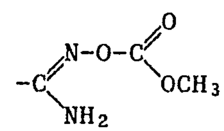 | Smp. 143-145°C |
| 1.12 | J | H | Cl | H | H | H | $-CH_2-$ | 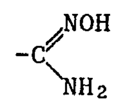 | Smp. 195-196°C (Zers.) |
| 1.13 | J | H | Cl | H | H | H | $-CH_2-$ | $-CN$ | Smp. 150,5-152°C |
| 1.14 | Br | H | Cl | H | H | H | $-CH_2-$ | 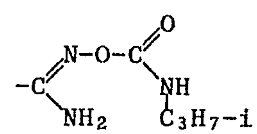 | Smp. 162-165°C |

<u>Tabelle 1</u> (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.15 | Cl | H | Cl | H | H | $CH_3$ | $-CH_2-$ | 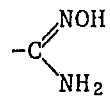 | Smp. 205-207°C (Zers.) |
| 1.16 | Cl | H | Cl | H | H | H | $-CH_2-$ | -CN | Smp. 150-152°C |
| 1.17 | J | H | Cl | H | H | H | $-CH_2-$ | 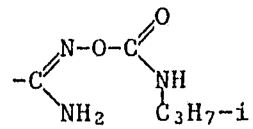 | Smp. 163-167°C |
| 1.18 | Cl | H | Cl | H | H | $CH_3$ | $-CH_2-$ | -CN | Smp. 157-158°C |
| 1.19 | Cl | H | Cl | H | H | $CH_3$ | $-CH_2-$ | 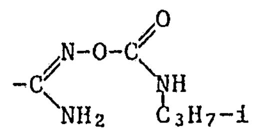 | Smp. 149-152°C |
| 1.20 | H | H | H | H | H | H | 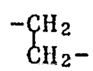 | -CN | Smp. 108-112°C |
| 1.21 | H | H | H | H | H | H | 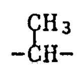 | -CN | Smp. 121-124°C |
| 1.22 | H | H | H | H | H | H | 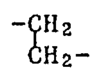 | 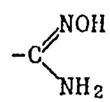 | Smp. 186-189°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.23 | H | H | Cl | H | H | H | 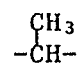 | -CN | Smp. 143-145° |
| 1.24 | H | H | H | H | H | H | 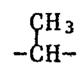 | 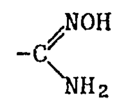 | Smp. 191-194°C (Zers.) |
| 1.25 | H | H | Cl | H | H | H | 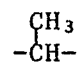 | 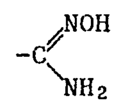 | Smp. 186-189°C (Zers.) |
| 1.26 | H | H | $NO_2$ | H | H | H | 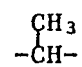 | -CN | Smp. 154-156°C |
| 1.27 | Cl | H | $NO_2$ | H | H | H | $-CH_2-$ | 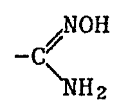 | Smp. 214-216°C (Zers.) |
| 1.28 | Cl | H | $NO_2$ | H | H | H | $-CH_2-$ | -CN | Smp. 166-169°C |
| 1.29 | H | H | H | H | H | H | $-CH_2-$ | 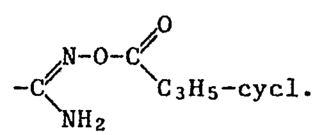 | Smp. 165-166°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.30 | H | H | H | H | H | H | $-CH_2-$ | 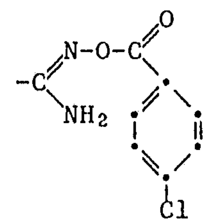 | Smp. 139-141°C |
| 1.31 | H | H | Cl | H | H | H | $-CH_2-$ | 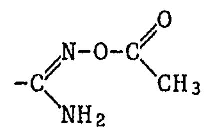 | Smp. 141-143°C |
| 1.32 | H | H | $NO_2$ | H | H | H | $-CH_2-$ | -CN | Smp. 162-164°C |
| 1.33 | H | H | $NO_2$ | H | H | H | $-CH_2-$ | 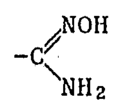 | Smp. 212-215°C (Zers.) |
| 1.34 | H | H | Cl | H | H | H | $-CH_2-$ | 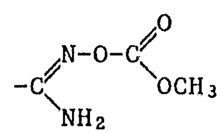 | Smp. 148-149°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.35 | H | H | Cl | H | H | H | $-CH_2-$ | 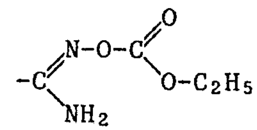 | Smp. 139-140°C |
| 1.36 | H | H | H | H | H | H | $-CH_2-$ | 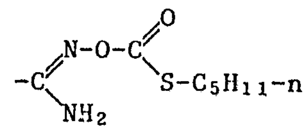 | Smp. 111-114°C |
| 1.37 | H | H | H | H | H | H | $-CH_2-$ | 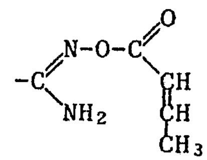 | Smp. 158-162°C |
| 1.38 | H | H | H | H | H | H | $-CH_2-$ | 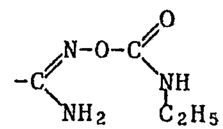 | Smp. 123-125°C |
| 1.39 | H | H | H | H | H | H | $-CH_2-$ | 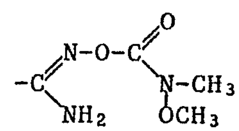 | Smp. 138-139°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.40 | H | H | H | H | H | H | $-CH_2-$ | 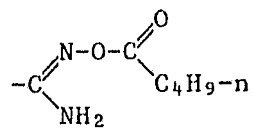 | Smp. 120-122°C |
| 1.41 | H | H | Cl | H | H | H | $-CH_2-$ | 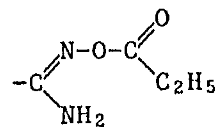 | Smp. 157-158°C (Zers.) |
| 1.42 | H | H | H | H | H | H | $-CH_2-$ | 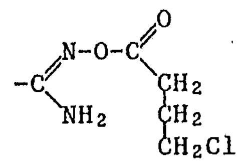 | Smp. 144-146°C |
| 1.43 | H | H | H | H | H | H | $-CH_2-$ | 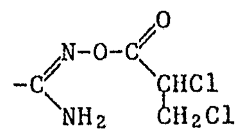 | Smp. 112-114°C |
| 1.44 | H | H | Cl | H | H | H | $-CH_2-$ | 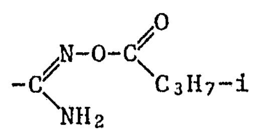 | Smp. 173-174°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.45 | H | H | H | H | H | H | $-CH_2-$ | 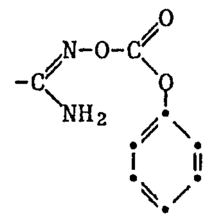 | Smp. 155-156°C |
| 1.46 | H | H | H | H | H | H | $-CH_2-$ | 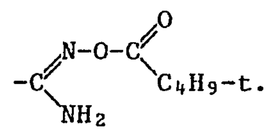 | Smp. 107-110,5°C |
| 1.47 | H | H | H | H | H | H | $-CH_2-$ | 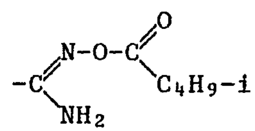 | Smp. 124-126°C |
| 1.48 | H | H | H | H | H | H | $-CH_2-$ | 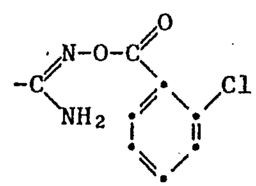 | Smp. 131-132°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.49 | H | H | H | H | H | H | $-CH_2-$ | 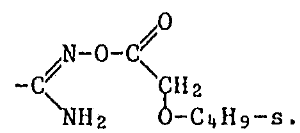 | Smp. 84-86°C |
| 1.50 | H | H | H | H | H | H | $-CH_2-$ | 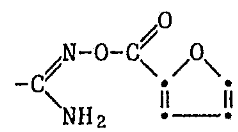 | Smp. 168-169°C |
| 1.51 | H | H | H | H | H | H | $-CH_2-$ | 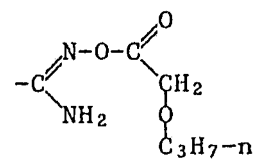 | Smp. 100-103°C |
| 1.52 | H | H | Cl | H | H | H | $-CH_2-$ | 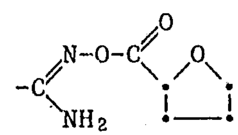 | Smp. 156-157°C (Zers.) |
| 1.53 | H | H | H | H | H | H | $-CH_2-$ | 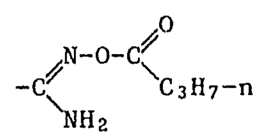 | Smp. 82-85°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.54 | H | H | H | H | H | H | $-CH_2-$ | 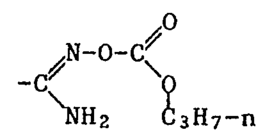 | Smp. 144-147°C |
| 1.55 | H | H | H | H | H | H | $-CH_2-$ | 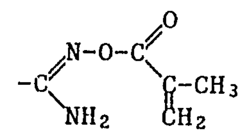 | Smp. 128-130°C |
| 1.56 | H | H | H | H | H | H | $-CH_2-$ | 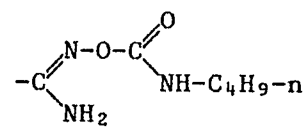 | Smp. 104-107°C |
| 1.57 | H | H | H | H | H | H | $-CH_2-$ | 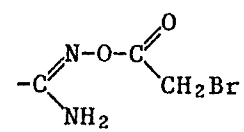 | Smp. 132-134°C |
| 1.58 | H | H | H | H | H | H | $-CH_2-$ | 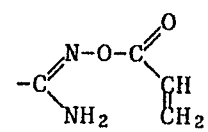 | Smp. 138-140°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.59 | H | H | H | H | H | H | $-CH_2-$ | 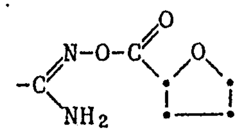 | Smp. 129-131°C |
| 1.60 | H | H | H | H | H | H | $-CH_2-$ | 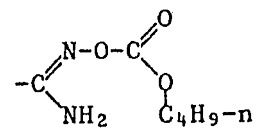 | Smp. 121-123°C |
| 1.61 | H | H | H | H | H | H | $-CH_2-$ | 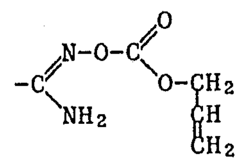 | Smp. 123-125°C |
| 1.62 | H | H | H | H | H | H | $-CH_2-$ | 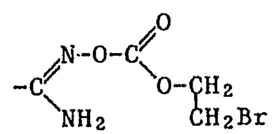 | Smp.127-128°C (Zers.) |
| 1.63 | H | H | Cl | H | H | H | $-CH_2-$ | 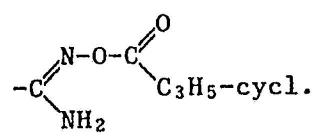 | Smp. 173-175°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.64 | H | H | H | H | H | H | $-CH_2-$ | 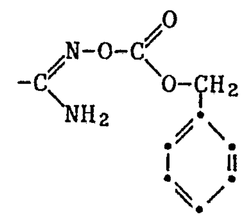 | Smp. 135-137°C |
| 1.65 | H | H | Cl | H | H | H | $-CH_2-$ | 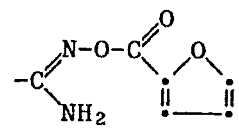 | Smp.191-192°C (Zers.) |
| 1.66 | H | H | H | H | H | H | $-CH_2-$ | 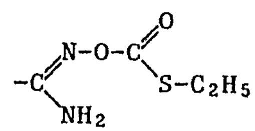 | Smp. 120-121°C |
| 1.67 | H | H | H | H | H | H | $-CH_2-$ | 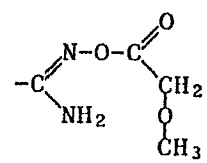 | Smp. 118-120°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.68 | H | H | Cl | H | H | H | $-CH_2-$ | 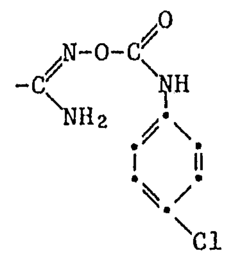 | Smp.191-192°C (Zers.) |
| 1.69 | H | H | H | H | H | H | $-CH_2-$ | 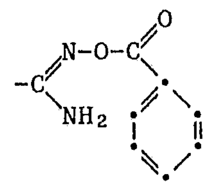 | Smp. 158-159°C |
| 1.70 | H | H | H | H | H | H | $-CH_2-$ | 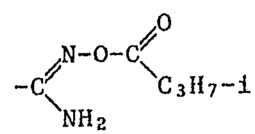 | Smp. 115-117,5°C |
| 1.71 | H | H | H | H | H | H | $-CH_2-$ | 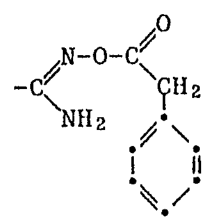 | Smp. 140-142°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.72 | H | H | H | H | H | H | $-CH_2-$ | 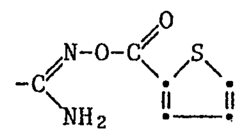 | Smp. 164-165°C |
| 1.73 | H | H | H | H | H | H | $-CH_2-$ | 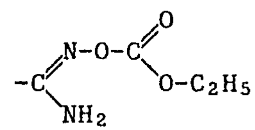 | Smp. 129-132°C |
| 1.74 | H | H | H | H | H | H | $-CH_2-$ | 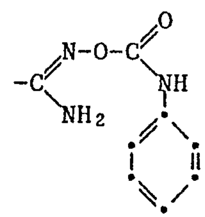 | Smp. 155-157,5°C |
| 1.75 | H | H | H | H | H | H | $-CH_2-$ | 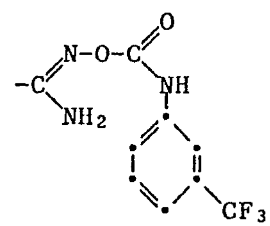 | Smp. 158-160°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.76 | H | H | Cl | H | H | H | $-CH_2-$ | 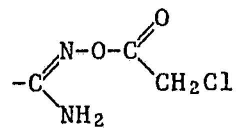 | Smp. 155-158°C (Zers.) |
| 1.77 | H | H | H | H | H | H | $-CH_2-$ | 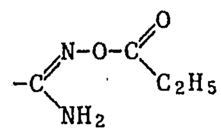 | Smp. 144-146°C |
| 1.78 | H | H | H | H | H | H | $-CH_2-$ | 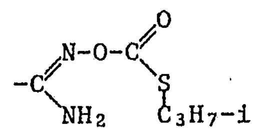 | Smp. 123-124°C |
| 1.79 | H | H | H | H | H | H | $-CH_2-$ | 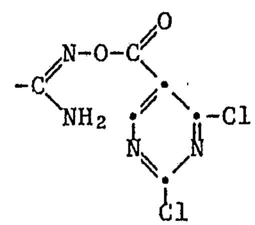 | Smp. 173-176°C (Zers.) |
| 1.80 | H | H | H | H | H | H | $-CH_2-$ | 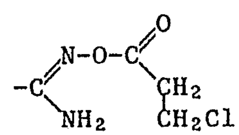 | Smp. 134-136°C (Zers.) |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.81 | H | H | H | H | H | H | $-CH_2-$ | 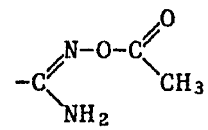 | Smp. 100-102°C |
| 1.82 | H | H | H | H | H | H | $-CH_2-$ | 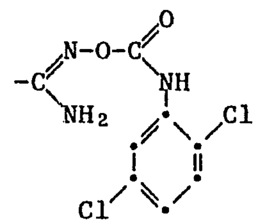 | Smp. 197-199°C |
| 1.83 | H | H | H | H | H | H | $-CH_2-$ | 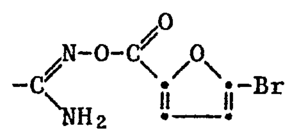 | Smp. 170-171°C |
| 1.84 | Cl | H | Cl | H | H | H | $-\underset{CH_3}{CH}-$ | $-COOCH_3$ | Smp. 65-66°C |
| 1.85 | H | H | H | H | H | H | $-\underset{CH_3}{CH}-$ | $-COOCH_3$ | Smp. 70-72°C |
| 1.86 | H | H | H | H | H | H | $-CH_2-$ | $-COOH \cdot H_2O$ | Smp. 184-185°C |
| 1.87 | H | H | H | H | H | H | $-CH_2-$ | $-COOCH_2CH_2OCH_3$ | Smp. 80-82°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.88 | H | H | H | H | H | H | $-CH_2-$ | $-COOCH_3$ | Smp. 46,5-67,0°C |
| 1.89 | H | H | H | H | H | H | $-CH_2-$ | $-COOC_2H_5 \cdot H_2O$ | Smp. 56-59°C |
| 1.90 | H | H | H | H | H | H | $-CH(CH_3)-$ | $-CONH(CH_2)_3OC_2H_5$ | Smp. 54-56°C |
| 1.91 | H | H | H | H | H | H | $-CH(CH_3)-$ | $-CONHC_2H_5$ | Smp. 86-88°C |
| 1.92 | H | H | H | H | H | H | $-CH_2-$ | $-COOC_3H_7$-n | Smp. 28-31°C |
| 1.93 | H | H | H | H | H | H | $-CH_2-$ | $-COOC_3H_7$-i | $n_D^{23} = 1.5696$ |
| 1.94 | H | H | H | H | H | H | $-CH_2-$ | $-CONHCH_3 \cdot H_2O$ | Smp. 74-81°C |
| 1.95 | H | H | H | H | H | H | $-CH_2-$ | $-CON(CH_3)_2$ | Smp. 142-145°C |
| 1.96 | H | H | H | H | H | H | $-CH_2-$ | $-CONHC_2H_5$ | $n_D^{22,5} = 1.6002$ |
| 1.97 | H | H | H | H | H | H | $-CH(CH_3)-$ | $-CONH(CH_2)_3OH$ | Smp. 120-122°C |
| 1.98 | H | H | H | H | H | H | $-CH_2-$ | $-COOCH_2CH_2OC_2H_5$ | $n_D^{24} = 1.5673$ |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.99 | H | H | H | H | H | H | 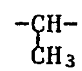 | $-CONHCH_2-$ 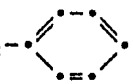 | Smp. 88-90°C |
| 1.100 | H | H | H | H | H | H | $-CH_2-$ | $-CONH(CH_2)_3CH_3$ | Smp. 66-68°C |
| 1.101 | H | H | H | H | H | H | 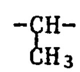 | 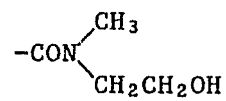 | $n_D^{22}$ = 1.6054 |
| 1.102 | H | H | H | H | H | H | $-CH_2-$ | 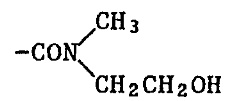 | Smp. 146-149°C |
| 1.103 | H | H | H | H | H | H | $-CH_2-$ | $-COOCH_2-$ 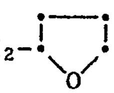 | zähe Masse |
| 1.104 | H | H | H | H | H | H | 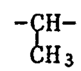 | $-CONH(CH_2)_3CH_3 \cdot H_2O$ | Smp. 73-76°C |
| 1.105 | H | H | H | H | H | H | 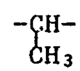 | -CO- 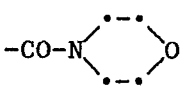 | Smp. 120-121°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.106 | H | H | H | H | H | H | 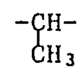 | 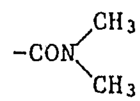 | Smp. 105-111°C |
| 1.107 | H | H | Cl | H | H | H | $-CH_2-$ | $-COOH$ | Smp. 232-233°C |
| 1.108 | H | H | Cl | H | H | H | $-CH_2-$ | $-COOCH_2CH_2OCH_3$ | Smp. 97-98°C |
| 1.109 | H | H | Cl | H | H | H | $-CH_2-$ | $-COOCH_3$ | Smp. 104-105,5°C |
| 1.110 | H | H | Cl | H | H | H | $-CH_2-$ | $-COOC_2H_5$ | Smp. 116-117°C |
| 1.111 | H | H | Cl | H | H | H | $-CH_2-$ | $-COOC_3H_7$-n | Smp. 108-109°C |
| 1.112 | H | H | Cl | H | H | H | $-CH_2-$ | 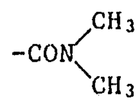 | Smp. 135-136°C |
| 1.113 | H | H | H | H | H | $CH_3$ | $-CH_2-$ | $-COOCH_3$ | Smp. 58-66°C |
| 1.114 | H | H | H | H | H | $CH_3$ | $-CH_2-$ | $-COOC_2H_5$ | $n_D^{22,5}$ = 1.5762 |
| 1.115 | H | H | Cl | H | H | H | $-CH_2-$ | $-COOC_4H_9$-t | Smp. 63-69°C |
| 1.116 | H | H | H | H | H | H | $-CH_2-$ | $-COOC_4H_9$-t | Smp. 68-70°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.117 | H | H | Cl | H | H | H | $-CH_2-$ | $-COOCH_2-C{\equiv}CH$ | Smp. 115-116°C |
| 1.118 | H | H | Cl | H | H | H | $-CH_2-$ | $-COOC_3H_7-i$ | Smp. 147-148°C |
| 1.119 | H | H | Cl | H | H | H | $-CH_2-$ | $-COOCH_2CH_2OC_2H_5$ | Smp. 102-104°C |
| 1.120 | H | H | Cl | H | H | H | $-CH_2-$ | $-COOCH_2-$ 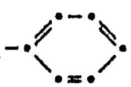 | Smp. 110-112°C |
| 1.121 | H | H | Cl | H | H | H | $-CH_2-$ | $-COOCH_2-CH{=}CH_2$ | Smp. 98-99°C |
| 1.122 | H | H | Cl | H | H | H | $-CH_2-$ | $-COO(CH_2)_{11}CH_3$ | Smp. 76-77°C |
| 1.123 | H | H | Cl | H | H | H | $-CH_2-$ | $-COOC_4H_9-s$ | Smp. 110-111°C |
| 1.124 | H | H | H | H | H | H | $-CH_2-$ | $-COO(CH_2)_7CH_3$ | $n_D^{24} = 1.5419$ |
| 1.125 | H | H | Cl | H | H | H | $-CH_2-$ | $-COOC_4H_9n$ | Smp. 90,5-92°C |
| 1.126 | H | H | H | H | H | H | $-CH_2-$ | $-COO(CH_2)_{11}CH_3$ | $n_D^{23} = 1.5232$ |
| 1.127 | H | H | H | H | H | H | $-CH_2-$ | $-COOCH_2-CH{=}CH_2$ | $n_D^{23} = 1.5885$ |
| 1.128 | H | H | Cl | H | H | H | $-CH_2-$ | $-COO(CH_2)_7CH_3$ | Smp. 87-88°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.129 | H | H | H | H | H | H | $-CH_2-$ | $-COOC_4H_9$-n | $n_D^{22}$ = 1.5642 |
| 1.130 | H | H | H | H | H | H | $-CH_2-$ | $-COOC_4H_9$-s | rotes Oel |
| 1.131 | H | H | Cl | H | H | H | $-CH_2-$ | $-COOCH_2CH_2Cl$ | Smp. 125-126°C |
| 1.132 | H | H | H | H | H | H | $-CH_2-$ | $-COOCH_2-$ 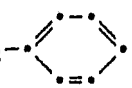 | $n_D^{23,5}$ = 1.6099 |
| 1.133 | H | H | Cl | H | H | H | $-CH_2-$ | $-COOCH_2-$ 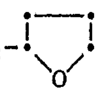 | Smp. 101-103°C |
| 1.134 | H | H | Cl | H | H | H | $-CH_2-$ | $-COS(CH_2)_7CH_3$ | Smp. 53-54°C |
| 1.135 | H | H | H | H | H | H | $-CH_2-$ | $-COOCH_2CH_2Cl$ | Smp. 109-110°C |
| 1.136 | J | H | Cl | H | H | H | $-CH_2-$ | $-COOC_4H_9$-t | Smp. 81-97°C |
| 1.137 | J | H | Cl | H | H | H | $-CH_2-$ | $-COOC_2H_5$ | Smp. 92-94°C |
| 1.138 | J | H | Cl | H | H | H | $-CH_2-$ | $-COO(CH_2)_{11}CH_3$ | Smp. 51-53°C |
| 1.139 | J | H | Cl | H | H | H | $-CH_2-$ | $-COOCH_3$ | Smp. 121-126°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.140 | J | H | Cl | H | H | H | $-CH_2-$ | $-COOCH_2CH_2Cl$ | Smp. 44-45°C |
| 1.141 | J | H | Cl | H | H | H | $-CH_2-$ | $-COOCH_2-$ 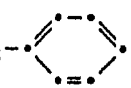 | Smp. 112-113°C |
| 1.142 | J | H | Cl | H | H | H | $-CH_2-$ | $-COOC_3H_7-n$ | Smp. 71-73°C |
| 1.143 | H | H | H | H | H | H | $-CH_2-$ | $-COOC_4H_9-i$ | $n_D^{22} = 1.5632$ |
| 1.144 | H | H | H | H | H | H | $-CH_2-$ | $-COOCHCH_2CH_2CH_3$<br>$CH_3$ | $n_D^{22} = 1.5391$ |
| 1.145 | H | H | H | H | H | H | $-CH_2-$ | $-COOCH(CH_2)_5CH_3$<br>$CH_3$ | $n_D^{22} = 1.5342$ |
| 1.146 | H | H | H | H | H | H | $-CH_2-$ | $-CONH(CH_2)_{11}CH_3$ | Smp. 56-61°C |
| 1.147 | H | H | H | H | H | H | $-CH_2-$ | $-CONHCH_2CH_2-$ 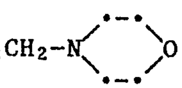 | Smp. 94-99°C |
| 1.148 | H | H | H | H | H | H | $-CH_2-$ | $-CONHCH_2CH_2CH_2OH$ | Smp. 138-139°C |
| 1.149 | H | H | H | H | H | H | $-CH_2-$ | $-CONH-$ 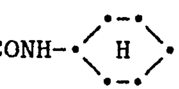 | Smp. 104-106°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.150 | H | H | H | H | H | H | $-CH_2-$ | 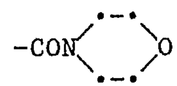 | Smp. 99-103°C |
| 1.151 | H | H | H | H | H | H | $-CH_2-$ | 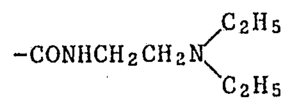 | $n_D^{23}$ = 1.5686 |
| 1.152 | H | H | H | H | H | H | $-CH_2-$ | 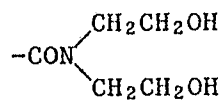 | Smp. 144-146°C |
| 1.153 | H | H | H | H | H | H | $-CH_2-$ | 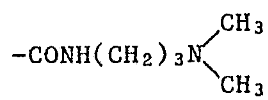 | $n_D^{23}$ = 1.5766 |
| 1.154 | H | H | H | H | H | H | $-CH_2-$ | 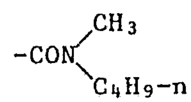 | $n_D^{22}$ = 1.5840 |
| 1.155 | H | H | H | H | H | H | $-CH_2-$ | 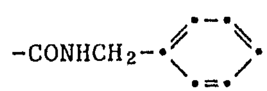 • $H_2O$ | Smp. 70,5-73,5°C |
| 1.156 | H | H | H | H | H | H | $-CH_2-$ | 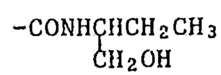 | Smp. 150-151°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.157 | H | H | H | H | H | H | $-CH_2-$ | 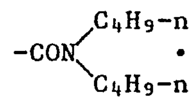 • $2H_2O$ | Smp. 105-106°C |
| 1.158 | H | H | H | H | H | H | $-CH_2-$ | 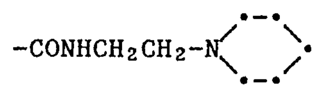 | $n_D^{26}$ = 1.5821 |
| 1.159 | H | H | H | H | H | H | $-CH_2-$ | 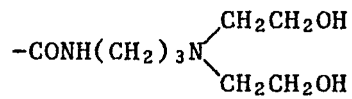 | Smp. 109-110°C |
| 1.160 | H | H | H | H | H | H | $-CH_2-$ | $-CONHCH_2-CH=CH_2$ • $H_2O$ | Smp. 71-75°C |
| 1.161 | H | H | H | H | H | H | $-CH_2-$ | 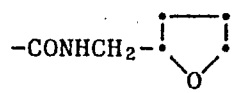 • $H_2O$ | Smp. 57-58°C |
| 1.162 | H | H | H | H | H | H | $-CH_2-$ | $-CONH(CH_2)_3OC_2H_5$ | Smp. 51-61°C |
| 1.163 | H | H | H | H | H | H | $-CH_2-$ | $-CONHCH_2CH_2NHCH_2CH_2OH$ | Smp. 70-91°C |
| 1.164 | H | H | Cl | H | H | H | $-CH_2-$ | $CONH(CH_2)_3OC_2H_5$ | Smp. 85-88°C |
| 1.165 | H | H | Cl | H | H | H | $-CH_2-$ | 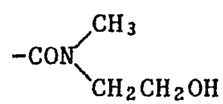 | Smp. 187-189°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.166 | H | H | Cl | H | H | H | $-CH_2-$ | 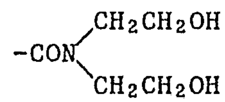 | Smp. 177-179°C |
| 1.167 | H | H | Cl | H | H | H | $-CH_2-$ | 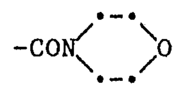 | Smp. 148-150°C |
| 1.168 | H | H | Cl | H | H | H | $-CH_2-$ | $-CONHCH_2CH_2CH_2OH$ | Smp. 157-160°C |
| 1.169 | H | H | Cl | H | H | H | $-CH_2-$ | $-CONHC_4H_9-n \cdot H_2O$ | Smp. 87-90°C |
| 1.170 | H | H | Cl | H | H | H | $-CH_2-$ | $-CONHC_2H_5$ | Smp. 94-98°C |
| 1.171 | H | H | Cl | H | H | H | $-CH_2-$ | $-CONHCH_2-$ 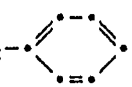 $\cdot \frac{1}{2} H_2O$ | Smp. 146-149°C |
| 1.172 | H | H | H | H | H | $CH_3$ | $-CH_2-$ | $-CONH_2$ | Smp. 193-196°C |
| 1.173 | H | H | H | H | H | H | $-CH_2-$ | $-CONHNH_2 \cdot H_2O$ | Smp. 121-124°C |
| 1.174 | H | H | H | H | H | H | $-CH_2-$ | $-COONa \cdot H_2O$ | Smp. 140-142°C |
| 1.175 | H | H | H | H | H | H | $-CH_2-$ | $-COOK \cdot H_2O$ | Smp. > 200°C |
| 1.176 | H | H | H | H | H | H | $-CH_2-$ | $-COO^{\ominus}\ \overset{\oplus}{H}N(CH_3)_3$ | Smp. 176-178°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.177 | H | H | H | H | H | H | $-CH_2-$ | $-COO^{\ominus}\ \overset{\oplus}{H}N(CH_2CH_2OH)_3$ | Smp. 97-98°C |
| 1.178 | H | H | Cl | H | H | H | $-CH_2-$ | $-COOK \cdot H_2O$ | Smp. > 260°C |
| 1.179 | H | H | Cl | H | H | H | $-CH_2-$ | $-COONa \cdot H_2O$ | Smp. > 260°C |
| 1.180 | H | H | H | H | H | H | $-CH_2-$ | $-COO^{\ominus}\ \overset{\oplus}{H}N(C_2H_5)_3$ | Smp. 255-257°C (Zers.) |
| 1.181 | H | H | Cl | H | H | H | $-CH_2-$ | $-COO^{\ominus}\ {}^{\oplus}NH_4$ | Smp. 227-228°C (Zers.) |
| 1.182 | H | H | Cl | H | H | H | $-CH_2-$ | $-COO^{\ominus}\ \overset{\oplus}{H}N(CH_2CH_2OH)_3$ | Smp. 132-156°C (Zers.) |
| 1.183 | H | H | Cl | H | H | H | $-CH(CH_3)-$ | 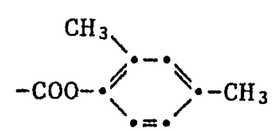 | Smp. 120-122°C |
| 1.184 | H | H | Cl | H | H | H | $-CH_2-$ | $-COOCH(CH_3)(CH_2)_5CH_3$ | Smp. 65-67°C |
| 1.185 | H | H | Cl | H | H | H | $-CH_2-$ | $-COOCH_2CH{=}CH-CH_3$ | Smp. 100-102°C |
| 1.186 | H | H | Cl | H | H | H | $-CH_2-$ | $-COOCH_2-C(CH_3){=}CH_2$ | Smp. 94-95°C |

<u>Tabelle 1</u> (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.187 | H | H | Cl | H | H | H | $-CH_2-$ | $-COOCH_2CH_2OC_3H_7-i$ | Smp. 70-72°C |
| 1.188 | H | H | Cl | H | H | H | $-CH_2-$ | $-COOCH_2CH_2-O-$ 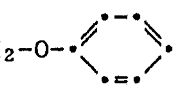 | Smp. 79-80,5°C |
| 1.189 | Br | H | Br | H | H | H | $-CH_2-$ | $-COOCH_3$ | Smp. 143-145°C |
| 1.190 | Br | H | Cl | H | H | H | $-CH_2-$ | $-COOC_3H_7-i$ | Smp. 71-73°C |
| 1.191 | Br | H | Br | H | H | H | $-CH_2-$ | $-COOC_3H_7-i$ | Smp. 47-51°C |
| 1.192 | Cl | H | Cl | H | H | H | $-CH_2-$ | $-COOC_4H_9-n$ | Smp. 42-43,5°C |
| 1.193 | Br | H | Cl | H | H | H | $-CH_2-$ | $-COOC_4H_9-n$ | Smp. ca. 28°C |
| 1.194 | Cl | H | Cl | H | H | H | $-CH_2-$ | $-COO(CH_2)_7CH_3$ | Smp. ca. 30°C |
| 1.195 | Br | H | Cl | H | H | H | $-CH_2-$ | $-COO(CH_2)_7CH_3$ | Smp. 41-42°C |
| 1.196 | Br | H | Cl | H | H | H | $-CH_2-$ | $-COO\overset{CH_3}{C}H(CH_2)_5CH_3$ | Smp. 46-48°C |
| 1.197 | Cl | H | Cl | H | H | H | $-CH_2-$ | $-COO(CH_2)_{11}CH_3$ | Smp. 49-50°C |
| 1.198 | Br | H | Cl | H | H | H | $-CH_2-$ | $-COO(CH_2)_{11}CH_3$ | Smp. 50-52°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.199 | Cl | H | Cl | H | H | H | $-CH_2-$ | $-COOCH_2-$ 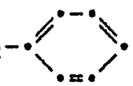 | Smp. 79-80°C |
| 1.200 | Br | H | Cl | H | H | H | $-CH_2-$ | $-COOCH_2-$ 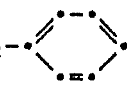 | Smp. 100-102°C |
| 1.201 | Br | H | Br | H | H | H | $-CH_2-$ | $-COOCH_2-$ 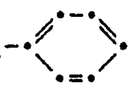 | Smp. 101-104°C |
| 1.202 | Br | H | Cl | H | H | H | $-CH_2-$ | $-COOCH_2CH_2OCH_3$ | Smp. 68-70°C |
| 1.203 | Cl | H | Cl | H | H | H | $-CH_2-$ | $-COOCH_2CH_2OC_2H_5$ | Smp. 81-82°C |
| 1.204 | Br | H | Cl | H | H | H | $-CH_2-$ | $-COOCH_2CH_2OC_2H_5$ | Smp. 71-72°C |
| 1.205 | Br | H | Cl | H | H | H | $-CH_2-$ | $-COOCH_2CH_2OC_3H_7-i$ | $n_D^{25}$ = 1.5763 |
| 1.206 | Br | H | Cl | H | H | H | $-CH_2-$ | $-COOCH_2CH_2O-$ 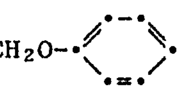 | Smp. 80-82°C |
| 1.207 | Cl | H | Cl | H | H | H | $-CH_2-$ | $-COOCH_2-$ 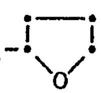 | Smp. 77-78°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.208 | Br | H | Cl | H | H | H | $-CH_2-$ | $-COOCH_2-$ 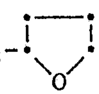 | Smp. 79-80°C |
| 1.209 | Cl | H | Cl | H | H | H | $-CH_2-$ | $-COOCH_2CH=CH_2$ | Smp. 72-73°C |
| 1.210 | Br | H | Cl | H | H | H | $-CH_2-$ | $-COOCH_2CH=CH_2$ | Smp. 66-68,5°C |
| 1.211 | Br | H | Br | H | H | H | $-CH_2-$ | $-COOCH_2CH=CH_2$ | Smp. 78-79°C |
| 1.212 | Br | H | Cl | H | H | H | $-CH_2-$ | $-COOCH_2CH=CH-CH_3$ | Smp. 60-64°C |
| 1.213 | Cl | H | Cl | H | H | H | $-CH_2-$ | $-COOCH_2-\overset{CH_3}{C}=CH_2$ | Smp. 62-65°C |
| 1.214 | Br | H | Cl | H | H | H | $-CH_2-$ | $-COOCH_2-\overset{CH_3}{C}=CH_2$ | Smp. 62-64°C |
| 1.215 | Br | H | Cl | H | H | H | $-CH_2-$ | $-COO-$ 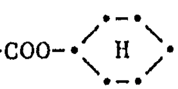 | Smp. 52-54°C |
| 1.216 | H | H | Cl | H | H | H | $-\underset{CH_3}{CH}-$ | $-COOC_3H_7-i$ | $n_D^{24} = 1.5642$ |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.217 | H | H | Cl | H | H | H | $-CH-$<br>$CH_3$ | $-COO(CH_2)_7CH_3$ | $n_D^{23}$ = 1.5356 |
| 1.218 | H | H | Cl | H | H | H | $-CH-$<br>$CH_3$ | $CH_3$<br>$-COOCH(CH_2)_5CH_3$ | $n_D^{25}$ = 1.5370 |
| 1.219 | H | H | Cl | H | H | H | $-CH-$<br>$CH_3$ | $-COO(CH_2)_{11}CH_3$ | Smp. 54-55°C |
| 1.220 | H | H | Cl | H | H | H | $-CH-$<br>$CH_3$ | $-COOCH_2-$ 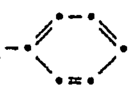 | Smp. 57-59°C |
| 1.221 | H | H | Cl | H | H | H | $-CH-$<br>$CH_3$ | $-COOCH_2CH_2OC_3H_7-i$ | $n_D^{32}$ = 1.5403 |
| 1.222 | H | H | Cl | H | H | H | $-CH-$<br>$CH_3$ | $-COOCH_2CH_2O-$ 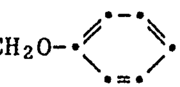 | $n_D^{29}$ = 1.5962 |
| 1.223 | H | H | Cl | H | H | H | $-CH-$<br>$CH_3$ | $-COOCH_2CH{=}CH_2$ | Smp. 40-41°C |
| 1.224 | H | H | Cl | H | H | H | $-CH-$<br>$CH_3$ | $-COOCH_2CH{=}CH-CH_3$ | Smp. 39-40°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.225 | H | H | Cl | H | H | H | $-\underset{CH_3}{CH}-$ | $-COOCH_2-\overset{CH_3}{C}=CH_2$ | Smp. 62-63°C |
| 1.226 | H | H | Cl | H | H | H | $-\underset{CH_3}{CH}-$ | -COO-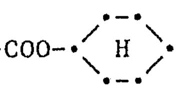 | $n_D^{30} = 1.5677$ |
| 1.227 | Br | H | Cl | H | H | H | $-\underset{CH_3}{CH}-$ | $-COO(CH_2)_7CH_3$ | $n_D^{28} = 1.5439$ |
| 1.228 | Cl | H | Cl | H | H | H | $-\underset{CH_3}{CH}-$ | $-COO\overset{CH_3}{CH}(CH_2)_5CH_3$ | $n_D^{25} = 1.5408$ |
| 1.229 | Br | H | Cl | H | H | H | $-\underset{CH_3}{CH}-$ | $-COO\overset{CH_3}{CH}(CH_2)_5CH_3$ | $n_D^{25} = 1.5527$ |
| 1.230 | Br | H | Cl | H | H | H | $-\underset{CH_3}{CH}-$ | $-COO(CH_2)_{11}CH_3$ | $n_D^{30} = 1.5347$ |
| 1.231 | Br | H | Cl | H | H | H | $-\underset{CH_3}{CH}-$ | $-COOCH_2-$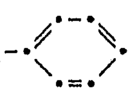 | Smp. 55-56°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.232 | Br | H | Cl | H | H | H | $-CH(CH_3)-$ | $-COOCH_2-$ 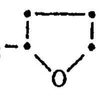 | $n_D^{30} = 1.5886$ |
| 1.233 | Br | H | Cl | H | H | H | $-CH(CH_3)-$ | $-COOCH_2CH_2OC_3H_7-i$ | $n_D^{28} = 1.5642$ |
| 1.234 | Br | H | Cl | H | H | H | $-CH(CH_3)-$ | $-COOCH_2CH_2O-$ 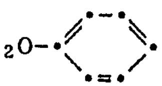 | $n_D^{20} = 1.6031$ |
| 1.235 | Br | H | Cl | H | H | H | $-CH(CH_3)-$ | $-COOCH_2CH=CH_2$ | Smp. 55-56°C |
| 1.236 | Cl | H | Cl | H | H | H | $-CH(CH_3)-$ | $-COOCH_2CH=CH-CH_3$ | Smp. 38-39°C |
| 1.237 | Br | H | Cl | H | H | H | $-CH(CH_3)-$ | $-COOCH_2CH=CH-CH_3$ | Smp. 38-40°C |
| 1.238 | Br | H | Cl | H | H | H | $-CH(CH_3)-$ | $-COOCH_2C(CH_3)=CH_2$ | $n_D^{28} = 1.5824$ |
| 1.239 | H | H | Cl | H | H | H | $-CH_2-$ | $-COO-$ 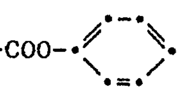 | Smp. 165-170°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.240 | H | H | Cl | H | H | H | $-CH_2-$ | 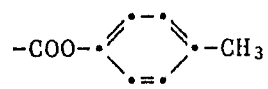 | Smp. 143-145°C |
| 1.241 | H | H | Cl | H | H | H | $-CH_2-$ | 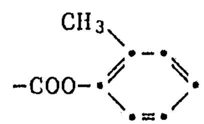 | Smp. 111-116°C |
| 1.242 | H | H | Cl | H | H | H | $-CH_2-$ | 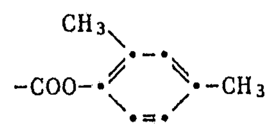 | Smp. 108-119°C |
| 1.243 | H | H | Cl | H | H | H | $-CH(CH_3)-$ | 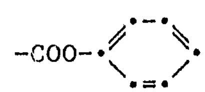 | Smp. 102-105°C |

Tabelle 1 (Fortsetzung)

| Nr. | $R^1$ | $R^2$ | $R^3$ | $R^4$ | $R^5$ | $R^6$ | A + Z | physikal. Konstante |
|---|---|---|---|---|---|---|---|---|
| 1.244 | H | H | Cl | H | H | H | 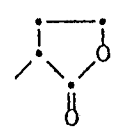 | Smp. 140-141,5°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.245 | H | H | Cl | H | H | H | $-CH_2-$ | $-COOCH(CH_3)CH_2CH_2CH_3$ | Smp. 65-70°C |
| 1.246 | H | H | H | H | H | H | $-CH_2-$ | $-COOCH_2-CH(CH_3)(CH_2)_2CH_3$ | $n_D^{22}$ = 1.5525 |
| 1.247 | H | H | Cl | H | H | H | $-CH_2-$ | -COO- 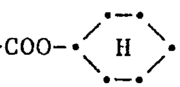 | Smp. 112-113°C |
| 1.248 | H | H | Cl | H | H | H | $-CH_2-$ | $-COOCH_2CH(CH_3)-CH_3$ | Smp. 113-114°C |
| 1.249 | H | H | H | H | H | H | $-CH_2-$ | $-COO(CH_2)_2CH(OCH_3)CH_3$ | $n_D^{22}$ =1.5580 |
| 1.250 | H | H | H | H | H | H | $-CH_2-$ | $-COOCH_2CH_2OCH_2CH_2O(CH_2)_3CH_3$ | $n_D^{22}$ =1.5389 |
| 1.251 | H | H | H | H | H | H | $-CH_2-$ | $-COS(CH_2)_3CH_3$ | $n_D^{23}$ =1.6096 |
| 1.252 | H | H | H | H | H | H | $-CH_2-$ | -COO- 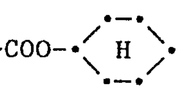 | $n_D^{23}$ =1.5755 |
| 1.253 | H | H | H | H | H | H | $-CH_2-$ | $-COO(CH_2)_4CH_3$ | $n_D^{23}$ =1.5591 |
| 1.254 | H | H | H | H | H | H | $-CH_2-$ | $-COS(CH_2)_7CH_3$ | $n_D^{22}$ =1.5697 |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.255 | H | H | Cl | H | H | H | $-CH_2-$ | $-COOCH_2-\overset{CH_3}{CH}(CH_2)_2CH_3$ | Smp.74-75°C |
| 1.256 | H | H | Cl | H | H | H | $-CH_2-$ | $-COS(CH_2)_3CH_3$ | $n_D^{22}$ =1.6076 |
| 1.257 | H | H | H | H | H | H | $-CH_2-$ | $-COOCH_2CH=CH-CH_3$ | $n_D^{22}$ =1.5833 |
| 1.258 | H | H | H | H | H | H | $-CH_2-$ | $-COOCH_2-\overset{C_2H_5}{CH}-C_2H_5$ | $n_D^{23}$ =1.5530 |
| 1.259 | H | H | Cl | H | H | H | $-CH_2-$ | $-COOCH_2CH_2OCH_2CH_2O(CH_2)_3CH_3$ | Smp. 39-41°C |
| 1.260 | H | H | Cl | H | H | H | $-CH_2-$ | $-COO(CH_2)_2\overset{OCH_3}{CH}CH_3$ | Smp. 72-73°C |
| 1.261 | H | H | Cl | H | H | H | $-CH_2-$ | $-COO(CH_2)_4CH_3$ | Smp. 78-79°C |
| 1.262 | H | H | Cl | H | H | H | $-CH_2-$ | $-COO\overset{C_2H_5}{CH}-(CH_2)_2CH_3$ | Smp. 37-46°C |
| 1.263 | H | H | H | H | H | H | $-CH_2-$ | $-COOCH_2CH_2OC_3H_7-i$ | $n_D^{22}$= 1.5546 |
| 1.264 | H | H | Cl | H | H | H | $-CH_2-$ | $-COO(CH_2)_{13}CH_3$ | Smp.75-76°C |
| 1.265 | H | H | H | H | H | H | $-CH_2-$ | $-COO\overset{C_2H_5}{CH}-C_2H_5$ | Smp. 47-50°C |
| 1.266 | H | H | H | H | H | H | $-CH_2-$ | 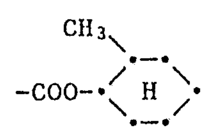 | Smp. 29-31°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.267 | H | H | Cl | H | H | H | $-CH_2-$ | $-COOCH_2-CH(C_2H_5)-C_2H_5$ | Smp. 58-63°C |
| 1.268 | H | H | H | H | H | H | $-CH_2-$ | $-COOCH_2CH_2OCH_2CH_2OC_2H_5$ | $n_D^{22}$ =1.5489 |
| 1.269 | H | H | H | H | H | H | $-CH_2-$ | $-COOCH_2CH_2O-$ 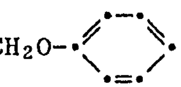 | Smp. 80-81°C |
| 1.270 | H | H | Cl | H | H | H | $-CH_2-$ | $-COOCH(C_2H_5)-C_2H_5$ | Smp. 55-80°C |
| 1.271 | H | H | H | H | H | H | $-CH_2-$ | $-COOCH(CH_3)CH_2CH(CH_3)-CH_3$ | $n_D^{22}$ =1.5463 |
| 1.272 | H | H | H | H | H | H | $-CH_2-$ | $-COO(CH_2)_{13}CH_3$ | Smp. 35-36°C |
| 1.273 | H | H | H | H | H | H | $-CH_2-$ | $-COOCH_2CH_2O(CH_2)_3CH_3$ | $n_D^{22}$=1.5495 |
| 1.274 | H | H | Cl | H | H | H | $-CH_2-$ | $-COOCH_2CH_2OCH_2CH_2OC_2H_5$ | Smp.42-43°C |
| 1.275 | H | H | H | H | H | H | $-CH_2-$ | $-COOCH_2-CH(CH_3)-C_2H_5$ | $n_D^{22}$ =1.5566 |
| 1.276 | H | H | Cl | H | H | H | $-CH_2-$ | $-COOCH(CH_3)CH_2CH(CH_3)-CH_3$ | Smp.63-64°C |
| 1.277 | H | H | H | H | H | H | $-CH_2-$ | $-COSCH(CH_3)-C_2H_5$ | $n_D^{22}$ =1.5973 |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.278 | H | H | Cl | H | H | H | $-CH_2-$ | 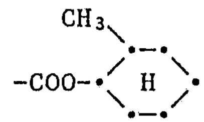 | Smp.98-101°C |
| 1.279 | H | H | H | H | H | H | $-CH_2-$ | 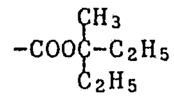 | $n_D^{22}$=1.5551 |
| 1.280 | H | H | H | H | H | H | $-CH_2-$ | 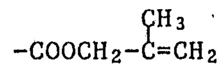 | $n_D^{22}$ =1.5805 |
| 1.281 | H | H | H | H | H | H | $-CH_2-$ | 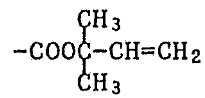 | $n_D^{22}$=1.5793 |
| 1.282 | H | H | H | H | H | H | $-CH_2-$ | $-COOCH_2CH_2OCH_2CH_2OCH_3$ | $n_D^{23}$ =1.5560 |
| 1.283 | H | H | Cl | H | H | H | $-CH_2-$ | 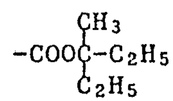 | $n_D^{22}$ =1.5632 |
| 1.284 | H | H | Cl | H | H | H | $-CH_2-$ | $-COO(CH_2)_{10}CH_3$ | Smp.70-71°C |
| 1.285 | H | H | Cl | H | H | H | $-CH_2-$ | 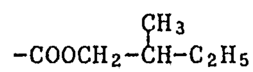 | Smp.78-79°C |
| 1.286 | H | H | H | H | H | H | $-CH_2-$ | 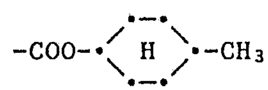 | Smp.40-42°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.287 | H | H | H | H | H | H | $-CH_2-$ | $-COO(CH_2)_6CH_3$ | $n_D^{23}=1.5469$ |
| 1.288 | H | H | H | H | H | H | $-CH_2-$ | $CH_3$<br>$-COOC-C_2H_5$<br>$CH_3$ | $n_D^{22}=1.5581$ |
| 1.289 | H | H | Cl | H | H | H | $-CH_2-$ | $-COOCH_2CH_2O(CH_2)_3CH_3$ | Smp. 69-70°C |
| 1.290 | H | H | Cl | H | H | H | $-CH_2-$ | $CH_3$<br>$-COSCH-C_2H_5$ | Smp. 55-56°C |
| 1.291 | H | H | Cl | H | H | H | $-CH_2-$ | $CH_3$<br>$-COOC-CH=CH_2$<br>$CH_3$ | Smp. 83-87°C |
| 1.292 | H | H | H | H | H | H | $-CH_2-$ | $-COSCH_3$ | Smp. 41-44°C |
| 1.293 | H | H | Cl | H | H | H | $-CH_2-$ | $-COOCH_2CH_2OCH_2CH_2OCH_3$ | $n_D^{23}= 1.5633$ |
| 1.294 | H | H | Cl | H | H | H | $-CH_2-$ | $-COSCH_3$ | Smp. 89-91°C |
| 1.295 | H | H | Cl | H | H | H | $-CH_2-$ | $CH_3$<br>$-COOC-C_2H_5$<br>$CH_3$ | Smp. 53-54°C |
| 1.296 | H | H | H | H | H | H | $-CH_2-$ | $-COO(CH_2)_{10}CH_3$ | $n_D^{23}=1.5310$ |
| 1.297 | H | H | Cl | H | H | H | $-CH_2-$ | $-COO(CH_2)_6CH_3$ | Smp.74-76°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.298 | H | H | H | H | H | H | $-CH_2-$ | $CH_3CH_3$<br>$-COOCH-CH-CH_3$ | $n_D^{23}=1.5554$ |
| 1.299 | H | H | Cl | H | H | H | $-CH_2-$ | -COO- 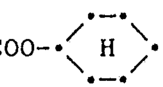 $-CH_3$ | Smp. 103-105°C |
| 1.300 | H | H | H | H | H | H | $-CH_2-$ | $CH_3$<br>$-COSC-CH_3$<br>$CH_3$ | $n_D^{23}=1.5987$ |
| 1.301 | H | H | Cl | H | H | H | $-CH_2-$ | $-COS(CH_2)_{11}CH_3$ | Smp.26-28°C |
| 1.302 | H | H | Cl | H | H | H | $-CH_2-$ | $-COS(CH_2)_9CH_3$ | Smp.29-31°C |
| 1.303 | H | H | Cl | H | H | H | $-CH_2-$ | $-COO(CH_2)_9CH_3$ | Smp.73-74°C |
| 1.304 | H | H | H | H | H | H | $-CH_2-$ | $CH_3$<br>$-COOCH(CH_2)_4CH_3$ | $n_D^{23}=1.5433$ |
| 1.305 | H | H | Cl | H | H | H | $-CH_2-$ | $C_3H_7-n$<br>$-COOCH-C \equiv CH$ | Smp. 81-82°C |
| 1.306 | H | H | H | H | H | H | $-CH_2-$ | $C_5H_{11}-n$<br>$-COOCH-CH-CH_2$ | $n_D^{23}=1.5472$ |
| 1.307 | H | H | Cl | H | H | H | $-CH_2-$ | $CH_3$ $CH_3$<br>$-COOCH-CH-CH_3$ | Smp.70-74°C |
| 1.308 | H | H | Cl | H | H | H | $-CH_2-$ | $CH_3$<br>$-COSC-CH_3$<br>$CH_3$ | $n_D^{22}=1.5996$ |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.309 | H | H | H | H | H | H | $-CH_2-$ | $CH_3$<br>$-COOCH-C\equiv CH$ | $n_D^{23}=1.5837$ |
| 1.310 | H | H | H | H | H | H | $-CH_2-$ | $-COS(CH_2)_{11}CH_3$ | $n_D^{23}=1.5523$ |
| 1.311 | H | H | H | H | H | H | $-CH_2-$ | $CH_3$<br>$-COOCH_2-C-CH_3$<br>$CH_3$ | $n_D^{22}=1.5524$ |
| 1.312 | H | H | H | H | H | H | $-CH_2-$ | $-COSC_2H_5$ | $n_D^{23}=1.6310$ |
| 1.313 | H | H | Cl | H | H | H | $-CH_2-$ | $CH_3$<br>$-COOCH_2-C-CH_3$<br>$CH_3$ | Smp.76-81°C |
| 1.314 | H | H | Cl | H | H | H | $-CH_2-$ | $-COSC_3H_7$-n | $n_D^{22}=1.6136$ |
| 1.315 | H | H | H | H | H | H | $-CH_2-$ | $-COO(CH_2)_9CH_3$ | $n_D^{22}=1.5308$ |
| 1.316 | H | H | Cl | H | H | H | $-CH_2-$ | $CH_3$<br>$-COOCH(CH_2)_4CH_3$ | Smp.65-67°C |
| 1.317 | H | H | H | H | H | H | $-CH_2-$ | $CH_3$<br>$-COO(CH_2)_2CH-CH_3$ | $n_D^{23}=1.5568$ |
| 1.318 | H | H | H | H | H | H | $-CH_2-$ | $C_2H_5$<br>$-COOCH(CH_2)_3CH_3$ | $n_D^{22}=1.5454$ |
| 1.319 | H | H | Cl | H | H | H | $-CH_2-$ | $-COO(CH_2)_8CH_3$ | Smp.78-79°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.320 | H | H | H | H | H | H | $-CH_2-$ | $-COSCH_2CH(CH_3)CH_3$ | $n_D^{23}=1.6049$ |
| 1.321 | H | H | Cl | H | H | H | $-CH_2-$ | $-COSC_2H_5$ | Smp. 55-57°C |
| 1.322 | H | H | H | H | H | H | $-CH_2-$ | $-COO(CH_2)_8CH_3$ | $n_D^{24}=1.5436$ |
| 1.323 | H | H | Cl | H | H | H | $-CH_2-$ | $-COOCH_2-CH(C_2H_5)(CH_2)_3CH_3$ | Smp. 45-47°C |
| 1.324 | H | H | Cl | H | H | H | $-CH_2-$ | $-COSCH_2CH(CH_3)-CH_3$ | $n_D^{23}=1.6045$ |
| 1.325 | H | H | H | H | H | H | $-CH_2-$ | $-COS(CH_2)_9CH_3$ | $n_D^{23}=1.5630$ |
| 1.326 | H | H | Cl | H | H | H | $-CH_2-$ | $-COO(CH_2)_2CH(CH_3)-CH_3$ | Smp. 72-74°C |
| 1.327 | H | H | Cl | H | H | H | $-CH_2-$ | $-COOCH(C_2H_5)(CH_2)_3CH_3$ | $n_D^{22}=1.5542$ |
| 1.328 | H | H | H | H | H | H | $-CH_2-$ | $-COO(CH_2)_5CH_3$ | $n_D^{22}=1.5512$ |
| 1.329 | H | H | H | H | H | H | $-CH_2-$ | $-COOCH(C_3H_7-n)(CH_2)_2CH_3$ | Smp. 48-50°C |
| 1.330 | H | H | H | H | H | H | $-CH_2-$ | $-COS(CH_2)_4CH_3$ | $n_D^{22}=1.5937$ |
| 1.331 | H | H | H | H | H | H | $-CH_2-$ | $-COSC_3H_7-iso$ | $n_D^{23}=1.5821$ |

<u>Tabelle 1</u> (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.332 | H | H | H | H | H | H | $-CH_2-$ | $C_2H_5$<br>$-COOCH_2CH-(CH_2)_3CH_3$ | $n_D^{22}=1.5395$ |
| 1.333 | H | H | Cl | H | H | H | $-CH_2-$ | $C_3H_7-n$<br>$-COOCH(CH_2)_2CH_3$ | Smp.55-57°C |
| 1.334 | H | H | Cl | H | H | H | $-CH_2-$ | $COS(CH_2)_5CH_3$ | $n_D^{22}=1.5882$ |
| 1.335 | H | H | Cl | H | H | H | $-CH_2-$ | $-COS(CH_2)_4CH_3$ | $n_D^{23}=1.5990$ |
| 1.336 | H | H | Cl | H | H | H | $-CH_2-$ | $-COO(CH_2)_5CH_3$ | Smp.71-72°C |
| 1.337 | H | H | Cl | H | H | H | $-CH_2-$ | $-COSC_3H_7-iso$ | Smp.62-64°C |
| 1.338 | H | H | Cl | H | H | H | $-CH_2-$ | $C_2H_5$ $CH_3$<br>$-COOCH-CH_2CHC_2H_5$ | Smp.25-29°C |
| 1.339 | H | H | H | H | H | H | $-CH_2-$ | $C_3H_7-i$<br>$-COOCH-C_3H_7-i$ | $n_D^{22}=1.5468$ |
| 1.340 | H | H | H | H | H | H | $-CH_2-$ | $CH_3$<br>$-COOCH-(CH_2)_3CH_3$ | $n_D^{23}=1.5531$ |
| 1.341 | H | H | Cl | H | H | H | $-CH_2-$ | $C_5H_{11}-n$<br>$-COOCH-CH=CH_2$ | $n_D^{23}=1.5579$ |
| 1.342 | H | H | H | H | H | H | $-CH_2-$ | $C_2H_5$<br>$-COOCH-(CH_2)_2CH_3$ | Smp.42-44°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.343 | H | H | H | H | H | H | $-CH_2-$ | $-COSC_3H_7-n$ | $n_D^{22}$ =1.6108 |
| 1.344 | H | H | Cl | H | H | H | $-CH_2-$ | $-COO\overset{CH_3}{C}H-(CH_2)_3CH_3$ | Smp.68-71°C |
| 1.345 | H | H | H | H | H | H | $-CH_2-$ | $-COO\overset{C_2H_5}{C}HCH_2\overset{CH_3}{C}HC_2H_5$ | $n_D^{23}$=1.5472 |
| 1.346 | H | H | Cl | H | H | H | $-CH_2-$ | $-COO\overset{C_3H_7-i}{C}H-C_3H_7-i$ | Smp.88-89°C |
| 1.347 | H | H | H | H | H | H | $-CH_2-$ | $-COS(CH_2)_5CH_3$ | $n_D^{22}$=1.5804 |
| 1.348 | H | H | H | H | H | H | $-CH_2-$ | $-COO(CH_2)_9-CH=CH_2$ | $n_D^{22}$=1.5386 |
| 1.349 | H | H | H | H | H | H | $-CH_2-$ | $-COO\overset{C_3H_7-n}{C}H-C\equiv CH$ | $n_D^{22}$=1.5659 |
| 1.350 | H | H | Cl | H | H | H | $-CH_2-$ | $-COO\overset{CH_3}{C}H-C\equiv CH$ | Smp.97-100°C |
| 1.351 | H | H | H | H | H | H | $-CH_2-$ | $-COO\overset{CH_3}{\underset{C_2H_5}{C}}-C\equiv CH$ | $n_D^{22}$=1.5688 |
| 1.352 | H | H | Cl | H | H | H | $-CH_2-$ | $-COO(CH_2)_9-CH=CH_2$ | Smp.66-67°C |
| 1.353 | H | H | Cl | H | H | H | $-CH_2-$ | $-COO-\overset{CH_3}{\underset{CH_3}{C}}-C\equiv CH$ | Smp.76-81°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.354 | H | H | H | H | H | H | $-CH_2$ | $CH_3$<br>$-COO-C-C\equiv CH$<br>$CH_3$ | $n_D^{23}=1.5740$ |
| 1.355 | H | H | Cl | H | H | H | $-CH_2-$ | $CH_3$<br>$-COO-C-C\equiv CH$<br>$C_2H_5$ | Smp.78-79°C |
| 1.356 | H | H | Cl | H | H | H | $-CH_2-$ | $CH_3\,CH_3$<br>$-COO-CH-CH-C_2H_5$ | Smp.71-73°C |
| 1.357 | Br | H | Cl | H | H | H | $-CH_2-$ | $-COOCH_3$ | Smp.126-128°C |
| 1.358 | Br | H | Cl | H | H | H | $-CH-$<br>$CH_3$ | $-COOC_3H_7-i$ | Smp.66-68°C |
| 1.359 | Cl | H | Cl | H | H | H | $-CH_2-$ | $-COOCH_2CH_2OCH_3$ | Smp.68-70°C |
| 1.360 | Cl | H | Cl | H | H | H | $-CH_2-$ | $-COOC_3H_7-i$ | Smp.60-63°C |
| 1.361 | H | H | Cl | H | H | H | $-CH-$<br>$CH_3$ | $-COOCH_2-$ 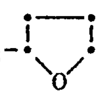 | $n_D^{30}=1.5734$ |
| 1.362 | H | H | Cl | H | H | H | $-CH_2-$ | $-COOCH_2COOC_4H_9-n$ | Smp.52-54°C |
| 1.363 | H | H | Cl | H | H | H | $-CH_2-$ | $-COOCH-COOC_4H_9-n$<br>$CH_3$ | $n_D^{22}=1.5508$ |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.364 | H | H | Cl | H | H | H | $-CH_2-$ | 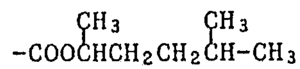 | Smp. 55-59°C |
| 1.365 | H | H | Cl | H | H | H | $-CH_2-$ | 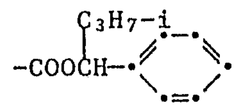 | Smp. 43-47°C |
| 1.366 | H | H | Cl | H | H | H | $-CH_2-$ | 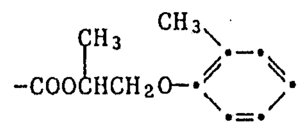 | Smp. 75-78°C |
| 1.367 | H | H | Cl | H | H | H | $-CH_2-$ | 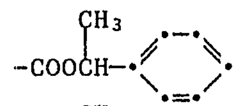 | Smp. 117-122°C |
| 1.368 | H | H | Cl | H | H | H | $-CH_2-$ | 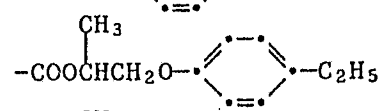 | Smp. 63-68°C |
| 1.369 | H | H | Cl | H | H | H | $-CH_2-$ | 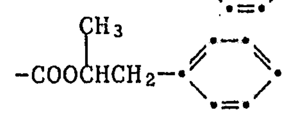 | Smp. 116-118°C |
| 1.370 | H | H | Cl | H | H | H | $-CH_2-$ | 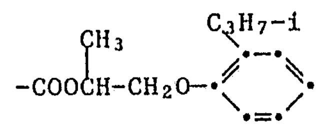 | Smp. 41.43°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.371 | H | H | Cl | H | H | H | $-CH_2-$ | 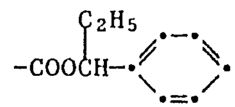 | Smp. 74-76°C |
| 1.372 | H | H | Cl | H | H | H | $-CH_2-$ | 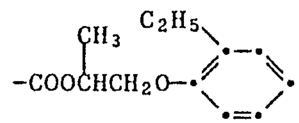 | Smp. 96-98°C |
| 1.373 | H | H | Cl | H | H | H | $-CH_2-$ | 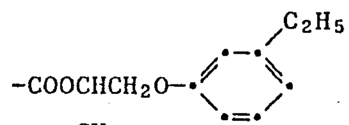 | Smp. 82-85°C |
| 1.374 | H | H | Cl | H | H | H | $-CH_2-$ | 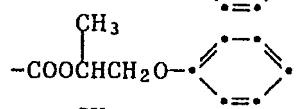 | Smp. 42-44°C |
| 1.375 | H | H | Cl | H | H | H | $-CH_2-$ | 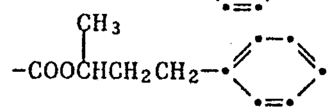 | Smp. 78-79°C |
| 1.376 | H | H | Cl | H | H | H | $-CH_2-$ | 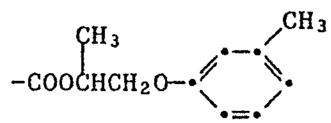 | Smp. 58-61°C |
| 1.377 | H | H | Cl | H | H | H | $-CH_2-$ | 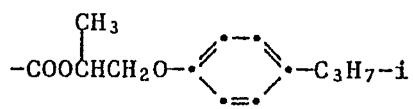 | Smp. 35-38°C |
| 1.378 | H | H | Cl | H | H | H | $-CH_2-$ | 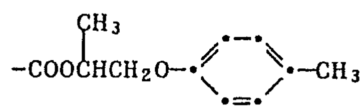 | Smp. 82-84°C |

Die Verbindungen der Formel I lassen sich nach bekannten Methoden herstellen, wie sie in den europäischen Patentpublikationen EP-A-86 750 und EP-A-94 349 beschrieben sind.

Die Chinolderivate der Formel I besitzen in hervorrangendem Massen die Eigenschaften, Kulturpflanzen gegen schädigende Wirkungen von herbizid wirksamen Silizium- und Phosphor-haltigen 2-[4-(5-Chlor-3-fluor-pyridin-2-yloxy)-phenoxy]-propionsäure-Derivaten zu schützen. Die vorgenannten herbiziden Wirkstoffe sind aus der Europäischen Patentanmeldung EP-A-200 677 bekannt und können nach den dort angegebenen Methoden hergestellt werden. Besonders wirkungsvolle und gemäss der erfindungsgemässen Lehre einsetzbare Silizium-und Phosphor-haltige 2-[4-(5-Chlor-3-fluorpyridin-2-yloxy)-phenoxy]-propionsäure-Derivate entsprechen der Formel II

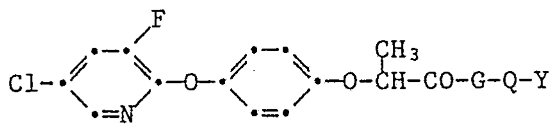

(II)

worin

G Sauerstoff oder Schwefel,

Q eine unsubstituierte oder einfach oder zweifach durch Methyl oder Phenyl substituierte $C_1$-$C_3$-Alkylenkette, und

Y eine Gruppe $-Si(OR^{17})_2R^{18}$, $-Si(R^{18})_3$, $-PO(OR^{19})-OR^{20}$, $-PO(OR^{19})-R^{20}$ oder $-O-PO(OR^{19})_2$ bedeuten, wobei

$R^{17}$ und $R^{18}$ unabhängig voneinander für $C_1$-$C_6$-Alkyl und

$R^{19}$ und $R^{20}$ unabhängig voneinander für Wasserstoff, $C_1$-$C_4$-Alkyl, $C_3$-$C_4$-Alkenyl, $C_3$-$C_4$-Alkinyl, $C_2$-$C_4$-Halogenalkyl, $C_1$-$C_4$-Alkoxy-($C_2$-$C_4$)-alkyl, $C_1$-$C_4$-Cyanoalkyl oder einen substituierten oder durch Halogen, $C_1$-$C_4$-Alkyl, $C_1$-$C_4$-Alkoxy, $C_1$-$C_4$-Halogenalkyl, $C_1$-$C_4$-Halogenalkoxy, Nitro oder Cyano ein- bis zweifach substituierten Phenyl-, Phenyl-($C_1$-$C_4$)-alkyl oder Naphthylrest stehen.

In den Verbindungen der Formel II bedeutet Halogen als selbstständiger Substituent oder Teil eines anderen Substituenten, wie Halogenalkyl oder Halogenalkoxy, Fluor, Chlor, Brom oder Jod, worunter Fluor oder Chlor bevorzugt sind.

Alkyl steht je nach der Anzahl der vorhandenen Kohlenstoffatome für Methyl, Aethyl, n-Propyl, i-Propyl sowie die isomere Butyl, Pentyl oder Hexyl. Die in den Resten Alkoxy, Alkoxyalkyl, Halogenalkyl oder Halogenalkoxy enthaltenen Alkylgruppen haben die gleiche Bedeutung. Bevorzugt sind jeweils Alkylgruppe mit niedriger Anzahl von Kohlenstoffatomen.

Bevorzugte Halogenalkylreste, bzw. Halogenalkylteile in Halogenalkoxyresten sind: Fluormethyl, Difluormethyl, Trifluormethyl, Chlormethyl, Trichlormethyl, 2-Fluoräthyl, 2,2,2-Trifluoräthyl, 1,1,2,2-Tetrafluoräthyl, Perfluoräthyl, 2-Chloräthyl, 2,2,2-Trichloräthyl, 2-Bromäthyl und 1,1,2,3,3,3,-Hexafluorpropyl.

Die obige Definition von Q umfasst Methylen, 1,2-Aethylen und 1,3-Propylen und die durch Substitution von 1 bis 2 Wasserstoffatomen durch Methyl oder Phenyl davon abgeleiteten Gruppen, wie z.B. Aethyliden, Isopropyliden (2,2-Propyliden), Benzyliden, 1-Phenyläthyliden, Diphenylmethylen, 1,2-Propylen, 2,3-Butylen, 1,1-Dimethyl-1,2-äthylen, 1,1-Diphenyl-1,2-äthylen, 1,2-Diphenyl-1,2-äthylen, 1-Methyl-1-phenyl-1,2-äthylen, 1,2-Butylen, 1,3-Butylen, 2,2-Dimethyl-1,3-propylen, 1-Methyl-1-phenyl-1,3-propylen, 1,2-Diphenyl-1,3-propylen und 1,3-Diphenyl-1,3-propylen.

Besonders bemerkenswert ist die Schutzwirkung von Chinolin-Derivaten der Formel I gegenüber solchen Herbiziden der Formel II, in denen die Gruppe -G-Q-Y für einen Silizium-haltigen Rest steht. Vorzugsweise wird -G-Q-Y durch einen Trialkylsilylalkoxyrest verkörpert.

Besonders hervorzuhebende Einzelbedeutungen für -G-Q-Y sind Trimethylsilylmethoxy und Trimethylsilyläthoxy.

Das optisch aktive Kohlenstoffatomen der Propionsäuregruppe hat üblicherweise sowohl R- als auch S-Konfiguration. Ohne besondere Angabe sind hierin die racemischen Gemische gemeint. Bevorzugte Herbizide der Formel II sind 2R-konfiguriert.

Beispiele für herbizid wirksame Silizium- und Phosphor-haltige 2-[4-(5-Chlor-3-fluorpyridin-2-yloxy)-phenoxy]-propionsäure-Derivate, gegen deren Wirkung Kulturpflanzen erfindungsgemäss geschützt werden können, sind in der nachfolgenden Tabelle 2 aufgeführt.

Tabelle 2:

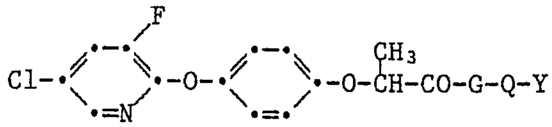

(II)

| Nr. | -G-Q-Y | physikalische Konstante |
|---|---|---|
| 2.1 | $-OCH_2P(O)(OC_2H_5)_2$ | |
| 2.2 | $-OCH_2P(O)(OCH_3)_2$ | $n_D^{30}$ = 1,5317 |
| 2.3 | $-OCH_2CH_2P(O)(OC_2H_5)_2$ | |
| 2.4 | $-OCH_2CH_2P(O)(OCH_3)_2$ | |
| 2.5 | $-OCH_2CH_2CH_2P(O)(OC_2H_5)_2$ | |
| 2.6 | $-OCH_2-CH(CH_3)P(O)(OC_5H_2)_2$ | |
| 2.7 | $-OCH_2P(O)[OCH(CH_3)_2]_2$ | |
| 2.8 | $-OCH_2P(O)(OCH_3)_2$ | |
| 2.9 | $-OCH(CH_3)P(O)(OCH_3)_2$ | |
| 2.10 | $-OC(CH_3)_2P(O)(OCH_3)_2$ | |
| 2.11 | $-OCH_2P(O)(OCH_3)CH_3$ | |
| 2.12 | $-OCH_2P(O)(OC_2H_5)CH_3$ | |
| 2.13 | $-OCH(CH_3)P(O)(OCH_3)CH_3$ | |
| 2.14 | $-OCH(CH_3)P(O)(OC_2H_5)_2$ | $n_D^{30}$ = 1,5140 |
| 2.15 | $-OCH_2P(O)(OCH_3)CH_2CH_3$ | |
| 2.16 | $-OCH_2CH_2P(O)(OCH_3)CH_2CH_3$ | |
| 2.17 | $-OCH_2-Si(CH_3)_3$ | $n_D^{35}$ = 1,5155 |
| 2.18 | $-OCH_2-Si(CH_3)_3$ (2R)-Enantiomeres | $[\alpha]_D^{20}$+41,3°(Aceton) |
| 2.19 | $-OCH_2CH_2-Si(CH_3)_3$ | $n_D^{35}$ = 1,5201 |
| 2.20 | $-OCH_2CH_2-Si(CH_3)_3$ (2R)-Enantiomeres | $[\alpha]_D^{20}$+37,0°(Aceton) |
| 2.21 | $-OCH_2CH_2CH_2-Si(CH_3)_3$ | |
| 2.22 | $-OCH(CH_3)-Si(CH_3)_3$ | |
| 2.23 | $-OCH_2-Si(CH_2CH_3)_3$ | |
| 2.24 | $-OCH_2CH_2-Si(CH_2CH_3)_3$ | |
| 2.25 | $-OCH_2CH_2-Si(CH_2CH_2CH_3)_3$ | |

| Nr. | -G-Q-Y | physikalische Konstante |
|---|---|---|
| 2.26 | $-S-CH_2-P(O)(OC_2H_5)_2$ | |
| 2.27 | $-S-CH_2CH_2-P(O)(OCH_3)_2$ | |
| 2.28 | $-O-CH-CH_2-P(O)(OC_2H_5)_2$<br>$C_6H_5$ | |
| 2.29 | $-O-CH-P(O)(OCH_3)_2$<br>$C_6H_5$ | |
| 2.30 | $-S-CH_2-CH_2-Si(CH_3)_3$ | |
| 2.31 | $-S-CH_2-Si(C_2H_5)_3$ | |
| 2.32 | $-O-CH-Si(CH_3)_3$<br>$CH_3$ | |
| 2.33 | $-O-C-Si(CH_3)_3$<br>$CH_3$ $CH_3$ | |
| 2.34 | $-O-CH_2-CH_2-CH_2-Si(CH_3)_3$ | |
| 2.35 | $-O-(CH_2)_2-Si(C_2H_5)_2-CH_3$ | |
| 2.36 | $-O-(CH_2)_2-Si(OC_2H_5)-C_2H_5$ | |

Als Kulturpflanzen, welche durch Chinolinderivate der Formel I gegen schädigende Wirkungen von Herbiziden der Formel II geschützt werden können, kommen insbesondere diejenigen in Betracht, die auf dem Nahrungs- oder Textilsektor von Bedeutung sind, beispielsweise Zuckerrohr und insbesondere Kulturhirse, Mais, Reis und andere Getreidearten (Weizen, Roggen, Gerste, Hafer). Ganz besonders ist an dieser Stelle die Verwendung in Weizen, Roggen, Gerste und Reis herauszustellen.

Eine bevorzugte Ausführungsform des erfindungsgemässen Verfahrens besteht in der Verwendung von
2-Chinolin-8-yloxy-essigsäureisopropylester,
2-(5-Chlorchinolin-8-yloxy)-essigsäure-n-dodecylester,
2-(5-Chlorchinolin-8-yloxy)-essigsäure-n-butylester,
2-(5-Chlorchinolin-8-yloxy)-essigsäure-n-oktylester,
2-Chinolin-8-yloxy-essigsäure-s-butylester,
2-(5-Chlorchinolin-8-yloxy)-thioessigsäure-n-oktylester,
2-(5-Chlorchinolin-8-yloxy)-essigsäure-(2-butenyl)-ester,
2-(5-Chlorchinolin-8-yloxy)-essigsäuremethallylester,
2-(5-Chlorchinolin-8-yloxy)-essigsäure-(2-isopropyloxyäthyl)-ester,
2-(5-Chlorchinolin-8-yloxy)-essigsäure-(2-phenoxyäthyl)-ester,
2-(5-Chlorchinolin-8-yloxy)-essigsäure-(1-methylbutyl)-ester,
2-(5-Chlorchinolin-8-yloxy)-essigsäurecyclohexylester,
2-(5-Chlorchinolin-8-yloxy)-essigsäure-s-butylester,
2-(5-Chlorchinolin-8-yloxy)-essigsäure-(2-methylpentyl)-ester,
2-(5-Chlorchinolin-8-yloxy)-thioessigsäure-n-butylester,
2-(5-Chlorchinolin-8-yloxy)-essigsäure-(3,6-dioxadecyl)-ester,
2-(5-Chlorchinolin-8-yloxy)-essigsäure-(3-methoxybutyl)-ester,
2-(5-Chlorchinolin-8-yloxy)-essigsäure-(1-äthylbutyl)-ester,
2-(5-Chlorchinolin-8-yloxy)-essigsäure-(2-äthylbutyl)-ester,
2-(5-Chlorchinolin-8-yloxy)-essigsäure-(1-methylsiopentyl)-ester,
2-(5-Chlorchinolin-8-yloxy)-essigsäure-n-undecylester,
2-(5-Chlorchinolin-8-yloxy)-essigsäure-(2-methylbutyl)-ester,
2-(5-Chlorchinolin-8-yloxy)-thioessigsäure-s-butylester,
2-(5-Chlorchinolin-8-yloxy)-essigsäure-(3,6-dioxaheptyl)-ester,
2-(5-Chlorchinolin-8-yloxy)-essigsäure-n-heptylester,
2-(5-Chlorchinolin-8-yloxy)-thioessigsäure-n-dodecylester,
2-(5-Chlorchinolin-8-yloxy)-thioessigsäure-n-decylester,
2-(5-Chlorchinolin-8-yloxy)-essigsäure-(1-propylpropargyl)-ester,
2-(5-Chlorchinolin-8-yloxy)-essigsäure-(1-methylisobutyl)-ester,

2-(5-Chlorchinolin-8-yloxy)-thioessigsäure-tert.-butylester,
2-(5-Chlorchinolin-8-yloxy)-essigsäure-neopentylester,
2-(5-Chlorchinolin-8-yloxy)-thioessigsäure-n-propylester,
2-(5-Chlorchinolin-8-yloxy)-essigsäure-(1-methylhexyl)-ester,
2-(5-Chlorchinolin-8-yloxy)-thioessigsäureäthylester,
2-(5-Chlorchinolin-8-yloxy)-essigsäure-(2-äthylhexyl)-ester,
2-(5-Chlorchinolin-8-yloxy)-thioessigsäure-i-butylester,
2-Chinolin-8-yloxy-thioessigsäure-n-decylester,
2-(5-Chlorchinolin-8-yloxy)-essigsäure-i-pentylester,
2-(5-Chlorchinolin-8-yloxy)-essigsäure-(1-äthylpentyl)-ester,
2-(5-Chlorchinolin-8-yloxy)-essigsäure-(1-propylbutyl)-ester,
2-(5-Chlorchinolin-8-yloxy)-thioessigsäure-n-hexylester,
2-(5-Chlorchinolin-8-yloxy)-essigsäure-n-hexylester,
2-(5-Chlorchinolin-8-yloxy)-thioessigsäure-i-propylester,
2-(5-Chlorchinolin-8-yloxy)-essigsäure-(1-pentylallyl)-ester,
2-(5-Chlorchinolin-8-yloxy)-essigsäure-(1-methylpentyl)-ester,
2-(5-Chlorchinolin-8-yloxy)-essigsäure-(1,1-dimethylpropargyl)-ester,
2-(5-Chlorchinolin-8-yloxy)-essigsäure-(1-äthyl-1-methylpropargyl)-ester,
2-(5-Chlorchinolin-8-yloxy)-essigsäure-n-butyloxycarbonylmethylester,
2-(5-Chlorchinolin-8-yloxy)-essigsäure-(1-n-butyloxycarbonyläthyl)-ester,
2-(5-Chlorchinolin-8-yloxy)-essigsäure-(1-methylisohexyl)-ester,
2-(5-Chlorchinolin-8-yloxy)-essigsäure-(1-phenylisobutyl)-ester,
2-(5-Chlorchinolin-8-yloxy)-essigsäure-[1-methyl-2-(2-methylphenoxy)äthyl]-ester,
2-(5-Chlorchinolin-8-yloxy)-essigsäure-(1-phenyläthyl)-ester,
2-(5-Chlorchinolin-8-yloxy)-essigsäure-[1-methyl-2-(4-äthylphenoxy)-äthyl]-ester,
2-(5-Chlorchinolin-8-yloxy)-essigsäure-(1-methyl-2-phenyläthyl)-ester,
2-(5-Chlorchinolin-8-yloxy)-essigsäure-[1-methyl-2-(2-isopropanolphenoxy)-äthyl]-ester,
2-(5-Chlorchinolin-8-yloxy)-essigsäure-(1-phenylpropyl)-ester,
2-(5-Chlorchinolin-8-yloxy)-essigsäure-[1-methyl-2-(2-äthylphenoxy)-äthyl-ester,
2-(5-Chlorchinolin-8-yloxy)-essigsäure-[1-methyl-2-(3-äthylphenoxy)-äthyl]-ester,
2-(5-Chlorchinolin-8-yloxy)-essigsäure-(1-methyl-2-phenoxyäthyl)-ester,
2-(5-Chlorchinolin-8-yloxy)-essigsäure-(1-methyl-3-phenylpropyl)-ester,
2-(5-Chlorchinolin-8-yloxy)-essigsäure-[1-methyl-2-(3-methylphenoxy) -äthyl]-ester,
2-(5-Chlorchinolin-8-yloxy)-essigsäure-[1-methyl-2-(4-isopropanolphenoxy)-äthyl]-ester oder
2-(5-Chlorchinolin-8-yloxy)-essigsäure-[1-methyl-2-(4-methylphenoxy)-äthyl]-ester
zum Schützen von Kulturpflanzen, insbesondere Getreide, gegen die schädigende Wirkung von 2-[4-(5-Chlor-3-fluorpyridin-2-yloxy)-phenoxy]-propionsäure-trimethylsilylmethylester, 2R-2-[4-(5-Chlor-3-fluorpyridin-2yloxy)-phenoxy]-propionsäure-trimethylsilylmethylester, 2-[4-(5-Chlor-3-fluorpyridin-2-yloxy)-phenoxy]-propionsäure-trimethylsilyläthylester oder 2R-2-[4-(5-Chlor-3-fluorpyridin-2-yloxy)-phenoxy]-propionsäure-trimethylsilyläthylester.

Wegen des hervorragenden erzielbaren Ergebnisses wird der Anwender vorzugsweise die Verbindungen
2-(5-Chlorchinolin-8-yloxy)-essigsäuremethallylester,
2-(5-Chlorchinolin-8-yloxy)-essigsäure-(2-phenoxyäthyl)-ester,
2-(5-Chlorchinolin-8-yloxy)-essigsäure-(1-methylbutyl)-ester,
2-(5-Chlorchinolin-8-yloxy)-essigsäure-(1-äthylbutyl)-ester,
2-(5-Chlorchinolin-8-yloxy)-essigsäure-(1-methylisopentyl)-ester,
2-(5-Chlorchinolin-8-yloxy)-essigsäure-(1-methylhexyl)-ester,
2-(5-Chlorchinolin-8-yloxy)-essigsäure-(1-äthylpentyl)-ester,
2-(5-Chlorchinolin-8-yloxy)-essigsäure-(1-propylbutyl)-ester,
2-(5-Chlorchinolin-8-yloxy)-essigsäure-(1-pentylallyl)-ester,
2-(5-Chlorchinolin-8-yloxy)-essigsäure-(1-methylpentyl)-ester,
2-(5-Chlorchinolin-8-yloxy)-essigsäure-(1-methylisohexyl)-ester,
2-(5-Chlorchinolin-8-yloxy)-essigsäure-(1-phenylisobutyl)-ester,
2-(5-Chlorchinolin-8-yloxy)-essigsäure-(1-phenyläthyl)-ester,
2-(5-Chlorchinolin-8-yloxy)-essigsäure-[1-methyl-2-(4-äthylphenoxy)-äthyl]-ester,
2-(5-Chlorchinolin-8-yloxy)-essigsäure-(1-methyl-2-phenyläthyl)-ester,
2-(5-Chlorchinolin-8-yloxy)-essigsäure-(1-phenylpropyl)-ester,
2-(5-Chlorchinolin-8-yloxy)-essigsäure-(1-methyl-2-phenoxyäthyl)-ester,
2-(5-Chlorchinolin-8-yloxy)-essigsäure-(1-methyl-3-phenylpropyl)-ester oder
2-(5-Chlorchinolin-8-yloxy)-essigsäure-[1-methyl-2-(4-methylphenoxy)-äthyl]-ester,
zum Schützen von Kulturpflanzen, insbesondere Getreide, gegen die schädigende Wirkung von 2-[4-(5-Chlor-3-fluorpyridin-2-yloxy)-phenoxy]-propionsäure-trimethylsilylmethylester, 2R-2-[4-(5-Chlor-3-fluorpyridin-2yloxy)-phenoxy]-propionsäure-trimethylsilylmethylester, 2-[4-(5-Chlor-3-fluorpyridin-2-yloxy)-phenoxy]-propionsäure-trimethylsilyläthylester oder 2R-2-[4-(5-Chlor-3-fluorpyridin-2-yloxy)-phenoxy]-propionsäure-trimethylsilyäthylester einsetzen.

Ein geeignetes Verfahren zum Schützen von Kulturpflanzen unter Verwendung von Verbindungen der Formel I besteht darin, dass man Kulturpflanzen, Teile dieser Pflanzen oder für den Anbau der Kulturpflanzen bestimmte Böden vor oder nach dem Einbringen des pflanzlichen Materials in den Boden mit einer Verbindung der Formel I oder einem Mittel, welches eine solche Verbindung enthält, behandelt. Die Behandlung kann vor, gleichzeitig mit oder nach dem Einsatz des Herbizids der Formel II erfolgen. Als Pflanzenteile kommen insbesondere diejenigen in Betracht, die zur Neubildung einer Pflanze befähigt sind, wie beispielsweise Samen, Früchte, Stengelteile und Zweige (Stecklinge) sowie auch Wurzeln, Knollen und Rhizome.

Die Erfindung betrifft auch ein Verfahren zur selektiven Bekämpfung von Unkräutern in Kulturpflanzenbeständen, wobei die Kulturpflanzenbestände, Teile der Kulturpflanzen oder Anbauflächen für Kulturpflanzen mit einem Herbizid der Formel II und einer Verbindung der Formel I oder einem Mittel, welches eine Kombination aus einem solchen Herbizid und einer Verbindung der Formel I enthält, behandelt.

Gegenstand der vorliegenden Erfindung sind ebenfalls herbizide Mittel, welche eine Kombination der antagonistischen Komponente I und der herbiziden Komponente II enthalten.

Vorzugsweise enthalten solche Mittel als antagonistische Komponente eine Verbindung aus der Reihe
2-Chinolin-8-yloxy-essigsäureisopropylester,
2-(5-Chlorchinolin-8-yloxy)-essigsäure-n-dodecylester,
2-(5-Chlorchinolin-8-yloxy)-essigsäure-n-butylester,
2-(5-Chlorchinolin-8-yloxy)-essigsäure-n-oktylester,
2-Chinolin-8-yloxy-essigsäure-s-butylester,
2-(5-Chlorchinolin-8-yloxy)-thioessigsäure-n-oktylester,
2-(5-Chlorchinolin-8-yloxy)-essigsäure-(2-butenyl)-ester,
2-(5-Chlorchinolin-8-yloxy)-essigsäuremethallylester,
2-(5-Chlorchinolin-8-yloxy)-essigsäure-(2-isopropyloxyäthyl)-ester,
2-(5-Chlorchinolin-8-yloxy)-essigsäure-(2-phenoxyäthyl)-ester,
2-(5-Chlorchinolin-8-yloxy)-essigsäure-(1-methylbutyl)-ester,
2-(5-Chlorchinolin-8-yloxy)-essigsäurecyclohexylester,
2-(5-Chlorchinolin-8-yloxy)-essigsäure-s-butylester,
2-(5-Chlorchinolin-8-yloxy)-essigsäure-(2-methylpentyl)-ester,
2-(5-Chlorchinolin-8-yloxy)-thioessigsäure-n-butylester,
2-(5-Chlorchinolin-8-yloxy)-essigsäure-(3,6-dioxadecyl)-ester,
2-(5-Chlorchinolin-8-yloxy)-essigsäure-(3-methoxybutyl)-ester,
2-(5-Chlorchinolin-8-yloxy)-essigsäure-(1-äthylbutyl)-ester,
2-(5-Chlorchinolin-8-yloxy)-essigsäure-(2-äthylbutyl)-ester,
2-(5-Chlorchinolin-8-yloxy)-essigsäure-(1-methylisopentyl)-ester,
2-(5-Chlorchinolin-8-yloxy)-essigsäure-n-undecylester,
2-(5-Chlorchinolin-8-yloxy)-essigsäure-(2-methylbutyl)-ester,
2-(5-Chlorchinolin-8-yloxy)-thioessigsäure-s-butylester,
2-(5-Chlorchinolin-8-yloxy)-essigsäure-(3,6-dioxaheptyl)-ester,
2-(5-Chlorchinolin-8-yloxy)-essigsäure-n-heptylester,
2-(5-Chlorchinolin-8-yloxy)-thioessigsäure-n-dodecylester,
2-(5-Chlorchinolin-8-yloxy)-thioessigsäure-n-decylester,
2-(5-Chlorchinolin-8-yloxy)-essigsäure-(1-propylpropargyl)-ester,
2-(5-Chlorchinolin-8-yloxy)-essigsäure-(1-methylisobutyl)-ester,
2-(5-Chlorchinolin-8-yloxy)-thioessigsäure-tert.butylester,
2-(5-Chlorchinolin-8-yloxy)-essigsäure-neopentylester,
2-(5-Chlorchinolin-8-yloxy)-thioessigsäure-n-propylester,
2-(5-Chlorchinolin-8-yloxy)-essigsäure-(1-methylhexyl)-ester,
2-(5-Chlorchinolin-8-yloxy)-thioessigsäureäthylester,
2-(5-Chlorchinolin-8-yloxy)-essigsäure-(2-äthylhexyl)-ester,
2-(5-Chlorchinolin-8-yloxy)-thioessigsäure-i-butylester,
2-(5-Chlorchinolin-8-yloxy)-thioessigsäure-n-decylester,
2-(5-Chlorchinolin-8-yloxy)-essigsäure-i-pentylester,
2-(5-Chlorchinolin-8-yloxy)-essigsäure-(1-äthylpentyl)-ester,
2-(5-Chlorchinolin-8-yloxy)-essigsäure-(1-propylbutyl)-ester,
2-(5-Chlorchinolin-8-yloxy)-thioessigsäure-n-hexylester,
2-(5-Chlorchinolin-8-yloxy)-essigsäure-n-hexylester,
2-(5-Chlorchinolin-8-yloxy)-thioessigsäure-i-propylester,
2-(5-Chlorchinolin-8-yloxy)-essigsäure-(1-pentylallyl)-ester,
2-(5-Chlorchinolin-8-yloxy)-essigsäure-(1-methylpentyl)-ester,
2-(5-Chlorchinolin-8-yloxy)-essigsäure-(1,1-dimethylpropargyl)-ester,
2-(5-Chlorchinolin-8-yloxy)-essigsäure-(1-äthyl-1-methylpropargyl)-ester,
2-(5-Chlorchinolin-8-yloxy)-essigsäure-n-butyloxycarbonylmethylester,
2-(5-Chlorchinolin-8-yloxy)-essigsäure-(1-n-butyloxycarbonyläthyl)-ester,
2-(5-Chlorchinolin-8-yloxy)-essigsäure-(1-methylisohexyl)-ester,
2-(5-Chlorchinolin-8-yloxy)-essigsäure-(1-phenylisobutyl)-ester,

2-(5-Chlorchinolin-8-yloxy)-essigsäure-[1-methyl-2-(2-methylphenoxy)-äthyl]-ester,
2-(5-Chlorchinolin-8-yloxy)-essigsäure-(1-phenyläthyl)-ester,
2-(5-Chlorchinolin-8-yloxy)-essigsäure-[1-methyl-2-(4-äthylphenoxy)-äthyl]-ester,
2-(5-Chlorchinolin-8-yloxy)-essigsäure-(1-methyl-2-phenyläthyl)-ester,
2-(5-Chlorchinolin-8-yloxy)-essigsäure-[1-methyl-2-(2-isopropylphenoxy)-äthyl]-ester,
2-(5-Chlorchinolin-8-yloxy)-essigsäure-(1-phenylpropyl)-ester,
2-(5-Chlorchinolin-8-yloxy)-essigsäure-[1-methyl-2-(2-äthylphenoxy)-äthyl]-ester,
2-(5-Chlorchinolin-8-yloxy)-essigsäure-[1-methyl-2-(3-äthylphenoxy)-äthyl]-ester,
2-(5-Chlorchinolin-8-yloxy)-essigsäure-(1-methyl-2-phenoxyäthyl)-ester,
2-(5-Chlorchinolin-8-yloxy)-essigsäure-(1-methyl-3-phenoxypropyl)-ester,
2-(5-Chlorchinolin-8-yloxy)-essigsäure-[1-methyl-2-(3-methylphenoxy)--äthyl]-ester,
2-(5-Chlorchinolin-8-yloxy)-essigsäure-[1-methyl-2-(4-isopropylphenoxy)-äthyl]-ester oder
2-(5-Chlorchinolin-8-yloxy)-essigsäure-[1-methyl-2-(4-methylphenoxy)-äthyl]-ester
und als Herbizidkomponente eine Verbindung aus der Reihe 2-[4-(5-Chlor-3-fluorpyridin-2-yloxy)-phenoxy]-propionsäure-trimethylsilylmethylester, 2R-2-[4-(5-Chlor-3-fluorpyridin-2-yloxy)-phenoxy]-propionsäuretrimethylsilylmethyl ester, 2-[4-(5-Chlor-3-fluorpyridin-2-yloxy)-phenoxy]-propionsäure-trimethylsilyläthylester oder 2R 2-[4-(5-Chlor-3-fluorpyridin-2-yloxy)-phenoxy]-propionsäure-trimethylsilyläthylester.

Besonders bevorzugt sind unter diesen Mitteln diejenigen, die als antagonistische Komponente die Verbindungen
2-(5-Chlorchinolin-8-yloxy)-essigsäuremethallylester,
2-(5-Chlorchinolin-8-yloxy)-essigsäure-(2-phenoxyäthyl)-ester,
2-(5-Chlorchinolin-8-yloxy)-essigsäure-(1-methylbutyl)-ester,
2-(5-Chlorchinolin-8-yloxy)-essigsäure-(1-äthylbutyl)-ester,
2-(5-Chlorchinolin-8-yloxy)-essigsäure-(1-methylisopentyl)-ester,
2-(5-Chlorchinolin-8-yloxy)-essigsäure-(1-methylhexyl)-ester,
2-(5-Chlorchinolin-8-yloxy)-essigsäure-(1-äthylpentyl)-ester,
2-(5-Chlorchinolin-8-yloxy)-essigsäure-(1-propylbutyl)-ester,
2-(5-Chlorchinolin-8-yloxy)-essigsäure-(1-pentylallyl)-ester,
2-(5-Chlorchinolin-8-yloxy)-essigsäure-(1-methylpentyl)-ester,
2-(5-Chlorchinolin-8-yloxy)-essigsäure-(1-methylisohexyl)-ester,
2-(5-Chlorchinolin-8-yloxy)-essigsäure-(1-phenylisobutyl)-ester,
2-(5-Chlorchinolin-8-yloxy)-essigsäure-(1-phenyläthyl)-ester,
2-(5-Chlorchinolin-8-yloxy)-essigsäure-[1-methyl-2-(4-äthylphenoxy)-äthyl]-ester,
2-(5-Chlorchinolin-8-yloxy)-essigsäure-(1-methyl-2-phenyläthyl)-ester,
2-(5-Chlorchinolin-8-yloxy)-essigsäure-(1-phenylpropyl)-ester,
2-(5-Chlorchinolin-8-yloxy)-essigsäure-(1-methyl-2-phenoxyäthyl)-ester,
2-(5-Chlorchinolin-8-yloxy)-essigsäure-(1-methyl-3-phenylpropyl)-ester oder
2-(5-Chlorchinolin-8-yloxy)-essigsäure-[1-methyl-2-(4-methylphenoxy)-äthyl]-ester,
und als herbizide Komponente die Verbindungen 2-[4-(5-Chlor-3-fluorpyridin-2-yloxy)-phenoxy]-propionsäure-trimethylsilylmethylester, 2R-2-[4-(5-Chlor-3-fluorpyridin-2-yloxy)-phenoxy]-propionsäuretrimethylsilylmethylester, 2-[4-(5-Chlor-3-fluorpyridin-2-yloxy)-phenoxy]-propionsäure-trimethylsilyläthylester oder 2R-2-[4-(5-Chlor-3-fluorpyridin-2-yloxy)-phenoxy]-propionsäure-trimethylsilyläthylester enthalten.

Von diesen Mitteln geniessen weiterhin solche Mittel den Vorzug, die die als antagonistische Wirkstoff
2-(5-Chlorchinolin-8-yloxy)-essigsäuremethallylester,
2-(5-Chlorchinolin-8-yloxy)-essigsäure-(2-phenoxyäthyl)-ester,
2-(5-Chlorchinolin-8-yloxy)-essigsäure-(1-methylisopentyl)-ester oder
2-(5-Chlorchinolin-8-yloxy)-essigsäure-(1-methylhexyl)-ester enthalten.

Bei den zu bekämpfenden Unkräutern kann es sich sowohl um monokotyle wie um dikotyle Unkräuter handeln.

Als Kulturpflanzen oder Teile dieser Pflanzen kommen beispielsweise die vorstehend genannten in Betracht. Als Anbauflächen gelten die bereits mit den Kulturpflanzen bewachsenen oder mit dem Saatgut dieser Kulturpflanzen beschickten Bodenareale, wie auch die zur Bebauung mit diesen Kulturpflanzen bestimmten Böden.

Die zu applizierende Aufwandmenge Antidot im Verhältnis zum Herbizid richtet sich weitgehend nach der Anwendungsart. Bei einer Feldbehandlung, welche entweder unter Verwendung einer Tankmischung mit einer Kombination von Antidot und Herbizid oder durch getrennte Applikation von Antidot und Herbizid erfolgt, liegt in der Regel ein Verhältnis von Antidot zu Herbizid von 1:100 bis 10:1, bevorzugt 1:20 bis 1:1, und insbesondere 1:1, vor. Dagegen werden bei der Samenbeizung weit geringere Mengen Antidot im Verhältnis zur Aufwandmenge an Herbizid pro Hektar Anbaufläche benötigt.

In der Regel werden bei der Feldbehandlung 0,001 bis 1 kg Antidot/ha, vorzugsweise 0,01 bis 0,1 kg Antidot/ha, appliziert.

Bei der Samenbeizung werden im allgemeinen 0,01 bis 10 g Antidot/kg Samen, vorzugsweise 0,05 bis 2 g Antidot/kg Samen, appliziert. Wird das Antidot in flüssiger Form kurz vor der Aussaat unter Samenquellung appliziert, so werden zweckmäßigerweise Antidot-Lösungen verwendet, welche den Wirkstoff in einer Konzentration von 1 bis 10 000, vorzugsweise von 10 bis 100 ppm, enthalten.

Zur Applikation werden die Verbindungen der Formel I oder Kombinationen von Verbindungen der Formel I mit den zu antagonisierenden Herbiziden zweckmässigerweise zusammen mit den in der Formulierungstecknik üblichen Hilfsmitteln eingesetzt und werden daher z.B. zu Emulsionskonzentraten, streichfähigen, Pasten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen, in bekannter Weise verarbeitet. Die Anwendungsverfahren wie Versprühen, Vernebeln, Verstäuben, Verstreuen, Bestreichen oder Giessen werden gleich wie die Art der zu verwendenden Mittel den angestrebten Zielen und den gegebenen Verhältnissen entsprechend gewählt.

Die Formulierungen, d.h. die den Wirkstoff der Formel I oder eine Kombination von Wirkstoff der Formel I mit zu antagonsierendem Herbizid und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage, Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach Art des zu formulierenden Wirkstoffs der Formel I und gegebenenfalls auch dem zu antagonisierenden Herbizid nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen seien die Alkali-, Erdalkali- oder gegebenenfalls substistuierten Ammoniumsalze von höheren Fettsäuren ($C_{10}$-$C_{22}$), wie z.B. die Na- oder K-Salze oder der Oel- oder Stearinsäure, oder von natürlichen Fettsäuregemischen, die z.B. aus Kokosnuss- oder Talgöl gewonnen werden können, genannt. Ferner sind auch die Fettsäure-methyltaurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz ser Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxyd-Addukten. Die sulfonierten Benzimidazolderivate enthalten vorzugsweise 2-Sulfonsäuregruppen und einen Fettsäurest mit 8 bis 22 C-Atomen. Alkylarysulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten

niedrige, gegebenenfalls halogenierte Alkyl-, Benzyl- oder niedrige Hydroxyalkylrest aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:
"Mc Cutcheon's Detergens and Emulsifiers Annual"
MC Publishing Corp., Ridgewood New Jersey, 1981.
Stache, H., "Tensid-Taschenbuch",
Carl Hanser Verlag, München/Wien, 1981.

Die agrochemischen Zubereitungen enthalten in der Regel 0,1 bis 99 Gewichtsprozent, insbesondere 0,1 bis 95 Gew.-%, Wirkstoff der Formel I oder Wirkstoffgemisch Antidot/Herbizid, 1 bis 99,9 Gew.-%, insbesondere 5 bis 99,8 Gew.-%, eines festen oder flüssigen Zusatzstoffes und 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Die Mittel können auch weiter Zusätze wie Stabilisatoren, Entschäumer, Viskositätsregulatoren, Bindemittel, Haftmittel sowie Dünger oder andere Wirkstoffe zur Erzielung spezieller Effekte enthalten.

Für die Verwendung von Verbindungen der Formel I oder sie enthaltender Mittel zum Schützen von Kulturpflanzen gegen schädigende Wirkungen von Herbiziden der Formel II kommen verschiedene Methoden und Techniken in Betracht, wie beispielsweise die folgenden:

i) Samenbeizung

a) Beizung der Samen mit einem als Spritzpulver formulierten Wirkstoff der Formel I durch Schütteln in einem Gefäss bis zur gleichmässigen Verteilung auf der Samenoberfläche (Trockenbeizung). Man verwendet dabei etwa 1 bis 500 g Wirkstoff der Formel I (4 g bis 2 kg Spritzpulver) pro 100 kg Saatgut.

b) Beizung der Samen mit einem Emulsionskonzentrat des Wirkstoffs der Formel I nach der Methode a) (Nassbeizung).

c) Beizung durch Tauchen des Saatguts in eine Brühe mit 1-10 000 ppm Wirkstoff der Formel I während 1 bis 72 Stunden und gegebenenfalls nachfolgendes Trocknen der Samen (Tauchbeizung).

Die Beizung des Saatguts oder die Behandlung des angekeimten Sämlings sind naturgemäss die bevorzugten Methoden der Applikation, weil die Wirkstoffbehandlung vollständig auf die Zielkultur gerichtet ist. Man verwendet in der Regel 1 bis 500 g Antidot, vorzugsweise 5 bis 250 g Antidot, pro 100 kg Saatgut, wobei man je nach Methodik, die auch den Zusatz anderer Wirkstoffe oder Mikronährstoffe ermöglicht, von den angegebenen Grenzkonzentrationen nach oben oder unten abweichen kann (Wiederholungsbeize).

ii) Applikation aus Tankmischung

Eine flüssige Aufarbeitung eines Gemisches von Antidot und Herbizid (gegenseitiges Mengenverhältnis zwischen 10:1 und 1:100) wird verwendet, wobei die Aufwandmenge an Herbizid 0,001 bis 10 kg pro Hektar beträgt. Solche Tankmischung wird vor oder nach der Aussaat appliziert.

iii) Applikation in der Saatfurche

Das Antidot wird als Emulsionskonzentrat, Spritzpulver oder als Granulat in die offene besäte Saatfurche eingebracht und hierauf wird nach dem Decken der Saatfurche in normaler Weise das Herbizid im Vorauflaufverfahren appliziert.

iv) Kontrollierte Wirkstoffabgabe

Der Wirkstoff der Formel I wird in Lösung auf mineralische Granulatträger oder polymerisierte Granulate (Harnstoff/Formaldehyde) aufgezogen und trocknen gelassen. Gegebenenfalls kann ein Ueberzug aufgebracht werden (Umhüllungsgranulate), der es erlaubt, den Wirkstoff über einen bestimmten Zeitraum dosiert abzugeben.

Formulierungsbeispiel für flüssige Wirkstoffe der Formel I (% = Gewichtsprozent)

| 1. Emulsions-Konzentrate | a) | b) | c) |
|---|---|---|---|
| Wirkstoff aus Tabelle 1 | 25 % | 40 % | 50 % |
| Ca-Dodecylbenzolsulfonat | 5 % | 8 % | 6 % |
| Ricinusöl-polyäthylenglykoläther (36 Mol AeO) | 5 % | - | - |
| Tributylphenol-polyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2. Lösungen | a) | b) | c) | d) |
|---|---|---|---|---|
| Wirkstoff aus Tabelle 1 | 80 % | 10 % | 5 % | 95 % |
| Aethylenglykol-monomethyl-äther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°) | - | - | 94 % | - |

Die Lösungen sind zur Anwendung in form kleinster Tropfen geeignet.

| 3. Granulate | a) | b) |
|---|---|---|
| Wirkstoff aus Tabelle 1 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 4. Stäubemittel | a) | b) |
|---|---|---|
| Wirkstoff aus Tabelle 1 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemitel.

Formulierungsbeispiel für feste Wirkstoffe der Formel I (% = Gewichtsprozent)

| 5. Spritzpulver | a) | b) | c) |
|---|---|---|---|
| Wirkstoff aus Tabelle 1 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

6. Emulsions-Konzentrate

Wirkstoffe aus Tabelle 1 10 %
Octylphenolpolyäthylenglykoläther (4-5 Mol AeO) 3 %
Ca-Dodecylbenzolsulfonat 3 %
Ricinusölpolyglykoläther (35 Mol AeO) 4 %
Cyclohexanon 30 %
Xylolgemische 50 %

Aus diesem Konzentrat können durch Verdünnen mit Wasser Emulsionen jeder gewünschten Konzentration hergestellt werden.

| 7. Stäubemittel | a) | b) |
|---|---|---|
| Wirkstoff aus Tabelle 1 | 5 % | 8 % |
| Talkum | 95 % | - |
| Kaolin | - | 92 % |

Man erhält anwendungsfertige Stäubemittel, indem der Wirkstoff mit den Trägerstoffen vermischt und auf einer geeigneten Mühle vermahlen wird.

8. Extruder-Granulate

Wirkstoff aus Tabelle 1 10 %
Na-Ligninsulfonat 2 %
Carboxymethylcellulose 1 %
Kaolin 87 %

Der Wirkstoff wird mit den Zusatzstoffen vermischt, vermahlen und mit Wasser angefeuchtet. Dieses Gemisch wird extrudiert und anschliessend im Luftstrom getrocknet.

9. Umhüllungs-Granulate

Wirkstoff aus Tabelle 1 3 %
Polyäthylenglykol (MG 200) 3 %
Kaolin 94 %

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

10. Suspensions-Konzentrate

Wirkstoff aus Tabelle 1 40 %
Aethylenglykol 10 %
Nonylphenolpolyäthylenglykoläther (15 Mol AeO) 6 %
Na-Ligninsulfonat 10 %
Carboxymethylcellulose 1 %

37%ige wässrige Formaldehyde-Lösung 0,2 %
Silikonöl in Form einer 75%igen wässrigen Emulsion 0,8 %
Wasser 32 %

Der feingemahlene Wirkstoff wird mit den Zusatztoffen innig vermischt. Man erhält so ein Suspensions-Konzentrat, aus welchem durch Verdünnen mit Wasser Suspensionen jeder gewünschten Konzentration hergestellt werden können.

Formulierungsbeispiel für Wirkstoffgemische (flüssig) (% = Gewichtsprozent)

| 11. Emulsions-Konzentrate | a) | b) | c) |
|---|---|---|---|
| Wirkstoffgemisch:Antidot aus Tabelle 1 und ein Herbizid der Formel II im Verhältnis 1:1 | 25 % | 40 % | 50 % |
| Ca-Dodecylbenzolsulfonat | 5 % | 8 % | 6 % |
| Ricinusöl-polyäthylenglykoläther (36 Mol AeO) | 5 % | - | - |
| Tributylphenol-polyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 12. Emulsions-Konzentrate | a) | b) | c) |
|---|---|---|---|
| Wirkstoffgemisch: Antidot aus Tabelle 1 und ein Herbizid der Formel II im Verhältnis 1:3 | 25 % | 40 % | 50 % |
| Ca-Dodecylbenzolsulfonat | 5 % | 8 % | 6 % |
| Ricinusöl-polyäthylenglykoläther (36 Mol AeO) | 5 % | - | - |
| Tributylphenol-polyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

**13. Emulsions-Konzentrate**

| | a) | b) | c) |
|---|---|---|---|
| Wirkstoffgemisch: Antidot aus Tabelle 1 und ein Herbizid der Formel II im Verhältnis 2:1 | 25 % | 40 % | 50 % |
| Ca-Dodecylbenzolsulfonat | 5 % | 8 % | 6 % |
| Ricinusöl-polyäthylenglykoläther (36 Mol AeO) | 5 % | - | - |
| Tributylphenol-polyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

**14. Emulsions-Konzentrate**

| | a) | b) | c) |
|---|---|---|---|
| Wirkstoffgemisch: Antidot aus Tabelle 1 und 2-[4-(5-Chlor-3-fluorpyridin-2-yloxy)-phenoxy]-propionsäure-trimethylsilyl-methylester im Verhältnis 1:1 | 25 % | 40 % | 50 % |
| Ca-Dodecylbenzolsulfonat | 5 % | 8 % | 6 % |
| Ricinusöl-polyäthylenglykoläther (36 Mol AeO) | 5 % | - | - |
| Tributylphenol-polyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 15. Emulsions-Konzentrat | a) | b) | c) |
|---|---|---|---|
| Wirkstoffgemisch: Antidot aus Tabelle 1 und 2-[4-(5-Chlor-3-fluorpyridin-2-yloxy)-phenoxy]-propionsäure-trimethylsilyl-methylester im Verhältnis 1:3 | 25 % | 40 % | 50 % |
| Ca-Dodecylbenzolsulfonat | 5 % | 8 % | 6 % |
| Ricinusöl-polyäthylenglykoläther (36 Mol AeO) | 5 % | - | - |
| Tributylphenol-polyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 16. Lösungen | a) | b) | c) | d) |
|---|---|---|---|---|
| Wirkstoffgemisch: Antidot aus Tabelle 1 und ein Herbizid der Formel II im Verhältnis 1:4 | 80 % | 10 % | 5 % | 95 % |
| Aethylenglykol-monomethyl-äther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| 17. Lösungen | a) | b) | c) | d) |
|---|---|---|---|---|
| Wirkstoffgemisch: Antidot aus Tabelle 1 und ein Herbizid der Formel II im Verhältnis 5:2 | 80 % | 10 % | 5 % | 95 % |
| Aethylenglykol-monomethyl-äther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| 18. Lösungen | a) | b) | c) | d) |
|---|---|---|---|---|
| Wirkstoffgemisch: Antidot aus Tabelle 1 und ein Herbizid der Formel II im Verhältnis 1:1 | 80 % | 10 % | 5 % | 95 % |
| Aethylenglykol-monomethyl-äther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| 19. Lösungen | a) | b) | c) | d) |
|---|---|---|---|---|
| Wirkstoffgemisch: Antidot aus Tabelle 1 und 2-[4-(5-Chlor-3-fluorpyridin-2-yloxy)-phenoxy]-propionsäure-trimethylsilyl-äthylester im Verhältnis 1:1 | 80 % | 10 % | 5 % | 95 % |
| Aethylenglykol-monomethyl-äther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| 20. Lösungen | a) | b) | c) | d) |
|---|---|---|---|---|
| Wirkstoffgemisch: Antidot aus Tabelle 1 und 2-[4-(5-Chlor-3-fluorpyridin-2-yloxy)-phenoxy]-propionsäure-trimethylsilyl-äthylester im Verhältnis 1:4 | 80 % | 10 % | 5 % | 95 % |
| Aethylenglykol-monomethyl-äther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| 21. Granulate | a) | b) |
|---|---|---|
| Wirkstoffgemisch: Antidot aus Tabelle 1 und ein Herbizid der Formel II im Verhältnis 1:1 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 22. Granulate | a) | b) |
|---|---|---|
| Wirkstoffgemisch: Antidot aus Tabelle 1 und 2-[4-(5-Chlor-3-fluorpyridin-2-yloxy)-phenoxy]-propionsäure-trimethylsilyl-methylester im Verhältnis 1:1 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 23. Stäubemittel | a) | b) |
|---|---|---|
| Wirkstoffgemisch: Antidot aus Tabelle 1 und ein Herbizid der Formel II im Verhältnis 1:1 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungsbeispiele für Wirkstoffgemische (fest) (% = Gewichtsprozent)

24. Spritzpulver

| | a) | b) | c) |
|---|---|---|---|
| Wirkstoffgemisch: Antidot aus Tabelle 1 und ein Herbizid der Formel II im Verhältnis 1:1 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

25. Spritzpulver

| | a) | b) | c) |
|---|---|---|---|
| Wirkstoffgemisch: Antidot aus Tabelle 1 und ein Herbizid der Formel II im Verhältnis 1:4 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 26. Spritzpulver | a) | b) | c) |
|---|---|---|---|
| Wirkstoffgemisch: Antidot aus Tabelle 1 und ein Herbizid der Formel II im Verhältnis 3:1 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

27. Emulsions-Konzentrate
Wirkstoffgemisch: Antidot aus Tabelle 1 und ein Herbizid der Formel II im Verhältnis 1:1 10 %
Octylphenolpolyäthylenglykoläther (4-5 Mol AeO) 3 %
Ca-Dodecylbenzolsulfonat 3 %
Ricinusölpolyglykoläther (35 Mol Aeo) 4 %
Cyclohexanon 30 %
Xylolgemisch 50 %
Aus diesem Konzentrat können durch Verdünnen mit Wasser Emulsionen jeder gewünschten Konzentration hergestellt werden.

28. Emulsions-Konzentrate
Wirkstoffgemisch: Antidot aus Tabelle 1 und ein Herbizid der Formel II im Verhältnis 5:2 10 %
Octylphenolpolyäthylenglykoläther (4-5 Mol AeO) 3 %
Ca-Dodecylbenzolsulfonat 3 %
Ricinusölpolyglykoläther (35 Mol AeO) 4 %
Cyclohexanon 30 %
Xylolgemisch 50 %
Aus diesem Konzentrat können durch Verdünnen mit Wasser Emulsionen jeder gewünschten Konzentration hergestellt werden.

29. Emulsions-Konzentrate
Wirkstoffgemisch: Antidot aus Tabelle 1 und ein Herbizid der Formel II im Verhältnis 1:4 10 %
Octylphenolpolyäthylenglykoläther (4-5 Mol AeO) 3 %
Ca-Dodecylbenzolsulfonat 3 %
Ricinusölpolyglykoläther (35 Mol AeO) 4 %
Cyclohexanon 30 %
Xylolgemisch 50 %
Aus diesem Konzentrat können durch Verdünnen mit Wasser Emulsionen jeder gewünschten Konzentration hergestellt werden.

| 30. Stäubemittel | a) | b) |
|---|---|---|
| Wirkstoffgemisch: Antidot aus Tabelle 1 und ein Herbizid der Formel II im Verhältnis 1:1 | 5 % | 8 % |
| Talkum | 95 % | - |
| Kaolin | - | 92 % |

Man erhält anwendungsfertige Stäubemittel, indem der Wirkstoff mit den Trägerstoffen vermischt und auf einer geeigneten Mühle vermahlen wird.

31. Extruder-Granulate

Wirkstoffgemisch: Antidot aus Tabelle 1 und ein Herbizid der Formel II im Verhältnis 1:1 10 %
Na-Ligninsulfonat 2 %
Carboxymethylcellulose 1 %
Kaolin 87 %

Der Wirkstoff wird mit den Zusatzstoffen vermischt, vermahlen und mit Wasser angefeuchtet. Dieses Gemisch wird extrudiert und anschliessend im Luftstrom getrocknet.

32. Umhüllungs-Granulate

Wirkstoffgemisch: Antidot aus Tabelle 1 und ein Herbizid der Formel II im Verhältnis 1:1 3 %
Polyäthylenglykol (MG 200) 3 %
Kaolin 94 %

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

33. Suspensions-Konzentrate

Wirkstoffgemisch: Antidot aus Tabelle 1 und ein Herbizid der Formel II im Verhältnis 1:1 40 %
Aethylenglykol 10 %
Nonylphenolpolyäthylenglykoläther (15 Mol AeO) 6 %
Na-Ligninsulfonat 10 %
Carboxymethylcellulose 1 %
37%ige wässrige Formaldehyd-Lösung 0,2 %
Silikonöl in Form einer 75%igen wässrigen Emulsion 0,8 %
Wasser 32 %

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

34. Suspensions-Konzentrate

Wirkstoffgemisch: Antidot aus Tabelle 1 und ein Herbizid der Formel II im Verhältnis 1:4 40%
Aethylenglykol 10 %
Nonylphenolpolyäthylenglykoläther (15 Mol AeO) 6 %
Na-Ligninsulfonat 10 %
Carboxymethylcellulose 1 %
37%ige wässrige Formaldehyd-Lösung 0,2 %
Silikonöl in Form einer 75%igen wässrigen Emulsion 0,8 %
Wasser 32 %

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

35. Suspensions-Konzentrate

Wirkstoffgemisch: Antidot aus Tabelle 1 und ein Herbizid der Formel II im Verhältnis 3:1 40%
Aethylenglykol 10%
Nonylphenolpolyäthylenglykoläther (15 Mol AeO) 6%
Na-Ligninsulfonat 10%
Carboxymethylcellulose 1%
37%ige wässrige Formaldehyd-Lösung 0,2%
Silifonöl in Form einer 75%igen wässrigen Emulsion 0,8%
Wasser 32%

Der fein gemahlene Wirkstoff wird mit den Zusatztoffen innig vermischt. Man erhält so ein Suspensions-Konzentrat, aus welchem durch Verdünnen mit Wasser Suspensionen jeder gewünschten Konzentration hergestellt werden können.

Biologische Beispiele

Testbeschreibung

Im Gewächshaus werden in Plastiktöpfen, welche 0,5 l Erde enthalten, Samen der zu testenden Pflanzen ausgesät. Wenn die Pflanzen das 2-bis 3-Blattstadium erreicht haben, werden ein Safener der Formel 1 und ein Herbizid der Formel II zusammen als Tankmischung appliziert. 14 Tage nach der Applikation wird die Schutzwirkung des Safeners in Prozent bonitiert. Als Referenz dienen dabei mit dem Herbizid allein behandelte Pflanzen sowie die vollstängig unbehandelte Kontrolle. Die zur Charakterisierung des

antagonistischen Effekts angegebene "relative Schutzwirkung in Prozent" berechnet sich aus der Differenz der Schädigungsgrade in Prozent durch das Herbizid bei An- und Abwesenheit der Safenerkomponente. Die festgestellten Schädigungsgrade werden nach einer linearen Skala zwischen vollständiger Abtötung der Versuchspflanze (100 % Herbizidwirkung) und unbeeinflusstem Pflanzenwachstum der unbehandelten Kontrollpflanze (0 % Herbizidwirkung) bestimmt.

In Weizen- und Gerstekulturen zeigen die geprüften Mischungen gegenüber den nur mit Herbizid behandelten Pflanzen einen deutlichen Schutzeffekt.

Die Prüfresultate sind in der nachfolgenden Tabelle 3 dargestellt.

Tabelle 3:

Relative Schutzwirkung in Prozent in Sommerweizen, Sorte "Besso" und Sommergerste, Sorte "Cornel".

(-: nicht geprüft)

| Safener Verb.Nr. | Aufwandmenge g AS/ha | Herbizid Nr. | Aufwandmenge g AS/ha | Relative Schutzwirkung in Weizen in % | Relative Schutzwirkung in Gerste in % |
|---|---|---|---|---|---|
| 1.125 | 8 | 2.17 | 62 | - | 45 |
| 1.125 | 16 | 2.17 | 62 | - | 45 |
| 1.125 | 31 | 2.17 | 62 | - | 45 |
| 1.125 | 62 | 2.17 | 62 | - | 45 |
| 1.125 | 16 | 2.17 | 125 | 60 | 65 |
| 1.125 | 31 | 2.17 | 125 | 60 | 70 |
| 1.125 | 62 | 2.17 | 125 | 65 | 70 |
| 1.125 | 125 | 2.17 | 125 | 60 | 60 |
| 1.125 | 31 | 2.17 | 250 | 55 | 35 |
| 1.125 | 62 | 2.17 | 250 | 70 | 30 |
| 1.125 | 125 | 2.17 | 250 | 75 | 55 |
| 1.125 | 250 | 2.17 | 250 | 55 | 65 |
| 1.125 | 62 | 2.17 | 500 | 55 | - |
| 1.125 | 125 | 2.17 | 500 | 60 | - |
| 1.125 | 250 | 2.17 | 500 | 65 | - |
| 1.125 | 500 | 2.17 | 500 | 60 | - |
| 1.125 | 8 | 2.18 | 62 | - | 35 |
| 1.125 | 16 | 2.18 | 62 | - | 40 |
| 1.125 | 31 | 2.18 | 62 | - | 50 |
| 1.125 | 62 | 2.18 | 62 | - | 50 |
| 1.125 | 16 | 2.18 | 125 | 65 | 30 |
| 1.125 | 31 | 2.18 | 125 | 60 | 50 |
| 1.125 | 62 | 2.18 | 125 | 60 | 50 |
| 1.125 | 125 | 2.18 | 125 | 60 | 55 |
| 1.125 | 31 | 2.18 | 250 | 65 | 25 |
| 1.125 | 62 | 2.18 | 250 | 70 | 35 |
| 1.125 | 125 | 2.18 | 250 | 75 | 45 |
| 1.125 | 250 | 2.18 | 250 | 70 | 55 |
| 1.125 | 62 | 2.18 | 500 | 55 | - |
| 1.125 | 125 | 2.18 | 500 | 65 | - |
| 1.125 | 250 | 2.18 | 500 | 65 | - |
| 1.125 | 500 | 2.18 | 500 | 60 | - |
| 1.125 | 8 | 2.19 | 62 | - | 55 |
| 1.125 | 15 | 2.19 | 62 | - | 55 |
| 1.125 | 31 | 2.19 | 62 | - | 55 |
| 1.125 | 62 | 2.19 | 62 | - | 40 |

| Safener Verb.Nr. | Aufwand-menge g AS/ha | Herbizid Nr. | Aufwand-menge g AS/ha | Relative Schutzwirkung in Weizen in % | Relative Schutzwirkung in Gerste in % |
|---|---|---|---|---|---|
| 1.125 | 16 | 2.19 | 125 | 65 | 45 |
| 1.125 | 31 | 2.19 | 125 | 70 | 50 |
| 1.125 | 62 | 2.19 | 125 | 70 | 70 |
| 1.125 | 125 | 2.19 | 125 | 65 | 70 |
| 1.125 | 31 | 2.19 | 250 | 70 | 40 |
| 1.125 | 62 | 2.19 | 250 | 75 | 45 |
| 1.125 | 125 | 2.19 | 250 | 80 | 45 |
| 1.125 | 250 | 2.19 | 250 | 75 | 45 |
| 1.125 | 62 | 2.19 | 500 | 70 | - |
| 1.125 | 125 | 2.19 | 500 | 70 | - |
| 1.125 | 250 | 2.19 | 500 | 85 | - |
| 1.125 | 500 | 2.19 | 500 | 70 | - |
| 1.125 | 8 | 2.20 | 62 | - | 50 |
| 1.125 | 15 | 2.20 | 62 | - | 55 |
| 1.125 | 31 | 2.20 | 62 | - | 60 |
| 1.125 | 62 | 2.20 | 62 | - | 55 |
| 1.125 | 15 | 2.20 | 125 | 70 | 35 |
| 1.125 | 31 | 2.20 | 125 | 75 | 45 |
| 1.125 | 62 | 2.20 | 125 | 80 | 50 |
| 1.125 | 125 | 2.20 | 125 | 75 | 60 |
| 1.125 | 31 | 2.20 | 250 | 65 | 35 |
| 1.125 | 62 | 2.20 | 250 | 70 | 40 |
| 1.125 | 125 | 2.20 | 250 | 75 | 45 |
| 1.125 | 250 | 2.20 | 250 | 70 | 55 |
| 1.125 | 62 | 2.20 | 500 | 50 | - |
| 1.125 | 125 | 2.20 | 500 | 60 | - |
| 1.125 | 250 | 2.20 | 500 | 60 | - |
| 1.125 | 500 | 2.20 | 500 | 60 | - |
| 1.188 | 8 | 2.17 | 62 | - | 25 |
| 1.188 | 15 | 2.17 | 62 | - | 35 |
| 1.188 | 31 | 2.17 | 62 | - | 40 |
| 1.188 | 62 | 2.17 | 62 | - | 35 |
| 1.188 | 15 | 2.17 | 125 | 70 | 45 |
| 1.188 | 31 | 2.17 | 125 | 70 | 55 |
| 1.188 | 62 | 2.17 | 125 | 70 | 70 |
| 1.188 | 125 | 2.17 | 125 | 70 | 60 |
| 1.188 | 31 | 2.17 | 250 | 70 | 30 |
| 1.188 | 62 | 2.17 | 250 | 70 | 40 |
| 1.188 | 125 | 2.17 | 250 | 75 | 45 |
| 1.188 | 250 | 2.17 | 250 | 75 | 50 |

| Safener Verb.Nr. | Aufwand-menge g AS/ha | Herbizid Nr. | Aufwand-menge g AS/ha | Relative Schutzwirkung in Weizen in % | Relative Schutzwirkung in Gerste in % |
|---|---|---|---|---|---|
| 1.188 | 62 | 2.17 | 500 | 55 | - |
| 1.188 | 125 | 2.17 | 500 | 65 | - |
| 1.188 | 250 | 2.17 | 500 | 65 | - |
| 1.188 | 500 | 2.17 | 500 | 65 | - |
| 1.188 | 8 | 2.18 | 62 | - | 25 |
| 1.188 | 15 | 2.18 | 62 | - | 40 |
| 1.188 | 31 | 2.18 | 62 | - | 50 |
| 1.188 | 62 | 2.18 | 62 | - | 40 |
| 1.188 | 15 | 2.18 | 125 | 55 | 30 |
| 1.188 | 31 | 2.18 | 125 | 60 | 25 |
| 1.188 | 62 | 2.18 | 125 | 55 | 35 |
| 1.188 | 125 | 2.18 | 125 | 60 | 40 |
| 1.188 | 31 | 2.18 | 250 | 55 | 15 |
| 1.188 | 62 | 2.18 | 250 | 55 | 20 |
| 1.188 | 125 | 2.18 | 250 | 65 | 20 |
| 1.188 | 250 | 2.18 | 250 | 65 | 30 |
| 1.188 | 62 | 2.18 | 500 | 35 | - |
| 1.188 | 125 | 2.18 | 500 | 50 | - |
| 1.188 | 250 | 2.18 | 500 | 60 | - |
| 1.188 | 500 | 2.18 | 500 | 65 | - |
| 1.188 | 8 | 2.19 | 62 | - | 60 |
| 1.188 | 15 | 2.19 | 62 | - | 60 |
| 1.188 | 31 | 2.19 | 62 | - | 60 |
| 1.188 | 62 | 2.19 | 62 | - | 55 |
| 1.188 | 15 | 2.19 | 125 | 70 | 45 |
| 1.188 | 31 | 2.19 | 125 | 70 | 60 |
| 1.188 | 62 | 2.19 | 125 | 70 | 65 |
| 1.188 | 125 | 2.19 | 125 | 70 | 55 |
| 1.188 | 31 | 2.19 | 250 | 70 | 30 |
| 1.188 | 62 | 2.19 | 250 | 85 | 30 |
| 1.188 | 125 | 2.19 | 250 | 85 | 45 |
| 1.188 | 250 | 2.19 | 250 | 85 | 60 |
| 1.188 | 62 | 2.19 | 500 | 65 | - |
| 1.188 | 125 | 2.19 | 500 | 60 | - |
| 1.188 | 250 | 2.19 | 500 | 75 | - |
| 1.188 | 500 | 2.19 | 500 | 75 | - |
| 1.188 | 8 | 2.20 | 62 | - | 50 |
| 1.188 | 15 | 2.20 | 62 | - | 50 |
| 1.188 | 31 | 2.20 | 62 | - | 60 |
| 1.188 | 62 | 2.20 | 62 | - | 60 |

| Safener Verb.Nr. | Aufwand-menge g AS/ha | Herbizid Nr. | Aufwand-menge g AS/ha | Relative Schutzwirkung in Weizen in % | Relative Schutzwirkung in Gerste in % |
|---|---|---|---|---|---|
| 1.188 | 15 | 2.20 | 125 | 55 | 35 |
| 1.188 | 31 | 2.20 | 125 | 70 | 35 |
| 1.188 | 62 | 2.20 | 125 | 70 | 50 |
| 1.188 | 125 | 2.20 | 125 | 65 | 55 |
| 1.188 | 31 | 2.20 | 250 | 70 | 25 |
| 1.188 | 62 | 2.20 | 250 | 75 | 25 |
| 1.188 | 125 | 2.20 | 250 | 75 | 25 |
| 1.188 | 250 | 2.20 | 250 | 70 | 40 |
| 1.188 | 62 | 2.20 | 500 | 55 | - |
| 1.188 | 125 | 2.20 | 500 | 60 | - |
| 1.188 | 250 | 2.20 | 500 | 60 | - |
| 1.188 | 500 | 2.20 | 500 | 65 | - |
| 1.316 | 8 | 2.17 | 62 | - | 40 |
| 1.316 | 15 | 2.17 | 62 | - | 30 |
| 1.316 | 31 | 2.17 | 62 | - | 35 |
| 1.316 | 62 | 2.17 | 62 | - | 45 |
| 1.316 | 15 | 2.17 | 125 | 70 | 50 |
| 1.316 | 31 | 2.17 | 125 | 70 | 65 |
| 1.316 | 62 | 2.17 | 125 | 70 | 65 |
| 1.316 | 125 | 2.17 | 125 | 70 | 55 |
| 1.316 | 31 | 2.17 | 250 | 65 | 30 |
| 1.316 | 62 | 2.17 | 250 | 75 | 50 |
| 1.316 | 125 | 2.17 | 250 | 75 | 60 |
| 1.316 | 250 | 2.17 | 250 | 70 | 60 |
| 1.316 | 62 | 2.17 | 500 | 55 | - |
| 1.316 | 125 | 2.17 | 500 | 65 | - |
| 1.316 | 250 | 2.17 | 500 | 70 | - |
| 1.316 | 500 | 2.17 | 500 | 65 | - |
| 1.316 | 8 | 2.18 | 62 | - | 35 |
| 1.316 | 15 | 2.18 | 62 | - | 35 |
| 1.316 | 31 | 2.18 | 62 | - | 40 |
| 1.316 | 62 | 2.18 | 62 | - | 45 |
| 1.316 | 125 | 2.18 | 125 | 55 | 30 |
| 1.316 | 125 | 2.18 | 125 | 65 | 40 |
| 1.316 | 125 | 2.18 | 125 | 60 | 35 |
| 1.316 | 125 | 2.18 | 125 | 60 | 50 |
| 1.316 | 31 | 2.18 | 250 | 70 | 30 |
| 1.316 | 62 | 2.18 | 250 | 70 | 30 |
| 1.316 | 125 | 2.18 | 250 | 75 | 45 |
| 1.316 | 250 | 2.18 | 250 | 75 | 50 |

| Safener Verb.Nr. | Aufwand-menge g AS/ha | Herbizid Nr. | Aufwand-menge g AS/ha | Relative Schutzwirkung in Weizen in % | Relative Schutzwirkung in Gerste in % |
|---|---|---|---|---|---|
| 1.316 | 62 | 2.18 | 500 | 55 | - |
| 1.316 | 125 | 2.18 | 500 | 60 | - |
| 1.316 | 250 | 2.18 | 500 | 65 | - |
| 1.316 | 500 | 2.18 | 500 | 60 | - |
| 1.316 | 8 | 2.19 | 62 | - | 55 |
| 1.316 | 15 | 2.19 | 62 | - | 60 |
| 1.316 | 31 | 2.19 | 62 | - | 60 |
| 1.316 | 62 | 2.19 | 62 | - | 50 |
| 1.316 | 15 | 2.19 | 125 | 70 | 50 |
| 1.316 | 31 | 2.19 | 125 | 70 | 45 |
| 1.316 | 62 | 2.19 | 125 | 65 | 55 |
| 1.316 | 125 | 2.19 | 125 | 65 | 50 |
| 1.316 | 31 | 2.19 | 250 | 80 | 35 |
| 1.316 | 62 | 2.19 | 250 | 75 | 35 |
| 1.316 | 125 | 2.19 | 250 | 70 | 55 |
| 1.316 | 250 | 2.19 | 250 | 80 | 60 |
| 1.316 | 62 | 2.19 | 500 | 80 | - |
| 1.316 | 125 | 2.19 | 500 | 80 | - |
| 1.316 | 250 | 2.19 | 500 | 90 | - |
| 1.316 | 500 | 2.19 | 500 | 75 | - |
| 1.316 | 8 | 2.20 | 62 | - | 60 |
| 1.316 | 15 | 2.20 | 62 | - | 60 |
| 1.316 | 31 | 2.20 | 62 | - | 60 |
| 1.316 | 62 | 2.20 | 62 | - | 55 |
| 1.316 | 15 | 2.20 | 125 | 80 | 30 |
| 1.316 | 31 | 2.20 | 125 | 80 | 35 |
| 1.316 | 62 | 2.20 | 125 | 75 | 45 |
| 1.316 | 125 | 2.20 | 125 | 75 | 50 |
| 1.316 | 31 | 2.20 | 250 | 60 | 35 |
| 1.316 | 62 | 2.20 | 250 | 65 | 30 |
| 1.316 | 125 | 2.20 | 250 | 80 | 45 |
| 1.316 | 250 | 2.20 | 250 | 70 | 55 |
| 1.316 | 62 | 2.20 | 500 | 60 | - |
| 1.316 | 125 | 2.20 | 500 | 65 | - |
| 1.316 | 250 | 2.20 | 500 | 65 | - |
| 1.316 | 500 | 2.20 | 500 | 65 | - |

**Patentansprüche**

1. Verfahren zum Schützen von Kulturpflanzen gegen schädigende Wirkungen herbizid wirksamer 2-[4-(5-Chlor-3-fluorpyridin-2-yloxy)-phenoxy]-propionsäure-Derivate der Formel II

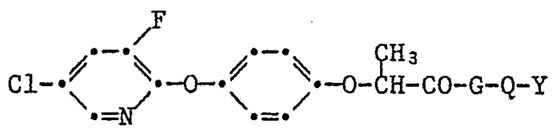

(II)

worin
G Sauerstoff oder Schwefel,
Q eine unsubstituierte oder einfach oder zweifach durch Methyl oder Phenyl substituierte $C_1$-$C_3$-Alkylenkette, und
Y eine Gruppe $-Si(OR^{17})_2R^{18}$, $-Si(R^{18})_3$, $-PO(OR^{19})-OR^{20}$, $-PO(OR^{19})-R^{20}$ oder $-O-PO(OR^{19})_2$ bedeuten, wobei
$R^{17}$ und $R^{18}$ unabhängig voneinander für $C_1$-$C_6$-Alkyl und
$R^{19}$ und $R^{20}$ unabhängig voneinander für Wasserstoff, $C_1$-$C_4$-Alkyl, $C_3$-$C_4$-Alkenyl, $C_3$-$C_4$-Alkinyl, $C_2$-$C_4$-Halogenalkyl, $C_1$-$C_4$-Alkoxy-($C_2$-$C_4$)-alkyl, $C_1$-$C_4$-Cyanoalkyl oder einen unsubstituierten oder durch Halogen, $C_1$-$C_4$-Alkyl, $C_1$-$C_4$-Alkoxy, $C_1$-$C_4$-Halogenalkyl, $C_1$-$C_4$-Halogenalkoxy, Nitro oder Cyano ein- bis zweifach substituierten Phenyl-, Phenyl-($C_1$-$C_4$)-alkyl oder Naphthylrest stehen,
dadurch gekennzeichnet, dass man die Kulturpflanzen, Teile dieser Pflanzen oder für den Anbau der Kulturpflanzen bestimmte Böden mit einer Verbindung der Formel I

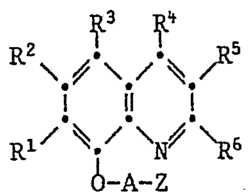

(I),

worin $R^1$, $R^2$ und $R^3$ unabhängig voneinander Wasserstoff, Halogen, Nitro, Cyano, $C_1$-$C_3$-Alkyl oder $C_1$-$C_3$-Alkoxy,
$R^4$, $R^5$ und $R^6$ unabhängig voneinander Wasserstoff, Halogen oder $C_1$-$C_3$-Alkyl,
A eine der Gruppen $-CH_2-$, $-CH_2-CH_2-$ oder $-CH(CH_3)-$ und
Z Cyan oder Amidoxim, welches am Sauerstoffatom acyliert sein kann, eine Carboxylgruppe oder ein Salz davon, eine Mercaptocarbonylgruppe oder ein Salz davon, eine Carbonsäureestergruppe, eine Carbonsäurethiolestergruppe, eine unsubstituierte oder substituierte Carbonsäureamidgruppe, ein cyclisiertes, unsubstituiertes oder substituiertes Derivat einer Carbonsäureamidgruppe oder eine Carbonsäurehydrazidgruppe oder
A und Z zusammen einen unsubstituierten oder substituierten Tetrahydrofuran-2-on-Ring bedeuten, unter Einschluss ihrer Säureadditionssalze und Metallkomplexe, oder einem Mittel, welches eine dieser Verbindungen enthält, behandelt.

2. Verfahren nach Anspruch 1, dadurch gekennzeichnet, dass man eine Verbindung der Formel I, in welcher $R^1$, $R^2$ und $R^3$ unabhängig voneinander Wasserstoff, Halogen, Nitro, Cyan, $C_1$-$C_3$-Alkyl oder $C_1$-$C_3$-Alkoxy,
$R^4$, $R^5$ und $R^6$ unabhängig voneinander Wasserstoff, Halogen oder $C_1$-$C_3$-Alkyl,
A eine der Gruppen $-CH_2-$, $-CH_2-CH_2-$ oder $-CH(CH_3)-$ und
Z Cyan, eine der Gruppen $-C(NH_2)=N-OH$ oder $-C(NH_2)=N-O-CO-E$ einen gegebenenfalls substituierten Oxazolin-2-yl-Rest, $-COOR^{12}$, $-COSR^{13}$ oder $-CONR^{14}R^{15}$ bedeuten, worin
E für $-R^7$, $-OR^8$, $-SR^9$ oder $-NR^{10}R^{11}$ steht, worin
$R^7$ $C_1$-$C_7$-Alkyl, welches unsubstituiert oder durch Halogen oder $C_1$-$C_4$-Alkoxy substituiert ist, $C_3$-$C_6$-Cycloalkyl, $C_2$-$C_4$-Alkenyl, Phenyl, welches unsubstituiert oder durch Halogen, Nitro oder $C_1$-$C_3$-Alkyl substituiert ist, Benzyl, welches unsubstituiert oder durch Halogen, Nitro oder $C_1$-$C_3$-Alkyl substituiert ist, oder einen 5-bis 6-gliedrigen heterocyclischen Ring, welcher ein oder zwei Heteroatome aus der Gruppe N, O und S enthält und unsubstituiert oder durch Halogen substituiert ist,
$R^8$, $R^9$ und $R^{10}$ unabhängig voneinander $C_1$-$C_8$-Alkyl, welches unsubstituiert oder durch Halogen substituiert ist, $C_2$-$C_4$-Alkenyl, $C_3$-$C_6$-Alkinyl, Phenyl, welches unsubstituiert oder durch Halogen, $C_1$-$C_3$-Alkyl, $C_1$-$C_3$-Alkoxy, Trifluormethyl oder Nitro substituiert ist, oder Benzyl, welches unsubstituiert

oder durch Halogen oder Nitro substituiert ist,
$R^{11}$ Wasserstoff, $C_1$-$C_8$-Alkyl oder $C_1$-$C_3$-Alkoxy oder
$R^{10}$ und $R^{11}$ gemeinsam mit dem Stickstoffatom, an das sie gebunden sind, einen 5- bis 6-gliedrigen Heterocyclus, welcher noch ein weiteres Heteroatom aus der Gruppe N, O und S enthalten kann,
$R^{12}$, $R^{13}$ und $R^{14}$ Wasserstoff oder einen gegebenenfalls substituierten Alkyl-, Alkenyl-, Alkinyl-, Cycloalkyl-, Phenyl- oder Naphthylrest oder einen gegebenenfalls substituierten heterocyclischen Rest oder $R^{12}$ und $R^{13}$ auch ein Kation oder $R^{14}$ auch einen Alkoxyrest und
$R^{15}$ Wasserstoff, Amino, mono- oder disubstituiertes Amino oder einen gegebenenfalls substituierten Alkyl-, Alkenyl-, Cycloalkyl- oder Phenylrest oder $R^{14}$ und $R^{15}$ gemeinsam mit dem Stickstoffatom, an das sie gebunden sind, einen gegebenenfalls substituierten heterocyclischen Rest bedeuten, oder A und Z zusammen einen gegebenenfalls substituierten Tetrahydrofuran-2-on-Ring bilden, unter Einschluss ihrer Säureadditionssalze und Metallkomplexe, oder ein Mittel, welches eine dieser Verbindungen enthält, verwendet.

3. Verfahren nach Anspruch 1, dadurch gekennzeichnet, dass man eine Verbindung der Formel I, in welcher $R^1$, $R^2$, $R^4$, $R^5$ und $R^6$ Wasserstoff bedeuten, $R^3$ für Wasserstoff oder Chlor und der Rest -A-Z für eine Gruppe $-CH_2-COOR^{16}$ oder $-CH(CH_3)-COOR^{16}$ steht, worin $R^{16}$ für $C_1$-$C_{12}$-Alkyl, $C_3$-$C_6$-Alkenyl, Phenyl-$C_1$-$C_4$-alkyl oder Phenoxy-$C_1$-$C_4$-alkyl steht.

4. Verfahren nach Anspruch 1, dadurch gekennzeichnet, dass man
2-Chinolin-8-yloxy-essigsäureisopropylester,
2-(5-Chlorchinolin-8-yloxy)-essigsäure-n-dodecylester,
2-(5-Chlorchinolin-8-yloxy)-essigsäure-n-butylester,
2-(5-Chlorchinolin-8-yloxy)-essigsäure-n-oktylester,
2-Chinolin-8-yloxy-essigsäure-s-butylester,
2-(5-Chlorchinolin-8-yloxy)-thioessigsäure-n-oktylester,
2-(5-Chlorchinolin-8-yloxy)-essigsäure-(2-butenyl)-ester,
2-(5-Chlorchinolin-8-yloxy)-essigsäuremethallylester,
2-(5-Chlorchinolin-8-yloxy)-essigsäure-(2-isopropyloxyäthyl)-ester,
2-(5-Chlorchinolin-8-yloxy)-essigsäure-(2-phenoxyäthyl)-ester,
2-(5-Chlorchinolin-8-yloxy)-essigsäure-(1-methylbutyl)-ester,
2-(5-Chlorchinolin-8-yloxy)-essigsäurecyclohexylester,
2-(5-Chlorchinolin-8-yloxy)-essigsäure-s-butylester,
2-(5-Chlorchinolin-8-yloxy)-essigsäure-(2-methylpentyl)-ester,
2-(5-Chlorchinolin-8-yloxy)-thioessigsäure-n-butylester,
2-(5-Chlorchinolin-8-yloxy)-essigsäure-(3,6-dioxadecyl)-ester,
2-(5-Chlorchinolin-8-yloxy)-essigsäure-(3-methoxybutyl)-ester,
2-(5-Chlorchinolin-8-yloxy)-essigsäure-(1-äthylbutyl)-ester,
2-(5-Chlorchinolin-8-yloxy)-essigsäure-(2-äthylbutyl)-ester,
2-(5-Chlorchinolin-8-yloxy)-essigsäure-(1-methylisopentyl)-ester,
2-(5-Chlorchinolin-8-yloxy)-essigsäure-n-undecylester,
2-(5-Chlorchinolin-8-yloxy)-essigsäure-(2-methylbutyl)-ester,
2-(5-Chlorchinolin-8-yloxy)-thioessigsäure-s-butylester,
2-(5-Chlorchinolin-8-yloxy)-essigsäure-(3,6-dioxaheptyl)-ester,
2-(5-Chlorchinolin-8-yloxy)-essigsäure-n-heptylester,
2-(5-Chlorchinolin-8-yloxy)-thioessigsäure-n-dodecylester,
2-(5-Chlorchinolin-8-yloxy)-thioessigsäure-n-decylester,
2-(5-Chlorchinolin-8-yloxy)-essigsäure-(1-propylpropargyl)-ester,
2-(5-Chlorchinolin-8-yloxy)-essigsäure-(1-methylisobutyl)-ester,
2-(5-Chlorchinolin-8-yloxy)-thioessigsäure-tert.butylester,
2-(5-Chlorchinolin-8-yloxy)-essigsäure-neopentylester,
2-(5-Chlorchinolin-8-yloxy)-thioessigsäure-n-propylester,
2-(5-Chlorchinolin-8-yloxy)-essigsäure-(1-methylhexyl)-ester,
2-(5-Chlorchinolin-8-yloxy)-thioessigsäureäthylester,
2-(5-Chlorchinolin-8-yloxy)-essigsäure-(2-äthylhexyl)-ester,
2-(5-Chlorchinolin-8-yloxy)-thioessigsäure-i-butylester,
2-Chinolin-8-yloxy-thioessigsäure-n-decylester,
2-(5-Chlorchinolin-8-yloxy)-essigsäure-i-pentylester,
2-(5-Chlorchinolin-8-yloxy)-essigsäure-(1-äthylpentyl)-ester,
2-(5-Chlorchinolin-8-yloxy)-essigsäure-(1-propylbutyl)-ester,
2-(5-Chlorchinolin-8-yloxy)-thioessigsäure-n-hexylester,
2-(5-Chlorchinolin-8-yloxy)-essigsäure-n-hexylester,
2-(5-Chlorchinolin-8-yloxy)-thioessigsäure-i-propylester,
2-(5-Chlorchinolin-8-yloxy)-essigsäure-(1-pentylallyl)-ester,
2-(5-Chlorchinolin-8-yloxy)-essigsäure-(1-methylpentyl)-ester,
2-(5-Chlorchinolin-8-yloxy)-essigsäure-(1,1-dimethylpropargyl)-ester,
2-(5-Chlorchinolin-8-yloxy)-essigsäure-(1-äthyl-1-methylpropargyl)-ester,

2-(5-Chlorchinolin-8-yloxy)-essigsäure-n-butyloxycarbonylmethyl-ester,
2-(5-Chlorchinolin-8-yloxy)-essigsäure-(1-n-butyloxycarbonyläthyl)-ester,
2-(5-Chlorchinolin-8-yloxy)-essigsäure-(1-methylisohexyl)-ester,
2-(5-Chlorchinolin-8-yloxy)-essigsäure-(1-phenylisobutyl)-ester,
2-(5-Chlorchinolin-8-yloxy)-essigsäure-[1-methyl-2-(2-methylphenoxy)-äthyl]-ester,
2-(5-Chlorchinolin-8-yloxy)-essigsäure-(1-phenyläthyl)-ester,
2-(5-Chlorchinolin-8-yloxy)-essigsäure-[1-methyl-2-(4-äthylphenoxy)-äthyl]-ester,
2-(5-Chlorchinolin-8-yloxy)-essigsäure-(1-methyl-2-phenyläthyl)-ester,
2-(5-Chlorchinolin-8-yloxy)-essigsäure-[1-methyl-2-(2-isopropylphenoxy)-äthyl]-ester,
2-(5-Chlorchinolin-8-yloxy)-essigsäure-(1-phenylpropyl)-ester,
2-(5-Chlorchinolin-8-yloxy)-essigsäure-[1-methyl-2-(2-äthylphenoxy)-äthyl]-ester,
2-(5-Chlorchinolin-8-yloxy)-essigsäure-[1-methyl-2-(3-äthylphenoxy)-äthyl]-ester,
2-(5-Chlorchinolin-8-yloxy)-essigsäure-(1-methyl-2-phenoxyäthyl)-ester,
2-(5-Chlorchinolin-8-yloxy)-essigsäure-(1-methyl-3-phenylpropyl)-ester,
2-(5-Chlorchinolin-8-yloxy)-essigsäure-[1-methyl-2-(3-methylphenoxy) -äthyl]-ester,
2-(5-Chlorchinolin-8-yloxy)-essigsäure-[1-methyl-2-(4-isopropylphenoxy)-äthyl]-ester oder
2-(5-Chlorchinolin-8-yloxy)-essigsäure-[1-methyl-2-(4-methylphenoxy)-äthyl]-ester
oder ein Mittel, welches eine dieser Verbindungen enthält, verwendet.

5. Verfahren nach Anspruch 1 dadurch gekennzeichnet, dass man
2-(5-Chlorchinolin-8-yloxy)-essigsäuremethallylester,
2-(5-Chlorchinolin-8-yloxy)-essigsäure-(2-phenoxyäthyl)-ester,
2-(5-Chlorchinolin-8-yloxy)-essigsäure-(1-methylbutyl)-ester,
2-(5-Chlorchinolin-8-yloxy)-essigsäure-(1-äthylbutyl)-ester,
2-(5-Chlorchinolin-8-yloxy)-essigsäure-(1-methylisopentyl)-ester,
2-(5-Chlorchinolin-8-yloxy)-essigsäure-(1-methylhexyl)-ester,
2-(5-Chlorchinolin-8-yloxy)-essigsäure-(1-äthylpentyl)-ester,
2-(5-Chlorchinolin-8-yloxy)-essigsäure-(1-propylbutyl)-ester,
2-(5-Chlorchinolin-8-yloxy)-essigsäure-(1-pentylallyl)-ester,
2-(5-Chlorchinolin-8-yloxy)-essigsäure-(1-methylpentyl)-ester,
2-(5-Chlorchinolin-8-yloxy)-essigsäure-(1-methylisohexyl)-ester,
2-(5-Chlorchinolin-8-yloxy)-essigsäure-(1-phenylisobutyl)-ester,
2-(5-Chlorchinolin-8-yloxy)-essigsäure-(1-phenyläthyl)-ester,
2-(5-Chlorchinolin-8-yloxy)-essigsäure-[1-methyl-2-(4-äthylphenoxy)-äthyl]-ester,
2-(5-Chlorchinolin-8-yloxy)-essigsäure-(1-methyl-2-phenyläthyl)-ester,
2-(5-Chlorchinolin-8-yloxy)-essigsäure-(1-phenylpropyl)-ester,
2-(5-Chlorchinolin-8-yloxy)-essigsäure-(1-methyl-2-phenoxyäthyl)-ester,
2-(5-Chlorchinolin-8-yloxy)-essigsäure-(1-methyl-3-phenylpropyl)-ester oder
2-(5-Chlorchinolin-8-yloxy)-essigsäure-[1-methyl-2-(4-methylphenoxy)-äthyl]-ester oder
ein Mittel, welches eine dieser Verbindungen enthält, verwendet.

6. Verfahren gemäss Anspruch 1, dadurch gekennzeichnet, dass man
2-(5-Chlorchinolin-8-yloxy)-essigsäuremethallylester,
2-(5-Chlorchinolin-8-yloxy)-essigsäure-(2-phenoxyäthyl)-ester,
2-(5-Chlorchinolin-8-yloxy)-essigsäure-(1-methylisopentyl)-ester oder
2-(5-Chlorchinolin-8-yloxy)-essigsäure-(1-methylhexyl)-ester oder ein Mittel, welches eine dieser Verbindungen enthält, verwendet.

7. Verfahren gemäss Anspruch 1 dadurch gekennzeichnet, dass man 2-(5-Chlorchinolin-8-yloxy)-essigsäure-(1-methylisopentyl)-ester oder ein Mittel, welches diese Verbindung enthält, verwendet,

8. Verfahren nach Anspruch 1, dadurch gekennzeichnet, dass man 2-(5-Chlorchinolin-8-yloxy)-essigsäuremethallylester oder ein Mittel, welches diese Verbindung enthält, verwendet.

9. Verfahren gemäss Anspruch 1, dadurch gekennzeichnet, dass man 2-(5-Chlorchinolin-8-yloxy)-essigsäure-(2-phenoxyäthyl)-ester oder ein Mittel, welches diese Verbindung enthält, verwendet.

10. Verfahren gemäss Anspruch 1, dadurch gekennzeichnet, dass man 2-(5-Chlorchinolin-8-yloxy)-essigsäure-(1-methylhexyl)-ester oder ein Mittel, welches diese Verbindung enthält, verwendet.

11. Verfahren nach Anspruch 1 zum Schützen von Kulturpflanzen gegen schädigende Wirkungen von Herbiziden der Formel II, worin die Gruppe -G-Q-Y für einen Silizium-haltigen Rest steht.

12. Verfahren gemäss Anspruch 11, dadurch gekennzeichnet, dass die Gruppe -G-Q-Y für Trimethylsilylmethoxy oder Trimethylsilyläthoxy steht.

13. Verfahren gemäss Anspruch 1 zum Schützen von Kulturpflanzen gegen schädigende Wirkungen von 2-[4-(5-Chlor-3-fluorpyridin-2-yloxy)-phenoxy]-propionsäure-trimethylsilylmethylester.

14. Verfahren gemäss Anspruch 1 zum Schützen von Kulturpflanzen gegen schädigende Wirkungen von 2R-2-[4-(5-Chlor-3-fluorpyridin-2-yloxy)-phenoxy]-propionsäure-trimethylsilylmethylester.

15. Verfahren nach Anspruch 1 zum Schützen von Kulturpflanzen gegen schädigende Wirkungen von 2-[4-(5-Chlor-3-fluorpyridin-2-yloxy)-phenoxy]-propionsäure-trimethylsilyläthylester.

16. Verfahren nach Anspruch 1 zum Schützen von Kulturpflanzen gegen schädigende Wirkungen von 2R-2-[4-(5-Chlor-3-fluorpyridin-2-yloxy)-phenoxy]-propionsäure-trimethylsilyläthylester.

17. Verfahren nach Anspruch 1 zum Schützen von Kulturpflanzen gegen schädigende Wirkungen von 2-[4-(5-Chlor-3-fluorpyridin-2-yloxy)-phenoxy]-propionsäure-trimethylsilylmethylester, 2R-2-[4-(5-Chlor-3-fluorpyridin-2yloxy)-phenoxy]-propionsäure-trimethylsilylmethylester, 2-[4-(5-Chlor-3-fluorpyridin-2-yloxy)-phenoxy]-propionsäure-trimethylsilyläthylester oder 2R-2-[4-(5-Chlor-3-fluorpyridin-2-yloxy)-phenoxy]-propionsäure-trimethylsilyläthylester, dadurch gekennzeichnet, dass man die Kulturpflanzen, Teile dieser Pflanzen oder für den Anbau der Kulturpflanzen bestimmte Böden mit
2-Chlorchinolin-8-yloxy-essigsäureisopropylester,
2-(5-Chlorchinolin-8-yloxy)-essigsäure-n-dodecylester,
2-(5-Chlorchinolin-8-yloxy)-essigsäure-n-butylester,
2-(5-Chlorchinolin-8-yloxy)-essigsäure-n-oktylester,
2-Chlorchinolin-8-yloxy-essigsäure-s-butylester,
2-(5-Chlorchinolin-8-yloxy)-thioessigsäure-n-oktylester,
2-(5-Chlorchinolin-8-yloxy)-essigsäure-(2-butenyl)-ester,
2-(5-Chlorchinolin-8-yloxy)-essigsäuremethallylester,
2-(5-Chlorchinolin-8-yloxy)-essigsäure-(2-isopropyloxyäthyl)-ester,
2-(5-Chlorchinolin-8-yloxy)-essigsäure-(2-phenoxyäthyl)-ester,
2-(5-Chlorchinolin-8-yloxy)-essigsäure-(1-methylbutyl)-ester,
2-(5-Chlorchinolin-8-yloxy)-essigsäurecyclohexylester,
2-(5-Chlorchinolin-8-yloxy)-essigsäure-s-butylester,
2-(5-Chlorchinolin-8-yloxy)-essigsäure-(2-methylpentyl)-ester,
2-(5-Chlorchinolin-8-yloxy)-thioessigsäure-n-butylester,
2-(5-Chlorchinolin-8-yloxy)-essigsäure-(3,6-dioxadecyl)-ester,
2-(5-Chlorchinolin-8-yloxy)-essigsäure-(3-methoxybutyl)-ester,
2-(5-Chlorchinolin-8-yloxy)-essigsäure-(1-äthylbutyl)-ester,
2-(5-Chlorchinolin-8-yloxy)-essigsäure-(2-äthylbutyl)-ester,
2-(5-Chlorchinolin-8-yloxy)-essigsäure-(1-methylisopentyl)-ester,
2-(5-Chlorchinolin-8-yloxy)-essigsäure-n-undecylester,
2-(5-Chlorchinolin-8-yloxy)-essigsäure-(2-methylbutyl)-ester,
2-(5-Chlorchinolin-8-yloxy)-thioessigsäure-s-butylester,
2-(5-Chlorchinolin-8-yloxy)-essigsäure-(3,6-dioxaheptyl)-ester,
2-(5-Chlorchinolin-8-yloxy)-essigsäure-n-heptylester,
2-(5-Chlorchinolin-8-yloxy)-thioessigsäure-n-dodecylester,
2-(5-Chlorchinolin-8-yloxy)-thioessigsäure-n-decylester, 2-(5-Chlorchinolin-8-yloxy)-essigsäure-(1-propylpropargyl)-ester,
2-(5-Chlorchinolin-8-yloxy)-essigsäure-(1-methylisobutyl)-ester,
2-(5-Chlorchinolin-8-yloxy)-thioessigsäure-tert.butylester,
2-(5-Chlorchinolin-8-yloxy)-essigsäure-neopentylester,
2-(5-Chlorchinolin-8-yloxy)-thioessigsäure-n-propylester,
2-(5-Chlorchinolin-8-yloxy)-essigsäure-(1-methylhexyl)-ester,
2-(5-Chlorchinolin-8-yloxy)-thioessigsäureäthylester,
2-(5-Chlorchinolin-8-yloxy)-essigsäure-(2-äthylhexyl)-ester,
2-(5-Chlorchinolin-8-yloxy)-thioessigsäure-i-butylester,
2-Chlorchinolin-8-yloxy-thioessigsäure-n-decylester,
2-(5-Chlorchinolin-8-yloxy)-essigsäure-i-pentylester,
2-(5-Chlorchinolin-8-yloxy)-essigsäure-(1-äthylpentyl)-ester,
2-(5-Chlorchinolin-8-yloxy)-essigsäure-(1-propylbutyl)-ester,
2-(5-Chlorchinolin-8-yloxy)-thioessigsäure-n-hexylester,
2-(5-Chlorchinolin-8-yloxy)-essigsäure-n-hexylester,
2-(5-Chlorchinolin-8-yloxy)-thioessigsäure-i-propylester,
2-(5-Chlorchinolin-8-yloxy)-essigsäure-(1-pentylallyl)-ester,
2-(5-Chlorchinolin-8-yloxy)-essigsäure-(1-methylpentyl)-ester,
2-(5-Chlorchinolin-8-yloxy)-essigsäure-(1,1-dimethylpropargyl)-ester,
2-(5-Chlorchinolin-8-yloxy)-essigsäure-(1-äthyl-1-methylpropargyl)-ester,
2-(5-Chlorchinolin-8-yloxy)-essigsäure-n-butyloxycarbonylmethyl-ester,
2-(5-Chlorchinolin-8-yloxy)-essigsäure-(1-n-butyloxycarbonyläthyl)-ester,
2-(5-Chlorchinolin-8-yloxy)-essigsäure-(1-methylisohexyl)-ester,
2-(5-Chlorchinolin-8-yloxy)-essigsäure-(1-phenylisobutyl)-ester,
2-(5-Chlorchinolin-8-yloxy)-essigsäure-[1-methyl-2-(2-methylphenoxyl)-äthyl]-ester,
2-(5-Chlorchinolin-8-yloxy)-essigsäure-(1-phenyläthyl)-ester,
2-(5-Chlorchinolin-8-yloxy)-essigsäure-[1-methyl-2-(4-äthylphenoxy)-äthyl)-ester,
2-(5-Chlorchinolin-8-yloxy)-essigsäure-(1-methyl-2-phenyläthyl)-ester,
2-(5-Chlorchinolin-8-yloxy)-essigsäure-[1-methyl-2-(2-isopropylphenoxy)-äthyl]-ester,
2-(5-Chlorchinolin-8-yloxy)-essigsäure-(1-phenylpropyl)-ester,
2-(5-Chlorchinolin-8-yloxy)-essigsäure-[1-methyl-2-(2-äthylphenoxy)-äthyl]-ester,
2-(5-Chlorchinolin-8-yloxy)-essigsäure-[1-methyl-2-(3-äthylphenoxy)-äthyl]-ester,

2-(5-Chlorchinolin-8-yloxy)-essigsäure-(1-methyl-2-phenoxyäthyl)-ester, 2-(5-Chlorchinolin-8-yloxy)-essigsäure-(1-methyl-3-phenylpropyl)-ester, 2-(5-Chlorchinolin-8-yloxy)-essigsäure-[1-methyl-2-(3-methylphenoxy) -äthyl]-ester, 2-(5-Chlorchinolin-8-yloxy)-essigsäure-[1-methyl-2-(4-isopropylphenoxy)-äthyl]-ester oder 2-(5-Chlorchinolin-8-yloxy)-essigsäure-[1-methyl-2-(4-methylphenoxy)-äthyl]-ester oder einem Mittel, welches eine dieser Verbindungen enthält, behandelt.

18. Verfahren gemäss Anspruch 17, dadurch gekennzeichnet, dass man die Kulturpflanzen, Teile dieser Pflanzen oder für den Anbau der Kulturpflanzen bestimmte Böden mit 2-(5-Chlorchinolin-8-yloxy)-essigsäuremethallylester, 2-(5-Chlorchinolin-8-yloxy)-essigsäure-(2-phenoxyäthyl)-ester, 2-(5-Chlorchinolin-8-yloxy)-essigsäure-(1-methylbutyl)-ester, 2-(5-Chlorchinolin-8-yloxy)-essigsäure-(1-äthylbutyl)-ester, 2-(5-Chlorchinolin-8-yloxy)-essigsäure-(1-methylisopentyl)-ester, 2-(5-Chlorchinolin-8-yloxy)-essigsäure-(1-methylhexyl)-ester, 2-(5-Chlorchinolin-8-yloxy)-essigsäure-(1-äthylpentyl)-ester, 2-(5-Chlorchinolin-8-yloxy)-essigsäure-(1-propylbutyl)-ester, 2-(5-Chlorchinolin-8-yloxy)-essigsäure-(1-pentylallyl)-ester, 2-(5-Chlorchinolin-8-yloxy)-essigsäure-(1-methylpentyl)-ester, 2-(5-Chlorchinolin-8-yloxy)-essigsäure-(1-methylisohexyl)-ester, 2-(5-Chlorchinolin-8-yloxy)-essigsäure-(1-phenylisobutyl)-ester, 2-(5-Chlorchinolin-8-yloxy)-essigsäure-(1-phenyläthyl)-ester, 2-(5-Chlorchinolin-8-yloxy)-essigsäure-[1-methyl-2-(4-äthylphenoxy)-äthyl]-ester, 2-(5-Chlorchinolin-8-yloxy)-essigsäure-(1-methyl-2-phenyläthyl)-ester, 2-(5-Chlorchinolin-8-yloxy)-essigsäure-(1-phenylpropyl)-ester, 2-(5-Chlorchinolin-8-yloxy)-essigsäure-(1-methyl-2-phenoxyäthyl)-ester 2-(5-Chlorchinolin-8-yloxy)-essigsäure-(1-methyl-3-phenylpropyl)-ester oder 2-(5-Chlorchinolin-8-yloxy)-essigsäure-[1-methyl-2-(4-methylphenoxy)-äthyl]-ester oder einem Mittel, welches eine dieser Verbindungen enthält, behandelt.

19. Verfahren nach Anspruch 17, dadurch gekennzeichnet, dass man die Kulturpflanzen, Teile dieser Pflanzen oder für den Anbau der Kulturpflanzen bestimmte Böden mit 2-(5-Chlorchinolin-8-yloxy)-essigsäure-(1-methylisopentyl)-ester, 2-(5-Chlorchinolin-8-yloxy)-essigsäure-(2-phenoxyäthyl)-ester, 2-(5-Chlorchinolin-8-yloxy)-essigsäure-(1-methylhexyl)-ester oder 2-(5-Chlorchinolin-8-yloxy)-essigsäure-methallylester oder einem Mittel, welches eine dieser Verbindungen enthält, behandelt.

20. Verfahren nach Anspruch 1 zum Schützen von Getreide.

21. Verfahren nach Anspruch 20 zum Schützen von Weisen, Gerste, Roggen und Reis.

22. Verfahren nach Anspruch 1, dadurch gekennzeichnet, dass man Kulturpflanzenbestände oder Anbauflächen für Kulturpflanzen mit 0,001 bis 1 kg/ha einer Verbindung der Formel I gemäss Anspruch 1 behandelt.

23. Verfahren nach Anspruch 22, dadurch gekennzeichnet, dass man Kulturpflanzenbestände oder Anbauflächen für Kulturpflanzen mit 0,01 bis 0,1 kg/ha einer Verbindung der Formel I gemäss Anspruch 1 behandelt.

24. Verfahren nach Anspruch 1, dadurch gekennzeichnet, dass man Samen der Kulturpflanzen mit einer Verbindung der Formel I gemäss Anspruch 1 behandelt.

25. Verfahren nach Anspruch 24, dadurch gekennzeichnet dass man Samen der Kulturpflanze mit 0.01 bis 10 g/kg Samen einer Verbindung der Formel I gemäss Anspruch 1 behandelt.

26. Verfahren nach Anspruch 25, dadurch gekennzeichnet, dass man Samen der Kulturpflanzen mit 0,05 bis 2 g/kg Samen einer Verbindung der Formel I gemäss Anspruch 1 behandelt.

27. Verfahren nach Anspruch 24 zum Schützen von Kulturpflanzen gegen schädigende Wirkungen von 2-[4-(5-Chlor-3-fluorpyridin-2-yloxy)-phenoxy]-propionsäure-trimethylsilylmethylester, 2R-2-[4-(5-Chlor-3-fluorpyridin-2-yloxy)-phenoxy]-propionsäure-trimethylsilylmethylester, 2-[4-(5-Chlor-3-fluorpyridin-2-yloxy)-phenoxy]-propionsäure-trimethylsilyläthylester oder 2R-2-[4-(5-Chlor-3-fluorpyridin-2-yloxy)-phenoxy-propionsäure-trimethylsilyläthylester, dadurch gekennzeichnet, dass man die Samen der Kulturpflanzen, mit 2-Chlorchinolin-8-yloxy-essigsäureisopropylester, 2-(5-Chlorchinolin-8-yloxy)-essigsäure-n-dodecylester, 2-(5-Chlorchinolin-8-yloxy)-essigsäure-n-butylester, 2-(5-Chlorchinolin-8-yloxy)-essigsäure-n-oktylester, 2-Chlorchinolin-8-yloxy-essigsäure-s-butylester, 2-(5-Chlorchinolin-8-yloxy)-thioessigsäure-n-oktylester, 2-(5-Chlorchinolin-8-yloxy)-essigsäure-(2-butenyl)-ester, 2-(5-Chlorchinolin-8-yloxy)-essigsäuremethallylester, 2-(5-Chlorchinolin-8-yloxy)-essigsäure-(2-isopropyloxyäthyl)-ester, 2-(5-Chlorchinolin-8-yloxy)-essigsäure-(2-phenoxyäthyl)-ester, 2-(5-Chlorchinolin-8-yloxy)-essigsäure-(1-methylbutyl)-ester, 2-(5-Chlorchinolin-8-yloxy)-essigsäurecyclohexylester,

2-(5-Chlorchinolin-8-yloxy)-essigsäure-s-butylester,
2-(5-Chlorchinolin-8-yloxy)-essigsäure-(2-methylpentyl)-ester,
2-(5-Chlorchinolin-8-yloxy)-thioessigsäure-n-butylester,
2-(5-Chlorchinolin-8-yloxy)-essigsäure-(3,6-dioxadecyl)-ester,
2-(5-Chlorchinolin-8-yloxy)-essigsäure-(3-methoxybutyl)-ester,
2-(5-Chlorchinolin-8-yloxy)-essigsäure-(1-äthylbutyl)-ester,
2-(5-Chlorchinolin-8-yloxy)-essigsäure-(2-äthylbutyl)-ester,
2-(5-Chlorchinolin-8-yloxy)-essigsäure-(1-methylisopentyl)-ester,
2-(5-Chlorchinolin-8-yloxy)-essigsäure-n-undecylester,
2-(5-Chlorchinolin-8-yloxy)-essigsäure-(2-methylbutyl)-ester,
2-(5-Chlorchinolin-8-yloxy)-thioessigsäure-s-butylester,
2-(5-Chlorchinolin-8-yloxy)-essigsäure-(3,6-dioxaheptyl)-ester,
2-(5-Chlorchinolin-8-yloxy)-essigsäure-n-heptylester,
2-(5-Chlorchinolin-8-yloxy)-thioessigsäure-n-dodecylester,
2-(5-Chlorchinolin-8-yloxy)-thioessigsäure-n-decylester,
2-(5-Chlorchinolin-8-yloxy)-essigsäure-(1-propylpropargyl)-ester,
2-(5-Chlorchinolin-8-yloxy)-essigsäure-(1-methylisobutyl)-ester,
2-(5-Chlorchinolin-8-yloxy)-thioessigsäure-tert.butylester,
2-(5-Chlorchinolin-8-yloxy)-essigsäure-neopentylester,
2-(5-Chlorchinolin-8-yloxy)-thioessigsäure-n-propylester,
2-(5-Chlorchinolin-8-yloxy)-essigsäure-(1-methylhexyl)-ester,
2-(5-Chlorchinolin-8-yloxy)-thioessigsäureäthylester,
2-(5-Chlorchinolin-8-yloxy)-essigsäure-(2-äthylhexyl)-ester,
2-(5-Chlorchinolin-8-yloxy)-thioessigsäure-i-butylester,
2-Chinolin-8-yloxy-thioessigsäure-n-decylester,
2-(5-Chlorchinolin-8-yloxy)-essigsäure-i-pentylester,
2-(5-Chlorchinolin-8-yloxy)-essigsäure-(1-äthylpentyl)-ester,
2-(5-Chlorchinolin-8-yloxy)-essigsäure-(1-propylbutyl)-ester,
2-(5-Chlorchinolin-8-yloxy)-thioessigsäure-n-hexylester,
2-(5-Chlorchinolin-8-yloxy)-essigsäure-n-hexylester,
2-(5-Chlorchinolin-8-yloxy)-thioessigsäure-i-propylester,
2-(5-Chlorchinolin-8-yloxy)-essigsäure-(1-pentylallyl)-ester,
2-(5-Chlorchinolin-8-yloxy)-essigsäure-(1-methylpentyl-ester,
2-(5-Chlorchinolin-8-yloxy)-essigsäure-(1,1-dimethylpropargyl)-ester,
2-(5-Chlorchinolin-8-yloxy)-essigsäure-(1-äthyl-1-methylpropargyl)-ester,
2-(5-Chlorchinolin-8-yloxy)-essigsäure-n-butyloxycarbonylmethyl-ester,
2-(5-Chlorchinolin-8-yloxy)-essigsäure-(1-n-butyloxycarbonyläthyl)-ester,
2-(5-Chlorchinolin-8-yloxy)-essigsäure-(1-methylisohexyl)-ester,
2-(5-Chlorchinolin-8-yloxy)-essigsäure-(1-phenylisobutyl)-ester,
2-(5-Chlorchinolin-8-yloxy)-essigsäure-[1-methyl-2-(2-methylphenoxy)-äthyl]-ester,
2-(5-Chlorchinolin-8-yloxy)-essigsäure-(1-phenyläthyl)-ester,
2-(5-Chlorchinolin-8-yloxy)-essigsäure-[1-methyl-2-(4-äthylphenoxy)-äthyl]-ester,
2-(5-Chlorchinolin-8-yloxy)-essigsäure-(1-methyl-2-phenyläthyl)-ester,
2-(5-Chlorchinolin-8-yloxy)-essigsäure-[1-methyl-2-(2-isopropylphenoxy)-äthyl]-ester,
2-(5-Chlorchinolin-8-yloxy)-essigsäure-(1-phenylpropyl)-ester,
2-(5-Chlorchinolin-8-yloxy)-essigsäure-[1-methyl-2-(2-äthylphenoxy)-äthyl]-ester,
2-(5-Chlorchinolin-8-yloxy)-essigsäure-[1-methyl-2-(3-äthylphenoxy)-äthyl]-ester,
2-(5-Chlorchinolin-8-yloxy)-essigsäure-(1-methyl-2-phenoxyäthyl)-ester
2-(5-Chlorchinolin-8-yloxy)-essigsäure-(1-methyl-3-phenylpropyl)-ester,
2-(5-Chlorchinolin-8-yloxy)-essigsäure-[1-methyl-2-(3-methylphenoxy) -äthyl]-ester,
2-(5-Chlorchinolin-8-yloxy)-essigsäure-[1-methyl-2-(4-isopropylphenoxy)-äthyl]-ester oder
2-(5-Chlorchinolin-8-yloxy)-essigsäure-[1-methyl-2-(4-methylphenoxy)-äthyl]-ester
oder einem Mittel, welches eine dieser Verbindungen enthält, behandelt.

28. Verfahren gemäss Anspruch 27, dadurch gekennzeichnet, dass man die Samen der Kulturpflanzen mit
2-(5-Chlorchinolin-8-yloxy)-essigsäuremethallylester,
2-(5-Chlorchinolin-8-yloxy)-essigsäure-(2-phenoxyäthyl)-ester,
2-(5-Chlorchinolin-8-yloxy)-essigsäure-(1-methylbutyl)-ester,
2-(5-Chlorchinolin-8-yloxy)-essigsäure-(1-äthylbutyl)-ester,
2-(5-Chlorchinolin-8-yloxy)-essigsäure-(1-methylisopentyl)-ester,
2-(5-Chlorchinolin-8-yloxy)-essigsäure-(1-methylhexyl)-ester,
2-(5-Chlorchinolin-8-yloxy)-essigsäure-(1-äthylpentyl)-ester,
2-(5-Chlorchinolin-8-yloxy)-essigsäure-(1-propylbutyl)-ester,
2-(5-Chlorchinolin-8-yloxy)-essigsäure-(1-pentylallyl)-ester oder
2-(5-Chlorchinolin-8-yloxy)-essigsäure-(1-methylpentyl)-ester oder

2-(5-Chlorchinolin-8-yloxy)-essigsäure-(1-methylisohexyl)-ester,
2-(5-Chlorchinolin-8-yloxy)-essigsäure-(1-phenylisobutyl-ester,
2-(5-Chlorchinolin-8-yloxy)-essigsäure-(1-phenyläthyl)-ester,
2-(5-Chlorchinolin-8-yloxy)-essigsäure-[1-methyl-2-(4-äthylphenoxy)-äthyl)-ester,
2-(5-Chlorchinolin-8-yloxy)-essigsäure-(1-methyl-2-phenyläthyl)-ester,
2-(5-Chlorchinolin-8-yloxy)-essigsäure-(1-phenylpropyl)-ester,
2-(5-Chlorchinolin-8-yloxy)-essigsäure-(1-methyl-2-phenoxyäthyl)-ester,
2-(5-Chlorchinolin-8-yloxy)-essigsäure-(1-methyl-3-phenylpropyl)-ester oder
2-(5-Chlorchinolin-8-yloxy)-essigsäure-[1-methyl-2-(4-methylphenoxy)-äthyl]-ester oder
einem Mittel, welches eine dieser Verbindungen enthält, behandelt.

29. Verfahren gemäss Anspruch 27, dadurch gekennzeichnet, dass man die Samen der Kulturpflanzen mit 2-(5-Chlorchinolin-8-yloxy)-essigsäure-(1-methylisopentyl)-ester, 2-(5-Chlorchinolin-8-yloxy)-essig säure-(2-phenoxyäthyl)-ester, 2-(5-Chlorchinolin-8-yloxy)-essigsäure-(1-methylhexyl)-ester oder 2-(5-Chlorchinolin-8-yloxy)-essigsäuremethallylester oder einem Mittel, welches eine dieser Verbindungen enthält, behandelt.

30. Verfahren nach Anspruch 27 zum Schützen von Getreide.

31. Verwendung von Verbindungen der Formel I gemäss Anspruch 1 zum Schützen von Kulturpflanzen gegen schädigende Wirkungen von Verbindungen der Formel II gemäss Anspruch 1.

32. Verfahren zur selektiven Bekämpfung von Unkräutern in Kulturpflanzenbeständen, dadurch gekennzeichnet, dass man die Kulturpflanzenbestände, Teile der Kulturpflanzen oder Anbauflächen der Kulturpflanzen mit einem Antidot der Formel I gemäss Anspruch 1 und einem Herbizid der Formel II gemäss Anspruch 1 behandelt.

33. Herbizides Mittel, dadurch gekennzeichnet, dass es neben eine herbiziden Wirkstoff der Formel II gemäss Anspruch 1 ein Antidot der Formel I gemäss Anspruch 1 enthält.

34. Mittel gemäss Anspruch 33, dadurch gekennzeichnet, dass es neben einem Herbizid aus der Reihe 2-[4-(5-Chlor-3-fluorpyridin-2-yloxy)-phenoxy]-propionsäure-trimethylsilylmethylester, 2R-2-[4-(5-Chlor-3-fluorpyridin-2-yloxy)-phenoxy]-propionsäure-trimethylsilylmethyl-ester, 2-[4-(5-Chlor-3-fluorpyridin-2-yloxy)-phenoxy]-propionsäure-trimethylsilyläthylester oder 2R-2-[4-(5-Chlor-3-fluorpyridin-2-yloxy)-phenoxy]-propionsäure-trimethylsilyläthylester ein Antidot aus der Reihe
2-Chinolin-8-yloxy-essigsäureisopropylester,
2-(5-Chlorchinolin-8-yloxy)-essigsäure-n-dodecylester,
2-(5-Chlorchinolin-8-yloxy)-essigsäure-n-butylester,
2-(5-Chlorchinolin-8-yloxy)-essigsäure-n-oktylester,
2-Chinolin-8-yloxy-essigsäure-s-butylester,
2-(5-Chlorchinolin-8-yloxy)-thioessigsäure-n-oktylester,
2-(5-Chlorchinolin-8-yloxy)-essigsäure-(2-butenyl)-ester,
2-(5-Chlorchinolin-8-yloxy)-essigsäuremethallylester,
2-(5-Chlorchinolin-8-yloxy)-essigsäure-(2-isopropyloxyäthyl)-ester,
2-(5-Chlorchinolin-8-yloxy)-essigsäure-(2-phenoxyäthyl)-ester,
2-(5-Chlorchinolin-8-yloxy)-essigsäure-(1-methylbutyl)-ester,
2-(5-Chlorchinolin-8-yloxy)-essigsäurecyclohexylester,
2-(5-Chlorchinolin-8-yloxy)-essigsäure-s-butylester,
2-(5-Chlorchinolin-8-yloxy)-essigsäure-(2-methylpentyl)-ester,
2-(5-Chlorchinolin-8-yloxy)-thioessigsäure-n-butylester,
2-(5-Chlorchinolin-8-yloxy)-essigsäure-(3,6-dioxadecyl)-ester,
2-(5-Chlorchinolin-8-yloxy)-essigsäure-(3-methoxybutyl)-ester,
2-(5-Chlorchinolin-8-yloxy)-essigsäure-(1-äthylbutyl)-ester,
2-(5-Chlorchinolin-8-yloxy)-essigsäure-(2-äthylbutyl)-ester,
2-(5-Chlorchinolin-8-yloxy)-essigsäure-(1-methylisopentyl)-ester,
2-(5-Chlorchinolin-8-yloxy)-essigsäure-n-undecylester,
2-(5-Chlorchinolin-8-yloxy)-essigsäure-(2-methylbutyl)-ester,
2-(5-Chlorchinolin-8-yloxy)-thioessigsäure-s-butylester,
2-(5-Chlorchinolin-8-yloxy)-essigsäure-(3,6-dioxaheptyl)-ester,
2-(5-Chlorchinolin-8-yloxy)-essigsäure-n-heptylester,
2-(5-Chlorchinolin-8-yloxy)-thioessigsäure-n-dodecylester,
2-(5-Chlorchinolin-8-yloxy)-thioessigsäure-n-decylester,
2-(5-Chlorchinolin-8-yloxy)-essigsäure-(1-propylpropargyl)-ester,
2-(5-Chlorchinolin-8-yloxy)-essigsäure-(1-methylisobutyl)-ester,
2-(5-Chlorchinolin-8-yloxy)-thioessigsäure-tert.butylester,
2-(5-Chlorchinolin-8-yloxy)-essigsäure-neopentylester,
2-(5-Chlorchinolin-8-yloxy)-thioessigsäure-n-propylester,
2-(5-Chlorchinolin-8-yloxy)-essigsäure-(1-methylhexyl)-ester,
2-(5-Chlorchinolin-8-yloxy)-thioessigsäureäthylester,
2-(5-Chlorchinolin-8-yloxy)-essigsäure-(2-äthylhexyl)-ester,
2-(5-Chlorchinolin-8-yloxy)-thioessigsäure-i-butylester,

2-Chinolin-8-yloxy-thioessigsäure-n-decylester,
2-(5-Chlorchinolin-8-yloxy)-essigsäure-i-pentylester,
2-(5-Chlorchinolin-8-yloxy)-essigsäure-(1-äthylpentyl)-ester,
2-(5-Chlorchinolin-8-yloxy)-essigsäure-(1-propylbutyl)-ester,
2-(5-Chlorchinolin-8-yloxy)-thioessigsäure-n-hexylester,
2-(5-Chlorchinolin-8-yloxy)-essigsäure-n-hexylester,
2-(5-Chlorchinolin-8-yloxy)-thioessigsäure-i-propylester,
2-(5-Chlorchinolin-8-yloxy)-essigsäure-(1-pentylallyl)-ester,
2-(5-Chlorchinolin-8-yloxy)-essigsäure-(1-methylpentyl)-ester,
2-(5-Chlorchinolin-8-yloxy)-essigsäure-(1,1-dimethylpropargyl)-ester,
2-(5-Chlorchinolin-8-yloxy)-essigsäure-(1-äthyl-1-methylpropargyl)-ester,
2-(5-Chlorchinolin-8-yloxy)-essigsäure-n-butyloxycarbonylmethyl-ester,
2-(5-Chlorchinolin-8-yloxy)-essigsäure-(1-n-butyloxycarbonyläthyl)-ester,
2-(5-Chlorchinolin-8-yloxy)-essigsäure-(1-methylisohexyl)-ester,
2-(5-Chlorchinolin-8-yloxy)-essigsäure-(1-phenylisobutyl)-ester,
2-(5-Chlorchinolin-8-yloxy)-essigsäure-[1-methyl-2-(2-methylphenoxy)-äthyl]-ester,
2-(5-Chlorchinolin-8-yloxy)-essigsäure-(1-phenyläthyl)-ester,
2-(5-Chlorchinolin-8-yloxy)-essigsäure-[1-methyl-2-(4-äthylphenoxy)-äthyl]-ester,
2-(5-Chlorchinolin-8-yloxy)-essigsäure-(1-methyl-2-phenyläthyl)-ester,
2-(5-Chlorchinolin-8-yloxy)-essigsäure-[1-methyl-2-(2-isopropylphenoxy)-äthyl]-ester,
2-(5-Chlorchinolin-8-yloxy)-essigsäure-(1-phenylpropyl)-ester,
2-(5-Chlorchinolin-8-yloxy)-essigsäure-[1-methyl-2-(2-äthylphenoxy)-äthyl]-ester,
2-(5-Chlorchinolin-8-yloxy)-essigsäure-[1-methyl-2-(3-äthylphenoxy)-äthyl]-ester,
2-(5-Chlorchinolin-8-yloxy)-essigsäure-(1-methyl-2-phenoxyäthyl)-ester,
2-(5-Chlorchinolin-8-yloxy)-essigsäure-(1-methyl-3-phenylpropyl)-ester,
2-(5-Chlorchinolin-8-yloxy)-essigsäure-[1-methyl-2-(3-methylphenoxy) -äthyl]-ester,
2-(5-Chlorchinolin-8-yloxy)-essigsäure-[1-methyl-2-(4-isopropylphenoxy)-äthyl]-ester oder
2-(5-Chlorchinolin-8-yloxy)-essigsäure-[1-methyl-2-(4-methylphenoxy)-äthyl]-ester enthält.

35. Mittel gemäss Anspruch 34, dadurch gekennzeichnet, dass es ein Antidot aus der Reihe
2-(5-Chlorchinolin-8-yloxy)-essigsäuremethallylester,
2-(5-Chlorchinolin-8-yloxy)-essigsäure-(2-phenoxyäthyl)-ester,
2-(5-Chlorchinolin-8-yloxy)-essigsäure-(1-methylbutyl)-ester,
2-(5-Chlorchinolin-8-yloxy)-essigsäure-(1-äthylbutyl)-ester,
2-(5-Chlorchinolin-8-yloxy)-essigsäure-(1-methylisopentyl)-ester,
2-(5-Chlorchinolin-8-yloxy)-essigsäure-(1-methylhexyl)-ester,
2-(5-Chlorchinolin-8-yloxy)-essigsäure-(1-äthylpentyl)-ester,
2-(5-Chlorchinolin-8-yloxy)-essigsäure-(1-propylbutyl)-ester,
2-(5-Chlorchinolin-8-yloxy)-essigsäure-(1-pentylallyl)-ester,
2-(5-Chlorchinolin-8-yloxy)-essigsäure-(1-methylpentyl)-ester,
2-(5-Chlorchinolin-8-yloxy)-essigsäure-(1-methylisohexyl)-ester,
2-(5-Chlorchinolin-8-yloxy)-essigsäure-(1-phenylisobutyl)-ester,
2-(5-Chlorchinolin-8-yloxy)-essigsäure-(1-phenyläthyl)-ester,
2-(5-Chlorchinolin-8-yloxy)-essigsäure-[1-methyl-2-(4-äthylphenoxy)-äthyl]-ester,
2-(5-Chlorchinolin-8-yloxy)-essigsäure-(1-methyl-2-phenyläthyl)-ester,
2-(5-Chlorchinolin-8-yloxy)-essigsäure-(1-phenylpropyl)-ester,
2-(5-Chlorchinolin-8-yloxy)-essigsäure-(1-methyl-2-phenoxyäthyl)-ester
2-(5-Chlorchinolin-8-yloxy)-essigsäure-(1-methyl-3-phenylpropyl)-ester oder
2-(5-Chlorchinolin-8-yloxy)-essigsäure-[1-methyl-2-(4-methylphenoxy)-äthyl]-ester enthält.

36. Mittel gemäss Anspruch 34, dadurch gekennzeichnet, dass es als Antidot
2-(5-Chlorchinolin-8-yloxy)-essigsäuremethallylester,
2-(5-Chlorchinolin-8-yloxy)-essigsäure-(2-phenoxyäthyl)-ester,
2-(5-Chlorchinolin-8-yloxy)-essigsäure-(1-methylisopentyl)-ester oder
2-(5-Chlorchinolin-8-yloxy)-essigsäure-(1-methylhexyl)-ester enthält.

37. Verfahren zur Herstellung der Mittel gemäss Anspruch 33, dadurch gekennzeichnet, dass man die Wirkstoffe der Formel I and II intensiv miteinander und gegebenenfalls einem Trägermaterial und/oder oberflächenaktivem Mittel vermischt.